Europäisches Patentamt

European Patent Office

Office européen des brevets

(19)

(11) Publication number: **0 173 280 B1**

(12) # EUROPEAN PATENT SPECIFICATION

(45) Date of publication of patent specification: **12.08.92**

(51) Int. Cl.⁵: **C12N 15/73**, C12P 21/02, C12N 15/67, C12P 19/34, //C12R1/19

(21) Application number: **85110683.1**

(22) Date of filing: **26.08.85**

Divisional application 92101250.6 filed on 26/08/85.

(54) **Plasmids containing lambda PI promoter, and engineered restriction site for convenient replacement of ribosomal binding site, hosts containing the plasmids and related methods.**

(30) Priority: **27.08.84 US 644105**
**27.08.84 US 644245**
**27.08.84 US 644551**
**27.08.84 US 644671**
**27.08.84 US 645119**

(43) Date of publication of application:
**05.03.86 Bulletin 86/10**

(45) Publication of the grant of the patent:
**12.08.92 Bulletin 92/33**

(84) Designated Contracting States:
**AT BE CH DE FR GB IT LI LU NL SE**

(56) References cited:
**EP-A- 0 041 767**
**EP-A- 0 049 619**
**EP-A- 0 131 843**
**EP-A- 0 138 111**

(73) Proprietor: **BIO-TECHNOLOGY GENERAL CORPORATION**
**375 Park Avenue, Suite 3303**
**New York New York 10152(US)**

(72) Inventor: **Aviv, Haim**
**40 Benjamin Street**
**Rehovot(IL)**
Inventor: **Bartfeld, Daniel**
**30 Negba Street**
**Nes Ziona(IL)**
Inventor: **Gorecki, Marian**
**5 Hanasi Harishon Street**
**Rehovot(IL)**
Inventor: **Hartman, Jacob R.**
**30 Kedoshay Kahir Street**
**Holon(IL)**
Inventor: **Kanner, Dov**
**21 Gordon Street**
**Rehovot(IL)**
Inventor: **Levanon, Avigdor**
**3 Borodetski Street**
**Netanya(IL)**

EP 0 173 280 B1

Nature, vol. 292, n 5819, July 9, 1981 (New York, London). H. Shimatake et al "Purified regulatory protein cII positively activates promoters for lysogenic developments" pages 128-132

The Embo Journal, vol. 1, n 10, 1982 (Oxford, GB). M. Zabeau et al "Enhanced expression of cro-B-galactosidase fusion proteins under the control of the P R promoter of bacteriophage 2", pages 1217-1224

Inventor: **Locker-Giladi, Hilla**
**39 Harlap Street**
**Jerusalem(IL)**
Inventor: **Oppenheim, Amos B.**
**5/12 Schrem Street**
**Jerusalem(IL)**

(74) Representative: **Henkel, Feiler, Hänzel und Partner**
**Möhlstrasse 37**
**W-8000 München 80(DE)**

## Description

Background of the Invention

One aspect of genetic engineering involves the insertion of foreign DNA sequences derived from eucaryotic sources into Escherichia coli or other microorganisms. A further refinement of genetic engineering concerns inducing the resulting microorganism to produce polypeptides encoded by the foreign DNA. Production of polypeptides can be considered a two-step process, with each step including numerous substeps. The two steps are transcription and translation. To produce a polypeptide efficiently and in quantity both steps of the process must be efficient. Transcription is the production of mRNA from the gene (DNA). Translation is the production of polypeptide from the mRNA.

A critical substep of the transcription process is initiation, that is, the binding of RNA polymerase to a promoter-operator region. The sequence of deoxyribonucleotide bases which make up the promoter region may vary and thereby affect the relative efficiency of the promoter. The efficiency depends on the affinity of the RNA polymerase for the promoter.

The efficiency of translation is affected by the stability of the mRNA. Increased stability of the mRNA permits improved translation. Although the exact determinants of mRNA stability are not precisely known, it is known that mRNA secondary structure as determined by the sequence of its bases has a role in stability.

The initial substep of translation involves binding of the ribosome to a base sequence on the mRNA known as the Shine-Dalgarno sequence or the ribosomal binding site (RBS). The synthesis of polypeptides begins when the ribosome migrates along the mRNA to the AUG start codon for translation. Generally these codons are found approximately 10 bases "downstream" from the Shine-Dalgarno site. Factors which increase the efficiency of translation include those which enhance binding of the ribosomes to the Shine-Dalgarno site. It has been shown that the structure of the mRNA in the region of the Shine-Dalgarno sequence and the AUG codon and the distance between the Shine-Dalgarno sequence and the AUG codon each play a critical role in determining the efficiency of translation. Other factors which affect the efficiency of translation are premature termination and attenuation. Efficiency of translation can be improved by removing the attenuation sites.

A difficulty encountered in attempts to produce high amounts of eucaryotic polypeptides in bacterial cells involves the inability of cells producing large amounts of mRNA to grow efficiently. This difficulty can be eliminated by preventing transcription by a process known as repression. In repression, genes are switched off due to the action of a protein inhibitor (repressor protein) which prevents transcription by binding to the operator region. After microorganisms have grown to desired cell densities, the repressed genes are activated by destruction of the repressor or by addition of molecules known as inducers which overcome the effect of the repressor.

Numerous reports may be found in the literature concerning the cloning of eucaryotic genes in plasmids containing the $P_L$ promoter from λ bacteriophage. (Bernard, H.V., et al., Gene (1979) 5, 59; Derom, C., et al., Gene (1982) 17, 45; Gheysen, D., et al., Gene (1982) 17, 55; Hedgpeth, J. et al., Mol. Gen. Genet. (1978) 163, 197; Remaut, E., et al., (1981) Gene 15, 81 and Derynck, R., et al., Nature (1980) 287, 193. In addition, European Patent Application No. 041,767, published December 16, 1981, describes expression vectors containing the $P_L$ promoter from λ bacteriophage. However, none of these references describe the use of the $C_{II}$ ribosomal binding site.

The use of a vector containing the $P_L$ promoter from λ bacteriophage and the $C_{II}$ ribosomal binding site has been described. (Oppenheim, A.B. et al., J. Mol. Biol. (1982) 158, 327 and Shimatake, H. and Rosenberg, M., Nature (1981) 292, 128.) These publications describe the production of increased levels of $C_{II}$ protein but do not involve or describe the production of eucaryotic proteins.

Other vectors which contain the $P_L$ promoter and the $C_{II}$ ribosomal binding site have also been described (Courtney, M. et al., PNAS (1984) 81, 669-673; Lautenberger, J.A. et al., Gene (1983) 23, 75-84 and Lautenberger, J.A. et al., Science (1983) 221, 858-860). However, all of these vectors lead to the production of fused proteins which contain the amino terminal portion of the $C_{II}$ protein.

In 1982 Shatzman and Rosenberg presented a poster at the 14th Miami Winter Symposium (Shatzman, A.R. and Rosenberg, M., 14 Miami Winter Symposium, abstract p98 [1982]). This abstract provides a non-enabling disclosure of the use of a vector containing $P_L$ from λ bacteriophage, Nut and the $C_{II}$ ribosomal binding site to synthesize a "eucaryotic" polypeptide (SV40 small T antigen is actually not a eucaryotic polypeptide but a viral protein) in an amount greater than 5% of the cell protein in an unnamed bacterial host. The operator used is not defined. Neither an origin of replication nor a gene for a selectable phenotype is identified. This system with which the vector is used is described as including certain host lysogens into which the vector can be stably transformed.

Applicants are aware of the existence of a pending U.S. patent application in the name of M. Rosenberg filed under Serial No. 457,352 by the National Institutes of Health, Dept. of Health and Human Services, U.S.A. Portions of this application have been obtained from the National Technical Information Service, U.S. Dept. of Commerce. However, the claims are not available and are maintained in confidence. The available portions of the application have been reviewed. This disclosure is not enabling. It indicates that the host is important (p8, line 17) but fails to identify any suitable host. It further depends upon the use of a λ mutant which is not specified (p4, line 20). It indicates that the host contains lysogens (p8, line 18) unlike the present invention in which the host is not lysogenic. It mentions cloning and expression of a eucaryotic gene, monkey metallothionein gene, (p7, line 18) but does not provide details. It specifies that neither the sequence nor the position of any nucleotide in the $C_{II}$ ribosomal binding region has been altered (p3, line 27).

Pending, co-assigned U.S. patent application Serial No. 514,188, filed July 15, 1983, describes novel vectors useful for the expression of polypeptides in bacteria. These vectors include $P_L O_L$, N utilization site for binding antiterminator N protein, ribosomal binding site, ATG codon, restriction enzyme site for inserting the gene encoding the desired polypeptide, an origin of replication and a selectable marker. In these vectors the distance between the N utilization site and the ribosomal binding site is greater than about 300 base pairs. In addition, each of these vectors contains a specific ribosomal binding site which cannot be readily replaced. These vectors were not equally useful for expression of different polypeptides.

$T_1 T_2$ rRNA transcription termination sequences have been described (Brosius, J., et al., J. Mol. Biol. 148, 107 (1981)). The placement of $T_1 T_2$ rRNA termination sequences at the 3' end of a procaryotic gene and the expression of such gene under the control of a promoter have been described (Amann, E., et al., Gene (1983) 25, 167; Zabeau, M., et al., The EMBO Journal (1982) 1, 1217).

European patent application no. 81304573.9, published April 14, 1982 under European publication no. 049,619, discloses the use of the λcI857 thermoinducible repressor as a stabilizing element. The repressor is cloned on the plasmid. A λcI90 prophage defective in repressor synthesis is introduced by infection. The prophage is maintained by the cloned repressor at temperatures below 32°C. Any cell losing the plasmid will be lysed. If the temperature is increased to above 38°C, the repressor is destroyed or inactivated and the cells lyse. This stabilization system is not compatible with the vectors of the invention which include λ$P_L$ promoter and which express polypeptides at temperatures above 38°C.

Origins of replication from constitutive high copy number plasmids are known. For example pOPIΔ6 origin of replication from ColE1 has been described (Gelfand, D. H., et al., PNAS (1978) 75, (12), 5869 and Muesing, M., et al. Cell (1981) 45, 235). In addition, high copy number run-away replication plasmids, as distinguished from, constitutive high copy number plasmids, are known (Remant, E., et al. Gene (1983) 22, 103).

The present invention relates to plasmids which unexpectedly provide enhanced expression of different polypeptides. By employing different ribosomal binding sites in the plasmids of this invention it is possible to achieve enhanced expression levels of different polypeptides relative to the levels achieved with the previous vectors. In addition, using the same ribosomal binding sites as in the previous vectors and plasmids, it is possible to achieve enhanced expression of the same polypeptides. Moreover, by placing $T_1 T_2$ rRNA termination sequences at the 3' end of the gene encoding a polypeptide whose expression is desired, it is possible to increase the amount of desired polypeptide relative to the total polypeptide produced by a bacterial host. As importantly, the presence of the $T_1 T_2$ rRNA transcription termination sequences permit origins of replication derived from constitutive high copy number plasmids to be incorporated into expression vector without loss of the ability to replicate in constitutive high copy number.

Origin of replication derived from pBR322 or nonconstitutive high copy number plasmids other than runaway high copy number plasmids when incorporated into a vector are capable of producing only a certain number of copies per cell, typically less than 40 copies per cell. By substituting an origin of replication from a constitutive high copy number plasmid it has unexpectedly been found that that level of polypeptide expression is increased.

The preferred plasmids of this invention include vectors that are stabilized in the bacterial host and when bacteria containing plasmids which include the vectors and genes encoding polypeptides are grown, the plasmids are not lost. In this way, yield reduction caused by plasmid instability is overcome. Moreover, use of such preferred vectors avoids the use of antibiotic resistance as a selectable marker, thus permitting lower costs for producing polypeptides.

Superoxide dismutase (SOD) and analogs thereof are some of several polypeptides which may be produced using the novel expression vectors of this invention.

The present invention relates in particular to expression plasmids which unexpectedly provide enhanced expression of superoxide dismutase and analogs thereof using the same ribosomal binding sites as in the previous vectors and by employing different ribosomal binding sites as described in this invention.

The present invention also relates to a method for enhanced production of SOD and analogs thereof in bacteria utilizing these plasmids.

Superoxide dismutase (SOD) and the phenomenon of oxygen free radicals ($O_2{}^-$) was discovered in 1968 by McCord and Fridovich (McCord, J.M. and Fridovich, I., J. Biol. Chem. 244, 6049-6055 (1969). Superoxide radicals and other highly reactive oxygen species are produced in every respiring cell as by-products of oxidative metabolism, and have been shown to cause extensive damage to a wide variety of macromolecules and cellular components (for review see Fridovich, I. in Advances in Inorganic Biochemistry, eds., Eichhorn, G.L. and Marzilli, L.G. (Elsevier/North Holland, New York), pp. 67-90 (1970) and Freeman, B.A. and Crapo, J.D., Laboratory Investigation 47, 412-426 (1982)). A group of metalloproteins known as superoxide dismutases catalyze the oxidation-reduction reaction $2O_2{}^{\bullet-} + {}_2H^+ \rightarrow H_2O_2 + O_2$ and thus provide a defense mechanism against oxygen toxicity. There are three known forms of SODs containing different metals in the protein molecules namely iron, manganese or both copper and zinc. All of them catalyze the same reaction with ultimate efficiency, and all operate by a similar mechanism in which the metal is the catalytic factor in the active site. These enzymes fall into several evolutionary groups. The Mn and Fe-SODs are found primarily in prokaryotic cells while CuZn-SOD have been demonstrated in virtually all eucaryotic organisms (Steinman, H.M. in Superoxide Dismutase, ed. Oberley, L.W. (CRC Press, Florida), pp. 11-68 (1982).

Human Cu/Zn SOD-1 is a dimeric metallo-protein composed of identical non-covalently linked subunits, each having a molecular weight of 16000 daltons and containing one atom of copper and one of zinc (Hartz, J.W. and Deutsch, H.F., J. Biol. Chem. 247, 7043-7050 (1972)). Each subunit is composed of 153 amino acids whose sequence was established (Jabusch, J.R., Farb, D.L. Kerschensteiner, D.A. and Deutsch, H.F., Biochemistry 19:2310-2316 (1980) and Barra, D., Martini, F., Bannister, J.V., Schinina, M.W. Rotilio, W.H. Bannister, W.H. and Bossa, F., FEBS Letters 120, 53-56 (1980)). Furthermore, a cDNA clone containing the entire coding region of human SOD-1 was recently isolated and sequenced (Lieman-Hurwitz, J., Dafni, N., Lavie, V. and Groner, Y., Proc. Natl. Acad. Sci. USA 79, 2808-2811 (1982) and Sherman, L., Dafni, N., Leiman-Hurwitz, J. and Groner, Y., Proc. Natl. Acad. Sci. USA 80, 5465-5469 (1983)).

The human Cu-Zn SOD analog produced differs from natural human Cu-Zn SOD in that the amino terminus alanine is not acetylated. The natural human SOD is acetylated at the amino terminus alanine (Hartz, J.W. and Deutsch, H.F., J. Biol. Chem. (1972) 247, 7043-7050, Jabusch, J.R., et al., Biochemistry (1980) 19, 2310-2316; Barra, et al., FEBS Letters (1980) 120, 53 and Oberly, L.W. Superoxide Dismutase, Vol. I, CRC Press, Florida, (1982), pp. 32-33). The natural human SOD is likely to be glycosylated like bovine SOD (Huber, W., U.S. Patent No. 3,579,495, issued May 18, 1971). Bacterial-produced human SOD is almost certainly not glycosylated as Escherichia coli does not glycosylate proteins which it produces. The amino acid sequence of the bacterial-produced SOD analog is identical to that of mature human SOD and does not contain a methionine residue at its N-terminus.

This invention provides a method for producing in bacteria and purifying an enzymatically active analog of human Cu/Zn SOD.

Superoxide dismutase is of considerable interest because of its pharmacological properties. Bovine-derived, naturally-occurring superoxide dismutase (orgotein) has been recognized to possess anti-inflammatory properties and is currently marketed in certain European countries, e.g., West Germany, for use in the treatment of inflammation. It is also sold in a number of countries including the United States as a veterinary product for treating inflammation, particularly for treating inflamed tendons in horses.

Additionally, the scientific literature suggests that SOD may be useful in a wide range of clinical applications. These include prevention of oncogenesis and tumor promotion and reduction of cytotoxic and cardiotoxic effects of anti-cancer drugs (Oberley, L.W. and Buettner, G.R., Cancer Research 39, 1141-1149 (1979)); protection of ischemic tissues (McCord, J.M. and Roy, R.S., Can. J. Physiol. Pharma. 60, 1346-1352 (1982)), and protection of spermatozoa (Alvarez, J.G. and Storey, B.T., Biol. Reprod. 28, 1129-1136 (1983)). In addition, there is a great interest in studying the effect of SOD on the aging process (Talmasoff, J.M., Ono, T. and Cutler, R.G., Proc. Natl. Acad. Sci. USA 77, 2777-2782 (1980)).

The recombinant human superoxide dismutase (hSOD) or analogs thereof can be used to catalyze the reduction of superoxide radicals in the presence of $H^+$, to hydrogen peroxide and molecular oxygen, to reduce reperfusion injury following ischemia and prolong the survival period of excised isolated organs and to reduce injury on reperfusion following organ transplantation and spinal cord ischemia.

## Summary of the Invention

This invention concerns a plasmid which upon introduction into a suitable bacterial host cell containing the thermolabile repressor $C_I$ renders the host cell capable, upon increasing the temperature of the host cell to a temperature at which the repressor is inactivated, of effecting expression of a desired eucaryotic gene inserted into the plasmid and production of a eucaryotic polypeptide encoded by the gene comprising:

a double-stranded DNA molecule which includes in 5' to 3' order the following:

a DNA sequence which contains the promoter and operator $P_LO_L$ from λ bacteriophage;

an N utilization site for binding antiterminator N protein;

a first restriction enzyme site permitting replacement of the DNA sequence containing the ribosomal binding site which follows thereafter;

a ribosomal binding site for rendering the mRNA of the desired gene capable of binding to ribosomes within the host cell selected from the group consisting of a mutant of $C_{II}$ from λ bacteriophage having the sequence:

TAAGGAAGTACTTACAT
ATTCCTTCATGAATGTA ,

a natural β-lactamase ribosomal binding site derived from pBR322 (ATCC Accession No. 37017), a synthetic oligonucleotide having the sequence:

AATTCGAGCGCAAGGAAACAGGCTCA
GCTCGCGTTCCTTTGTCCGAGTAT

and a synthetic oligonucleotide having the sequence:

AATTCAATAATATTGAAAAAGGAAGAG

GTTATTATAACTTTTTCCTTCTCAT ;

an ATG initiation codon; and

a second restriction enzyme site having inserted therein the desired gene encoding the eucaryotic polypeptide or analog thereof in phase with the ATG initiation codon;

and which additionally includes a DNA sequence which contains an origin of replication from a bacterial plasmid capable of autonomous replication in the host cell and a selection means selected from the group consisting of DNA sequences which contain a gene associated with a selectable or identifiable phenotypic trait which is manifested when the vector is present in the host cell and DNA sequences which contain the fragment designated cl[4 34], such fragment including the gene for the cl[4 34] repressor protein and its associated promoter and operator sequence, and wherein the distance between the 3' end of the $P_LO_L$ promoter and operator sequence and the 5' end of the N utilization site is less than about 80 base pairs and the distance between the 3' end of the N utilization site and the 5' end of the ribosomal binding site is less than about 300 base pairs.

Preferred vectors are those wherein the selection means is a gene associated with a phenotypic trait, such as pJH200 and pRO211.

The vectors may additionally include a DNA sequence containing a $T_1T_2$ rRNA sequence located 3' of the second restriction enzyme site. Desirably, the $T_1T_2$ rRNA transcription termination sequence is less than about 100 base pairs from the 3' end of the second restriction enzyme site, more desirably it is less than about 20 base pairs from the 3' end of the site.

The presently preferred vector containing the $T_1T_2$ rRNA transcription termination sequence is p579 wherein the selection means is a gene associated with a phenotypic trait.

According to the invention the vectors containing the cl[4 34] fragment, desirably, the cl[4 34] fragment is located 3' of the $T_1T_2$ rRNA termination sequence.

Desirably the vectors include both the gene associated with the phenotypic trait and the cl[4 34] fragment. The presently preferred vector containing both genes is p579 having the cl[4 34] fragment cloned into the ClaI site.

Vectors may also contain origin of replications from a bacterial plasmid capable of autonomous replication in the host cell and production of at least 400 constitutive copies of the vector.

The presently preferred origin of replication is pOPIΔ6 which is derived from a colE1 plasmid.

Preferred are vectors including the $T_1T_2$ rRNA termination sequence, an origin of replication capable of producing at least 400 constitutive copies in the host cell and containing both a gene for a phenotypic trait and a gene for the $cl^{434}$ fragment.

Genes, e.g., cDNAs, encoding desired polypeptides, such as growth hormones, e.g., bovine, porcine, chicken or human growth hormones, human superoxide dismutase, human apolipoprotein E or analogs thereof, may be inserted into the second restriction enzyme site of the vector to create plasmids. The plasmids in turn can be introduced into suitable hosts where the genes can be expressed and the desired polypeptide produced. The presently preferred plasmids are: for bGH, pRO12, pSAL 5200-6, pHG44, pSAL 5600-1, p7200-22, pHG50, p8300-10A, pSAL-170/10 pSAL-210/4 and p9200; for human superoxide dismutase, pSODα2, pSODβ1, pSODβ$_1$T$_{11}$, pSODβ$_1$-BA2 and pSODβ$_1$TT-1; for pGH, p3008 and p3009; for cGH, p5002 and p5003 for hGH, pTV 300; and for human apolipoprotein E, pTV-188, pSAL 160-5, pTV-170, pTV-190, pTV-194, pTV-214 and pTVR 279-8.

Preferred hosts include Escherichia coli A1637, A1645, A2602, A2097 and A1563 and A1645 (λi434cl⁻miniTn10) if the plasmid contains the $cl^{434}$ fragment. Preferred prototrophic hosts include E. coli A4200, A4255 and A4346. Preferred lytic hosts include E. coli A4048.

The resulting host vector systems can be employed to manufacture polypeptides. Host cells containing the plasmids are grown under suitable conditions permitting production of polypeptide and the resulting polypeptide is recovered. Using the host vector systems, analogs of human apolipoprotein E, bovine growth hormone, porcine growth hormone, chicken growth hormone, human growth hormone and human superoxide dismutase have been prepared.

SOD analogs so prepared have been incorporated into veterinary and pharmaceutical compositions containing the SOD analogs and suitable carriers.

These superoxide dismutase analogs have been used to catalyze the following reaction:

$$2O_2^{\bullet -} + 2H^+ \rightarrow H_2O_2 + O_2$$

More particularly, these analogs have been used to reduce injury caused by reperfusion following ischemia or organ transplantation, to reduce cardiac infarct size, or to increase the survival time of excised isolated organs. These analogs may also be to reduce spinal cord injury and for bronchial pulmonary dysplasia.

A method of producing enzymatically active eucaryotic superoxide dismutase or an analog thereof in a bacterial cell has also been discovered. The bacterial cell contains and is capable of expressing a DNA sequence encoding the superoxide dismutase or analog. The method comprises maintaining the bacterial cell under suitable conditions and in a production medium supplemented with an amount of $Cu^{++}$ so that the concentration of $Cu^{++}$ available to the cell in the medium is greater than about 2ppm.

According to the invention the bacterial cell is an Escherichia coli cell containing a plasmid which contains a DNA sequence encoding for an analog of human superoxide dismutase.

The enzymatically active eucaryotic superoxide dismutase or an analog thereof produced in the bacterial cell in accordance with the methods of this invention may be purified by a method which comprises isolating the bacterial cell from the growth medium and suspending the bacterial cell in a suitable solution having a pH from about 7.0 to about 8.0. The cell wall of the suspended bacterial cell is disrupted and the resulting homogeneous solution is then sonicated under suitable conditions. The sonicated solution is centrifuged under suitable conditions and the supernatant is removed and heated for about 2 hours at about 65°C. The supernatant is then cooled and centrifuged under suitable conditions to produce a clear supernatant protein solution. The resulting supernatant is concentrated to an appropriate volume and subjected to ion exchange chromatography on a suitable anion exchanger equilibrated at a pH from about 7.0 to about 8.0. The resulting solution containing the superoxide dismutase or analog is collected, concentrated to an appropriate volume and dialyzed against a buffered solution with a pH from about 7.0 to about 8.0. This concentrated buffered solution is subjected to ion exchange chromatography on a suitable anion exchanger equilibrated at a pH from about 7.0 to about 8.0 and the anion exchange-protein complex is subjected to a suitable salt gradient. Fractions containing the superoxide dismutase or analog are collected, concentrated and dialyzed against distilled water. The pH of the solution of interest is adjusted to a pH from about 4.0 to about 5.0 with a suitable buffer. The buffered solution is then subjected to ion exchange chromatography with a suitable cation exchanger equilibrated at a pH from about 4.0 to about 5.0. The protein-cation exchanger complex is subjected to a suitable salt gradient and the resulting fractions which contain the purified super-oxide dismutase or analog are collected.

Description of the Figures

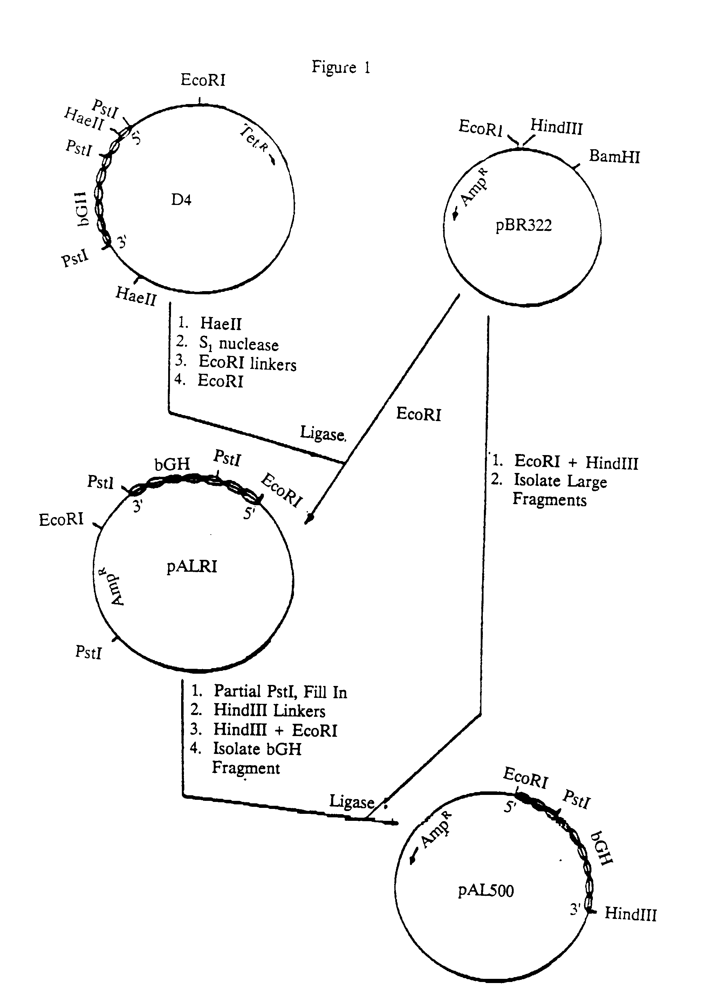

The restriction maps for each of the plasmids shown in Figures 1-31 do not identify all restriction sites present on each plasmid. In some cases restriction sites are shown in one figure but not in another. However, in all cases those restriction sites necessary for a complete understanding of the invention are shown.

Fig. 1. Construction of pAL500.

A plasmid containing bGH cDNA, D4 (ATCC No. 31826), was digested with HaeII. The resulting 1600 base pair large fragment was purified and digested at 37°C for 5 minutes with S1 exonuclease. A synthetic EcoRI linker with the sequence:

GGAATTCC

CCTTAAGG

was attached to the ends of the resulting fragments by ligation. The ligation mixture was cleaved with EcoRI and inserted into pBR322 (ATCC No. 37017) which had been cleaved with EcoRI. A clone, pALRI, was obtained which upon cleavage with EcoRI released a 1200 base pair fragment with the sequence:

AATTCCCAGCCATG....

GGGTCGGTAC....

at the 5' end. This sequence demonstrates that pALRI contains an EcoRI restriction site which includes the TTC codon for residue number 1 (phenylalanine) of natural bGH. pALRI was subjected to a partial cleavage with PstI. The digest was treated with DNA polymerase I large fragment (Klenow) and HindIII linkers with the sequence:

GAAGCTTC

CTTCGAAG

were attached by ligation. The ligation mixture was cleaved with EcoRI and HindIII. The fragment containing bGH cDNA was isolated and subcloned into pBR322 between the EcoRI and HindIII restriction sites to give pAL500 (ATCC No. 39782).

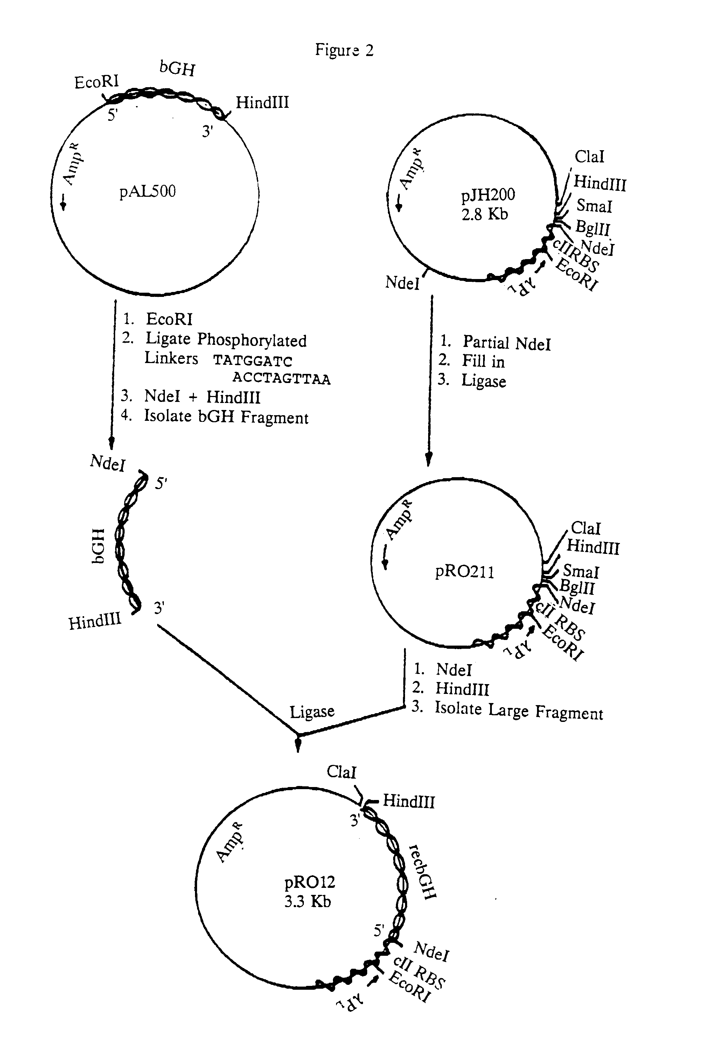

Fig. 2. Construction of pRO211 and pRO12.

The plasmid pJH200 (ATCC No. 39783) was partially digested with NdeI, treated with DNA polymerase I (Klenow) to fill in the ends and the resulting ends were religated to form the expression vector pRO211. The expression vector pRO211 was digested with NdeI and HindIII, the large fragment isolated and ligated to an NdeI-HindIII bGH fragment isolated from pAL500 (ATCC No. 39782) to give pRO12. (The NdeI-HindIII fragment was produced from pAL500 by digesting it with EcoRI and ligating to the ends of the digestion product synthetic linkers with the sequence:

TATGGATC

ACCTAGTTAA

8

The ligation mixture was digested with NdeI and HindIII and the resulting NdeI-HindIII bGH fragment isolated.)

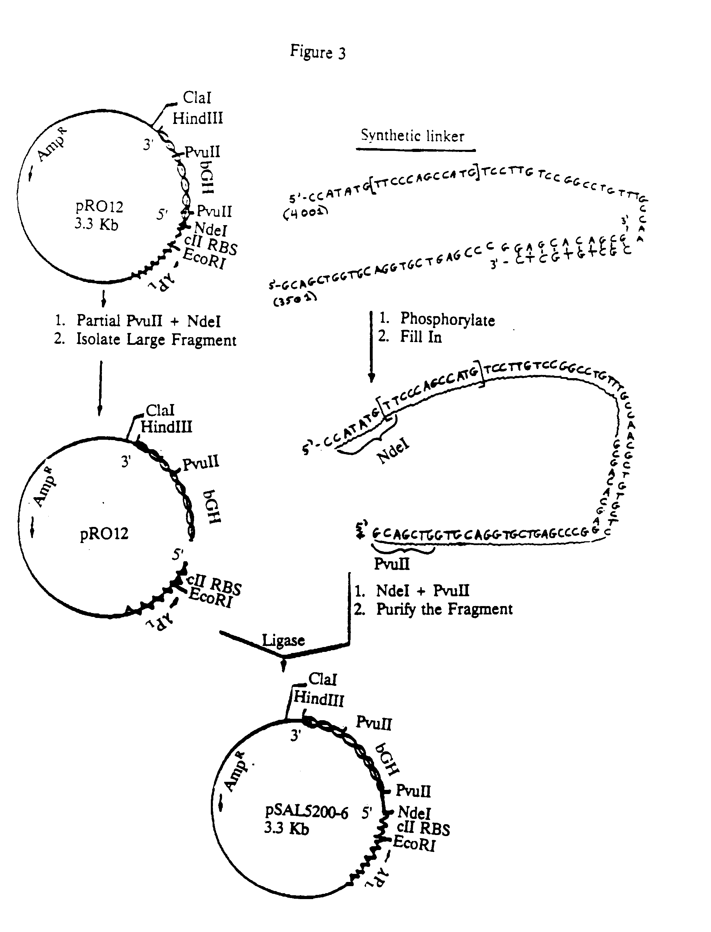

Fig. 3. Construction of pSAL 5200-6

pRO12 (Fig. 2) was partially digested with PvuII followed by digestion with NdeI to eliminate a 72 base pair fragment. A synthetic DNA fragment coding for the first 24 amino acids of the N-terminus of authentic bGH was ligated to the digested pRO12.

The synthetic DNA fragment was constructed by annealing two phosphorylated synthetic single-stranded DNAs of the sequence:

CCATATGTTCCCAGCCATGTCC–

–TTGTCCGGCCTGTTTGCCAACGCTGTGCTC–3'
                    3'–GCGACACGAGGCCCGAGTCGTGGACGTGGTCGACG

The annealed fragment was treated with DNA polymerase I (Klenow) in the presence of all four deoxyribonucleoside triphosphates in order to form the full length double-stranded DNA. The fragment was digested with PvuII and NdeI before ligation to pRO12 to form pSAL 5200-6.

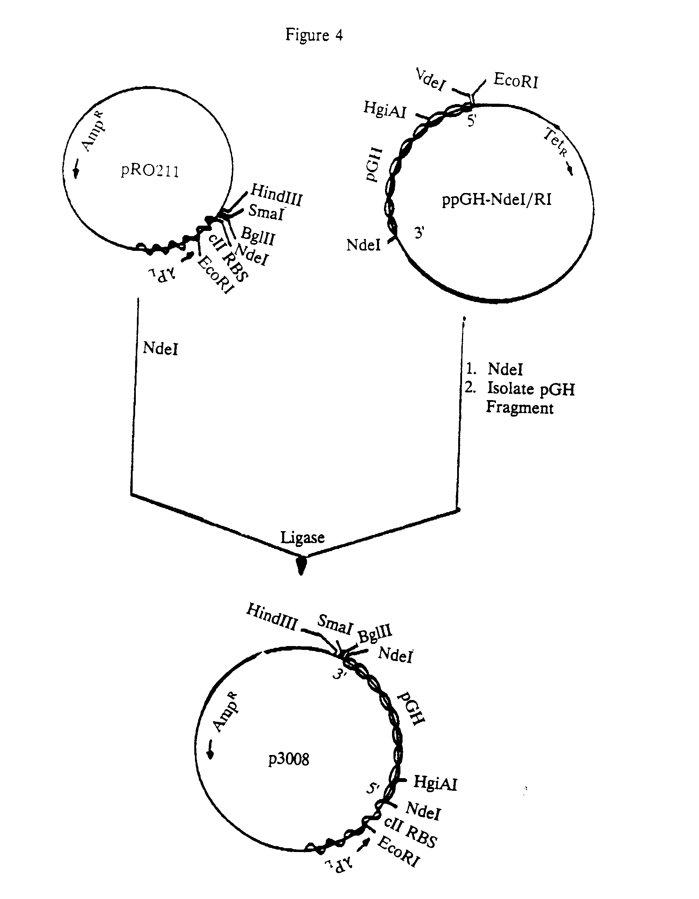

Fig. 4. Construction of p3008.

p3008 (ATCC No.39804) was constructed by ligating NdeI-digested pRO211 (Fig. 2) with the pGH fragment isolated from an NdeI digest of the plasmid ppGH-Ndel/RI.

ppGH-NdeI/RI contains full length pGR cDNA to both ends of which NdeI sites have been added by means of synthetic linkers.

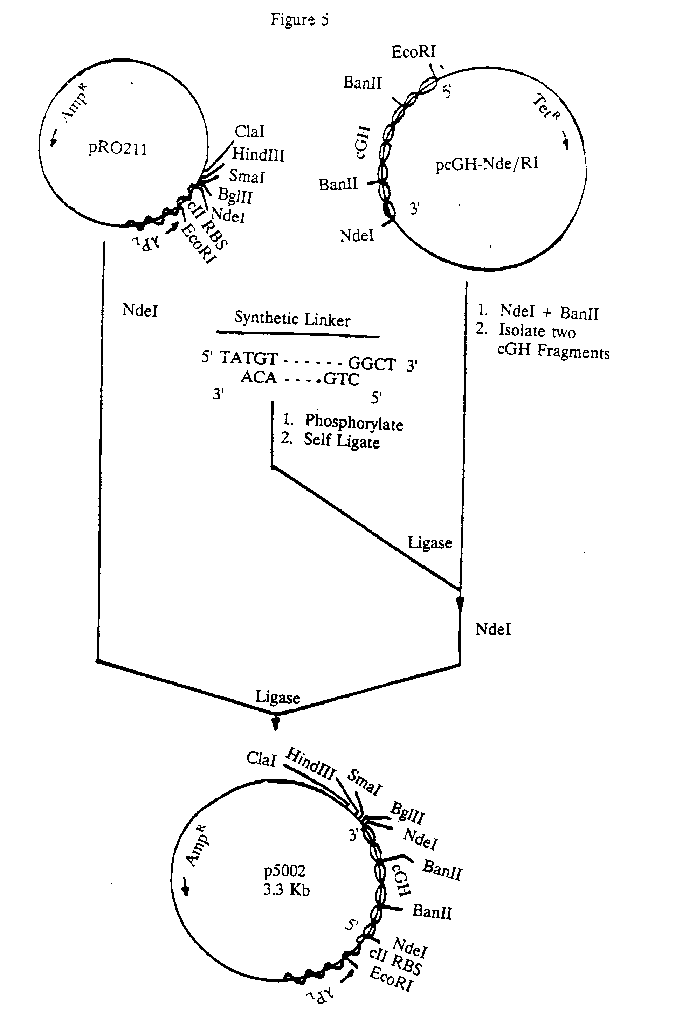

Fig. 5. Construction of p5002.

p5002 was constructed by tripartite ligation of a dimerized synthetic linker and the 2 cGH fragments isolated from an NdeI and BanII digest of the plasmid pcGH-NdeI/RI. The ligation mixture was digested with NdeI and then ligated to the expression vector pRO211 (Fig. 2) after it had been restricted with NdeI. A colony containing the plasmid p5002 was isolated.

The synthetic linker was constructed from two single-stranded synthetic DNAs of the sequence:

TATGTTCCCTGCCATGCCCCTCTCCAACCTGTTTGCCAACGCTGTGCTGAGGGCT

ACAAGGGACGGTACGGGGAGAGGTTGGACAAACGGTTGCGACACGACTC

The linker was phosphorylated before ligation. The linker codes for the first 18 amino acids of the N-terminus of the authentic cGH.

The plasmid pcGH-NdeI/RI contains full length cGH cDNA at the 5' end of which there is an EcoRI restriction site and at the 3' end of which there is an NdeI restriction site. These restriction sites were added by means of synthetic linkers.

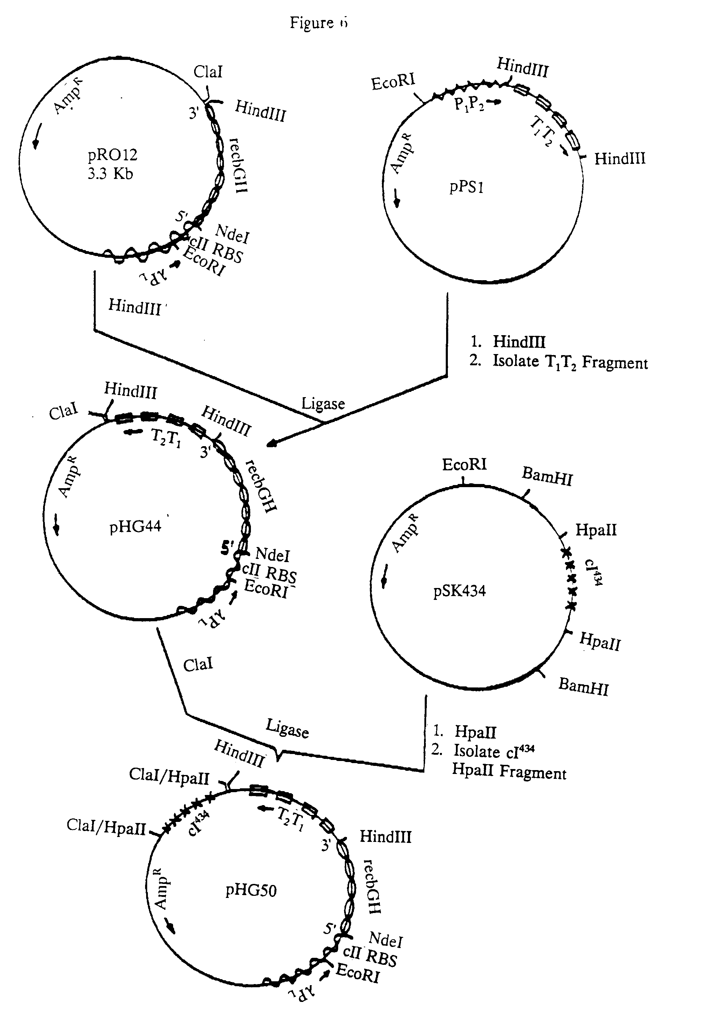

Fig. 6. Construction of pHG44 and pHG50.

pRO12 (Fig. 2) was digested with HindIII. The linear form DNA (form III) was purified from agarose gel and ligated to a HindIII-HindIII fragment of about 1200 base pairs which contains the rRNA operon transcription termination sequences $T_1T_2$. The $T_1T_2$ HindIII-HindIII fragment was isolated from plasmid pPS1 (ATCC No. 39807) which had been digested with HindIII. The resulting plasmid pHG44 (ATCC No. 39806) contains the $T_1T_2$ sequences at the 3' end of the recombinant (rec) bGH sequence.

The plasmid pSK434 (ATCC No. 39784) containing the $\lambda cI^{434}$ repressor sequences was digested with HpaII. The $\lambda cI^{434}$ HpaII-HpaII fragment was isolated and ligated to pHG44 which had been digested with ClaI. The resulting plasmid pHG50 (ATCC No. 39805) contains the $T_1T_2$ transcription termination sequences

and the $\lambda cl^{4\,34}$ repressor sequence.

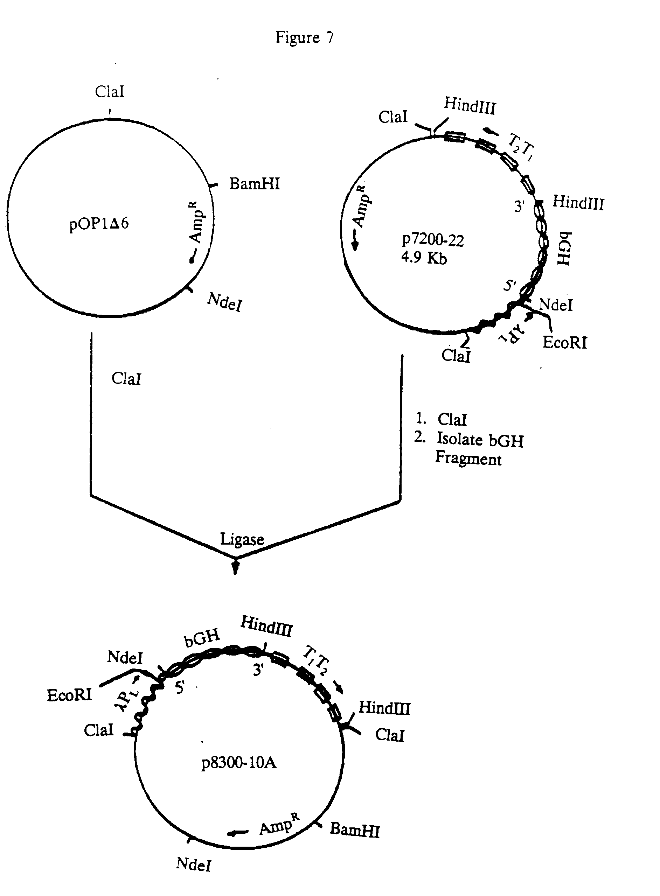

Fig. 7. Construction of p8300-10A.

The plasmid p8300-10A (ATCC No. 39785) which expresses an analog of the natural phenylalanine form of bGH having methionine at the N-terminus (met-phe bGH) was prepared as follows. The plasmid p7200-22 contains the $\lambda P_L$ promoter and ribosomal binding site derived from pJH200 (ATCC No. 39783), DNA encoding met-phe bGH and the $T_1T_2$ rRNA termination sequences. The ClaI-ClaI fragment containing the $\lambda P_L$ promoter, the $C_{II}$ ribosomal binding site, the met-phe bGH gene and the $T_1T_2$ transcription termination sequences was inserted into the unique ClaI site of plasmid pOPI∆6, a constitutive high copy number plasmid, to form p8300-10A.

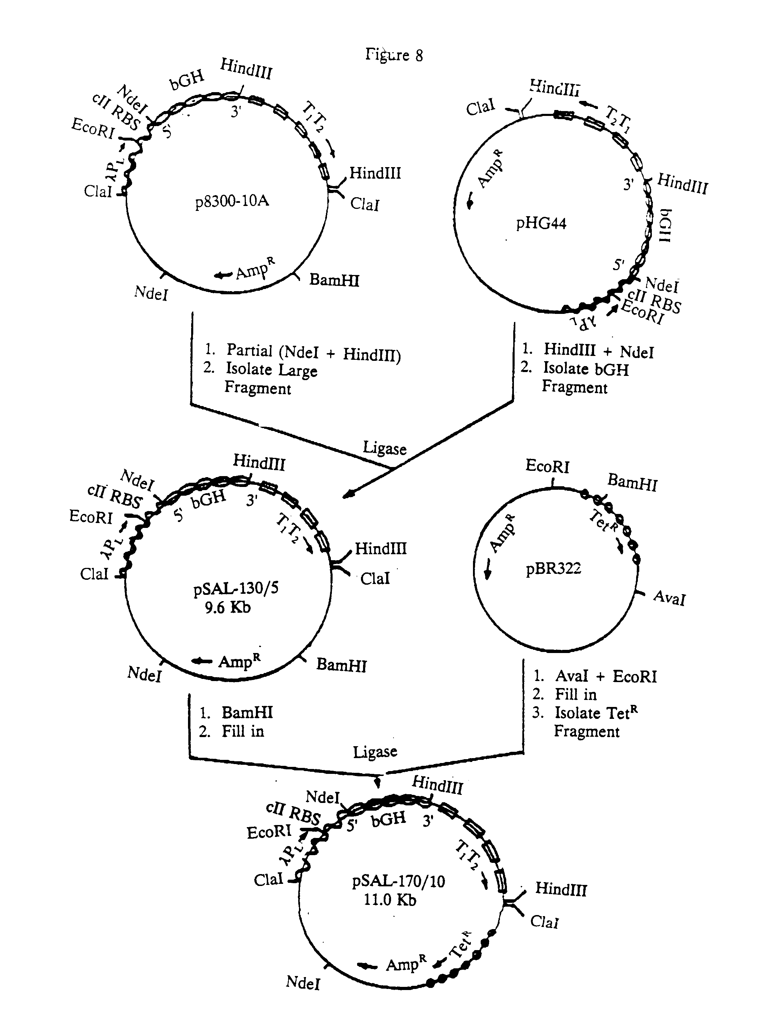

Fig. 8. Construction of pSAL-130/5 and pSAL-170/10.

The plasmid pHG44 (ATCC No. 39806) expressing met-asp-gln bGH protein was digested with NdeI and HindIII. The resulting NdeI-HindIII bGH fragment was isolated and ligated to a fragment from p8300-10A (ATCC No. 39785) prepared by partial digestion with both NdeI and HindIII. Such a ligation replaces the met-phe bGH gene fragment with the met-asp-gln bGH gene fragment. The plasmid so obtained, pSAL-130/5, expresses rec bGH. pSAL-170/10 was obtained by treating the EcoRI-AvaI fragment containing the $Tet^R$ gene of pBR322 plasmid (ATCC No. 37017) with DNA polymerase I (Klenow) and inserting it into pSAL-130/5 which had been digested with BamHI and filled in with DNA polymerase I (Klenow).

Fig. 9. Construction of pSAL-210/4.

Linear form DNA (form III) was prepared by partial ClaI digestion of pSAL-170/10. It was purified from an agarose gel and ligated to a HpaII-HpaII $cl^{4\,34}$ gene fragment which was isolated from a HpaII digest of the plasmid pSK434 (ATCC No. 39784).

Fig. 10. Construction of pSAL 5600-1.

pSAL 5200-6 (Fig. 3) was digested with HindIII. The linear form DNA (form III) was purified from an agarose gel and ligated to a HindIII-HindIII fragment of about 1200 base pairs which contains the rRNA operon transcription termination sequences, $T_1T_2$. The $T_1T_2$ HindIII-HindIII fragment was isolated from the plasmid pPSI (ATCC No. 39807) which was digested with HindIII. The resulting plasmid pSAL 5600-1 contains the $T_1T_2$ sequences at the 3' end of the met-asp-gln bGH sequence.

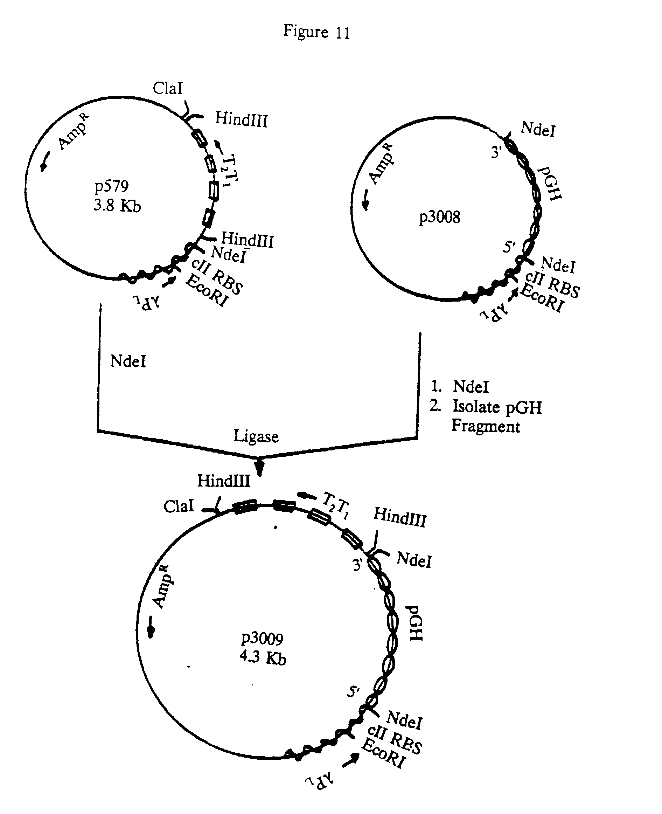

Fig. 11. Construction of p3009.

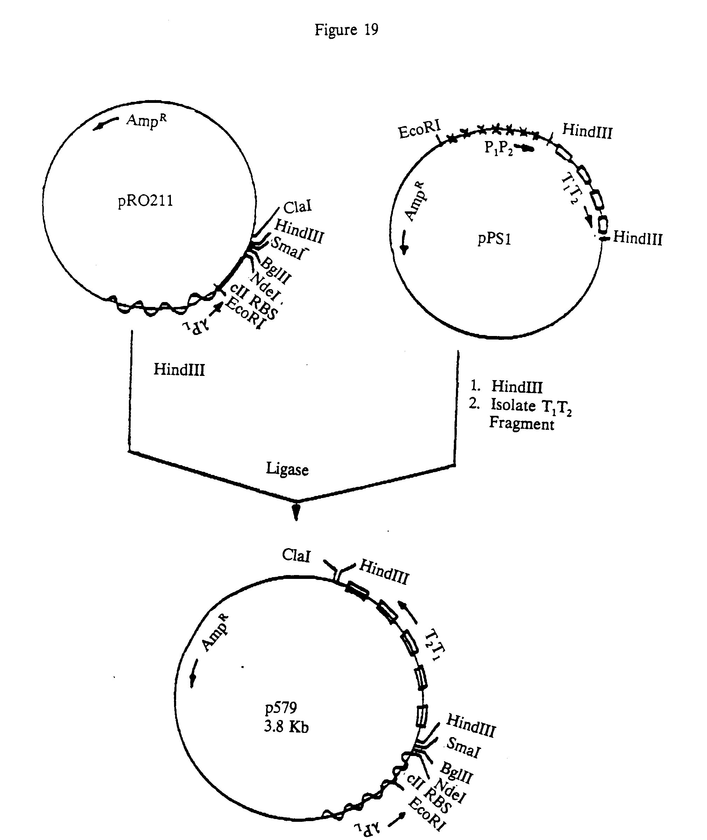

The NdeI-NdeI pGH fragment was isolated from plasmid p3008 (ATCC No. 39804) (Fig. 4). The fragment was inserted into the unique NdeI site of the expression vector p579 (Fig. 19) which had been digested with NdeI. The resulting plasmid p3009 expresses an analog of natural porcine growth hormone protein having a methionine residue added at the N-terminus.

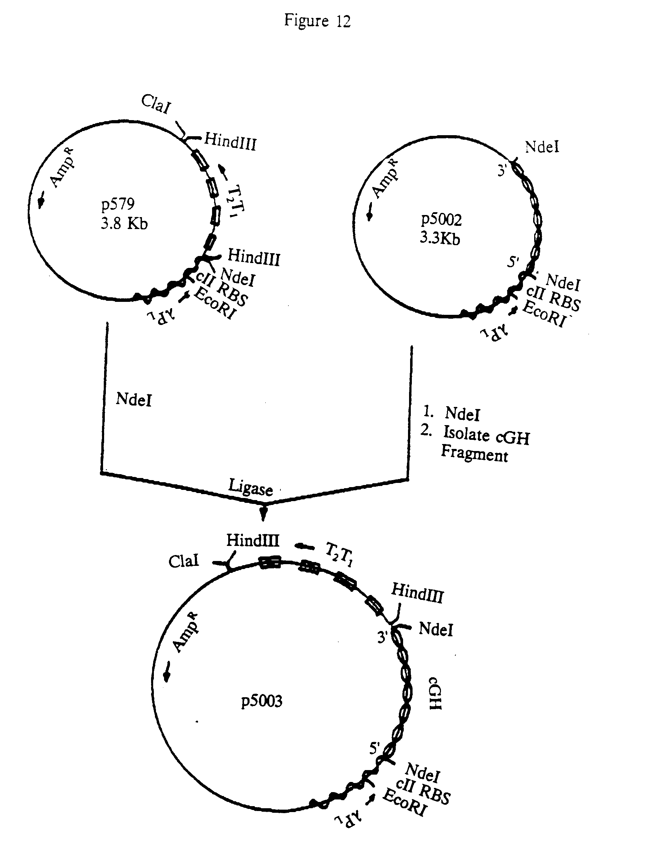

Fig. 12. Construction of p5003.

The NdeI-NdeI cGH fragment was isolated from plasmid p5002. The fragment was inserted into the unique NdeI site of the expression vector p579 (Fig. 19) which had been digested with NdeI. The resulting plasmid p5003 (ATCC No. 39792) expresses an analog of natural chicken growth hormone protein having a methionine residue added at the N-terminus.

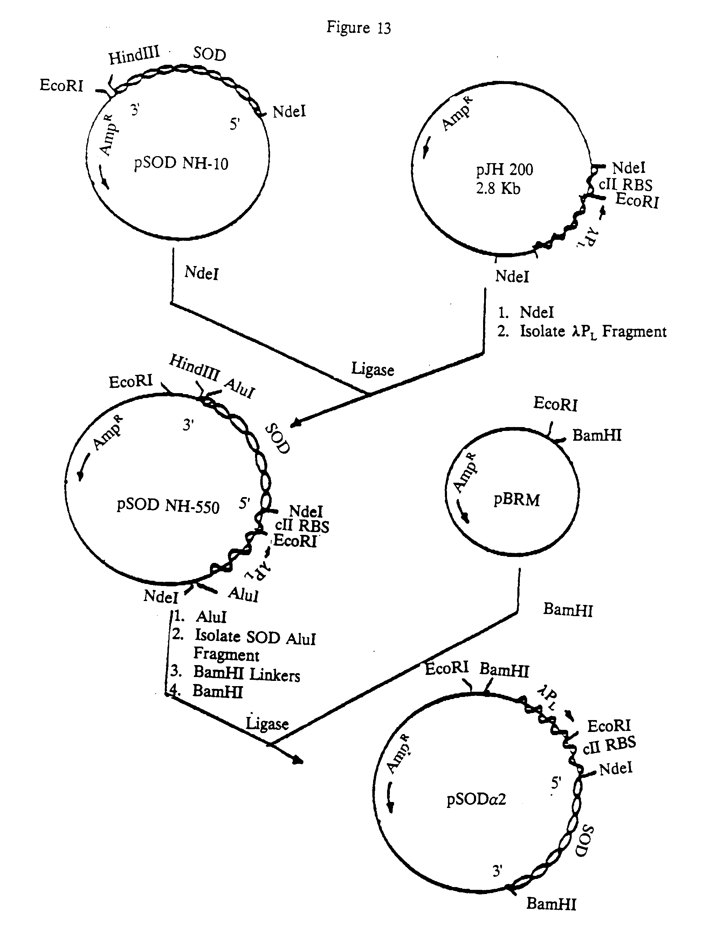

Fig. 13. Construction of pSODα2.

The pJH200 (ATCC No. 39783) expression vector was digested with NdeI. The 550 base pair NdeI fragment containing the $\lambda P_L$ promoter and $C_{II}$ ribosomal binding site was isolated and inserted into the unique NdeI site of plasmid pSOD NH-10 which had been digested with NdeI. (Plasmid pSOD NH-10 is derived from a cDNA clone of human SOD [Lieman-Hurwitz, J., et al., PNAS (1982) 79: 2808 ]) The resulting plasmid pSOD NH-550 was digested with AluI. (Only the relevant AluI site is shown in the figure.) The large AluI fragment containing the $\lambda P_L$ promoter and the SOD gene was isolated. BamHI linkers were attached and the resulting fragment was digested with BamHI. The BamHI digestion product was inserted into the unique BamHI site of pBRM (ATCC No. 37283) to form pSODα2 (ATCC No. 39786).

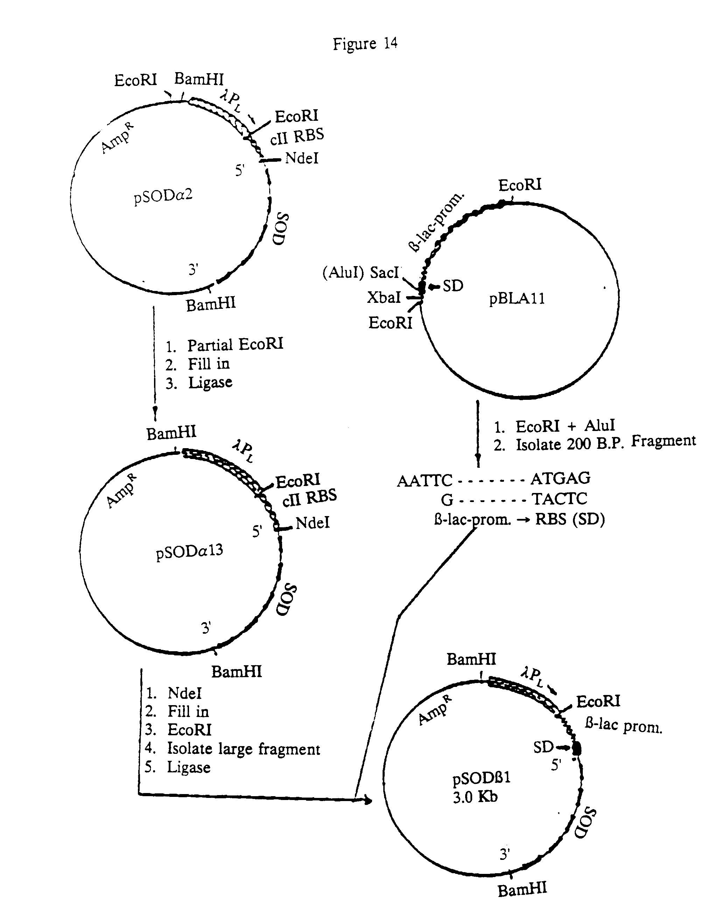

Fig. 14. Construction of pSODα13 and pSODβ1.

The plasmid pSODα2 (ATCC No. 39786) was partially digested with EcoRI and the resulting linear form DNA was isolated from an agarose gel. The purified DNA was filled in with DNA polymerase I (Klenow) and religated. The resulting clone pSODα13 contains one EcoRI site located at the 5' end of the ribosomal binding site. A fragment containing the β-lactamase promoter and ribosomal binding site was isolated from plasmid pBLA11 (ATCC No. 39788) which had been digested with EcoRI and AluI. The 200 base pair fragment was ligated to the large fragment isolated from pSODα13 which had been digested with NdeI, filled in with DNA polymerase I (Klenow) and then digested with EcoRI. The resulting plasmid pSODβ1 contains the ribosomal binding site of the β-lactamase gene and the λP$_L$ promoter.

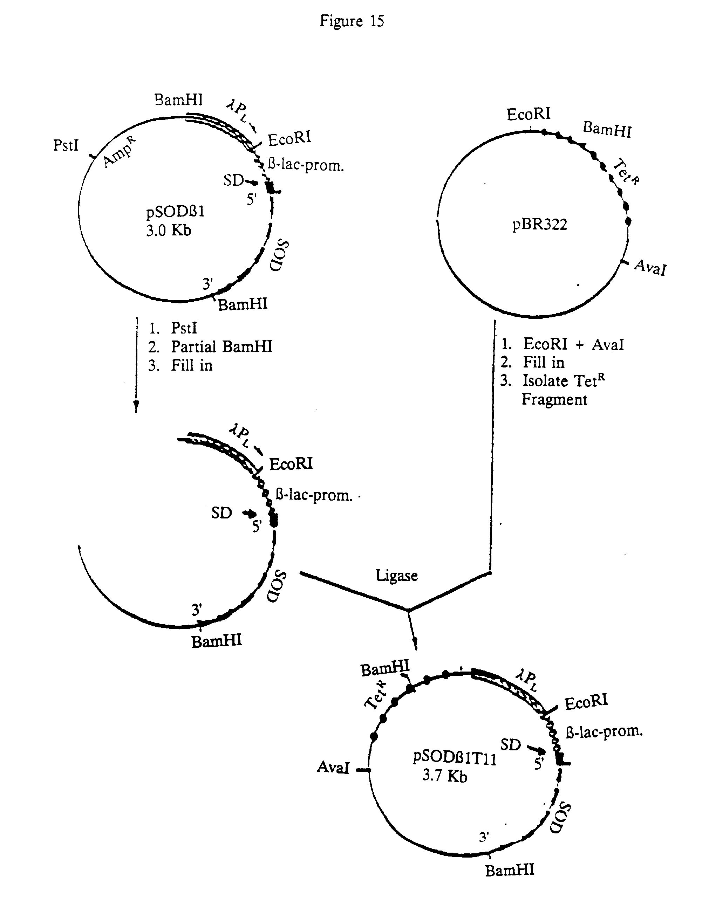

Fig. 15. Construction of pSODβ$_1$T$_{11}$.

Plasmid pBR322 (ATCC No. 37017) was digested with EcoRI and AvaI. The resulting DNA was filled in with DNA polymerase I (Klenow). The Tet$^R$ gene fragment was then isolated and ligated to the large fragment isolated from pSODβ1 (Fig. 14) plasmid which had been digested with PstI followed by a partial BamHI digest and then filled in with DNA polymerase I (Klenow). The resulting plasmid pSODβ$_1$T$_{11}$ contains the Tet$^R$ gene.

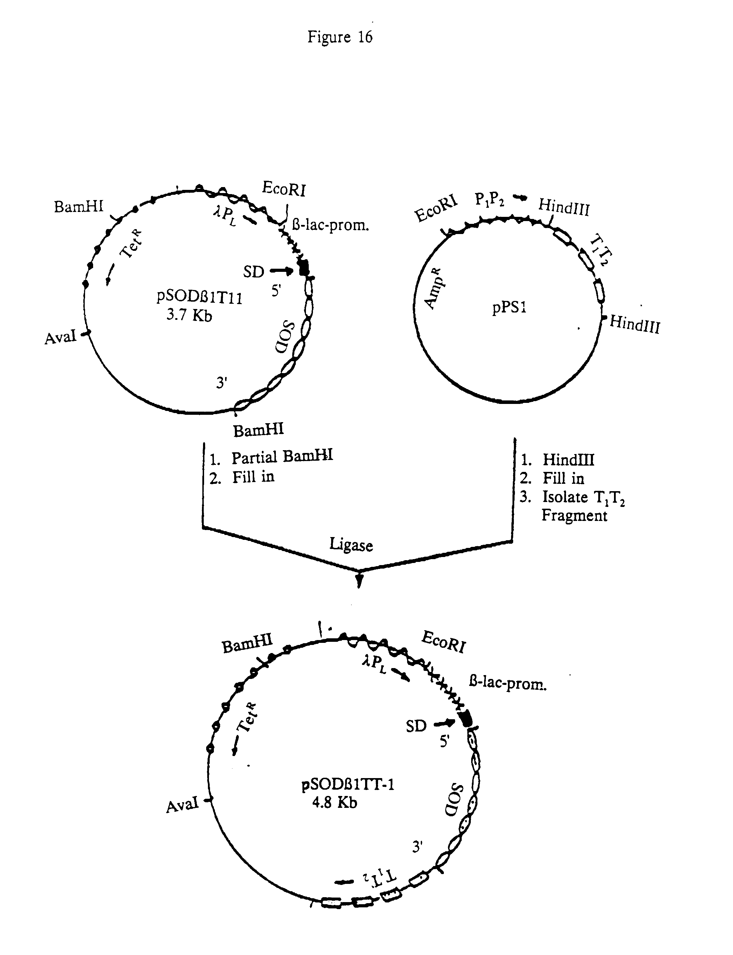

Fig. 16. Construction of pSODβ$_1$TT-1.

The rRNA T$_1$T$_2$ transcription termination fragment was isolated from plasmid pPS1 (ATCC No. 39807) which had been digested with HindIII and filled in with DNA polymerase I (Klenow). The fragment was ligated to plasmid pSODβ$_1$T$_{11}$ (Fig. 15) which had been partially digested with BamHI and filled in with DNA polymerase I (Klenow).

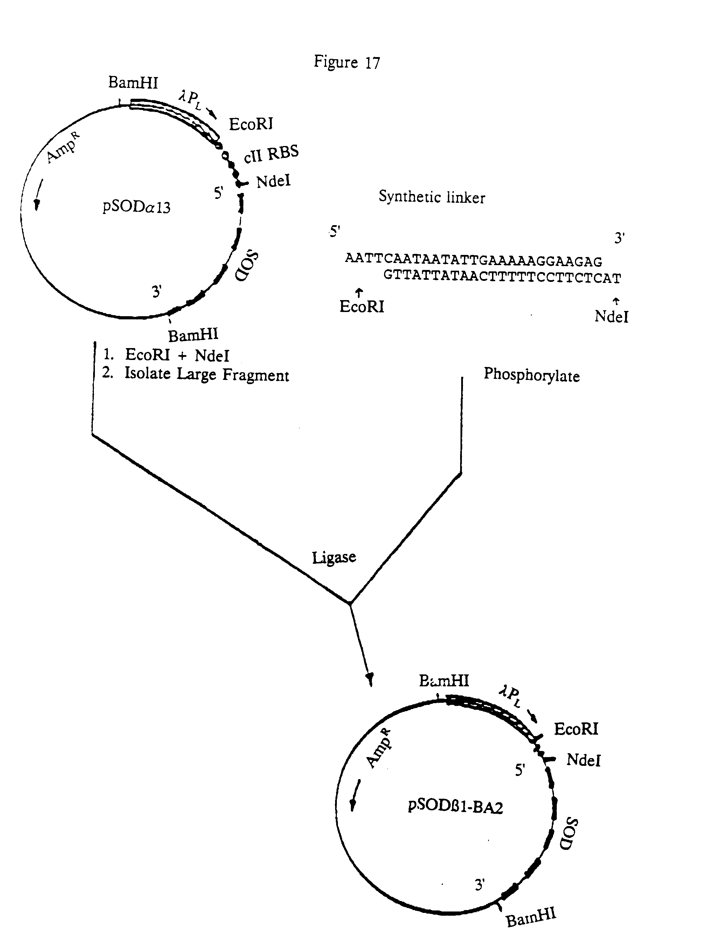

Fig. 17. Construction of pSODβ$_1$-BA2.

A synthetic DNA fragment with the sequence:

$$5'-AATTCAATAATATTGAAAAAGGAAGAG-3'$$
$$GTTATTATAACTTTTTCCTTCTCAT$$

which is similar to the sequence of the natural β-lactamase ribosomal binding site, was phosphorylated and ligated to the large fragment of pSODα13 plasmid (Fig. 14) which had been digested with NdeI and EcoRI.

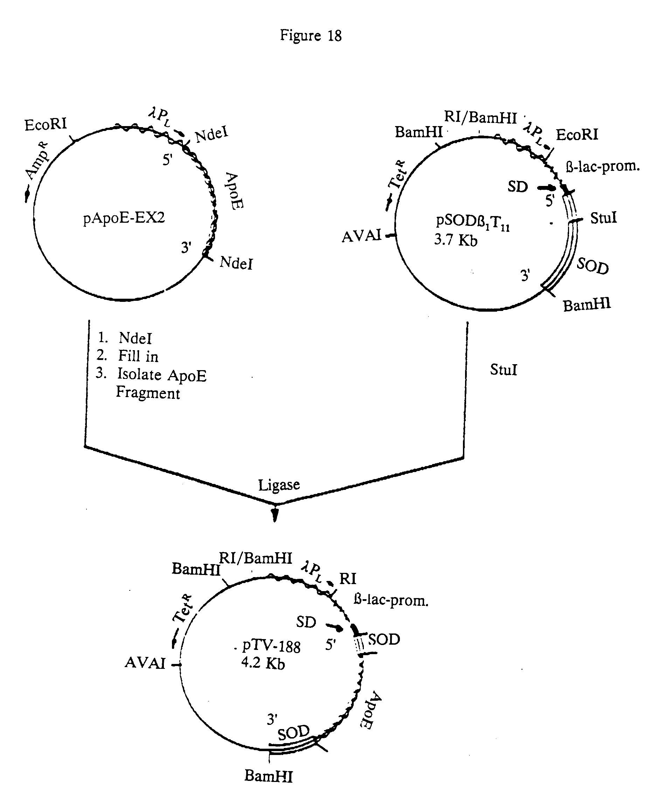

Fig. 18. Construction of pTV-188.

Plasmid pApoE-EX2 (ATCC No. 39787) was digested with NdeI and then fragments filled in with DNA polymerase I (Klenow). The resulting ApoE gene fragment was isolated and inserted into the unique blunt end StuI site of the pSODβ$_1$T$_{11}$ plasmid (Fig. 15). The resulting plasmid pTV-188 expresses an ApoE fused protein.

Fig. 19. Construction of p579.

The rRNA operon T$_1$T$_2$ transcription termination fragment was isolated from plasmid pPS1 (ATCC No. 39807) which had been digested with HindIII. The T$_1$T$_2$ fragment was inserted into the unique HindIII site of pRO211 (Fig. 2) which had been digested with HindIII. The resulting expression vector, p579, contains the λP$_L$ promoter, the C$_{II}$ ribosomal binding site, followed by the T$_1$T$_2$ transcription termination signals.

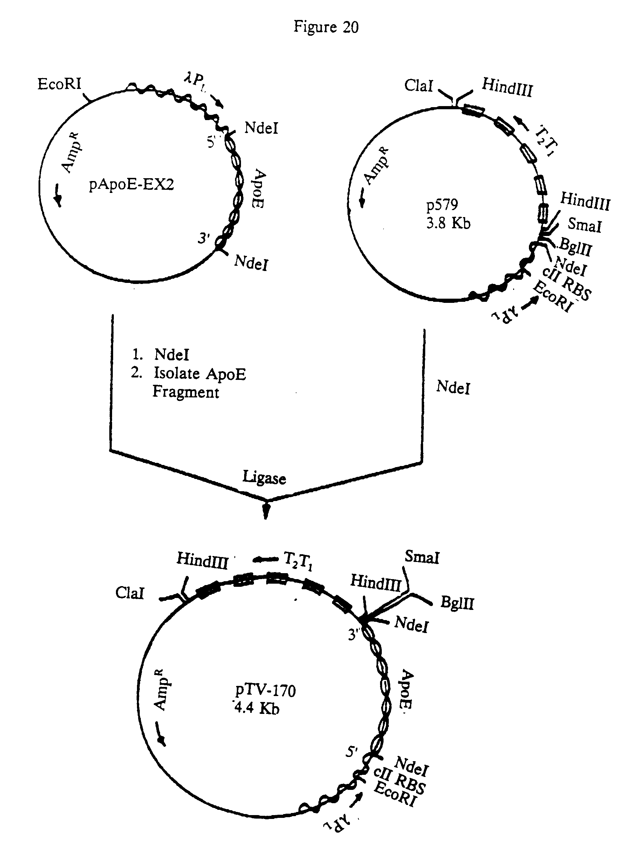

Fig. 20. Construction of pTV-170.

The NdeI-NdeI ApoE fragment was isolated from plasmid pApoE-EX2 (ATCC No. 39787) and inserted into the unique NdeI site of the expression vector p579 (Fig. 19) which had been digested with NdeI. The resulting plasmid pTV-170 expresses an analog of natural human ApoE protein having a methionine residue added at the N-terminus.

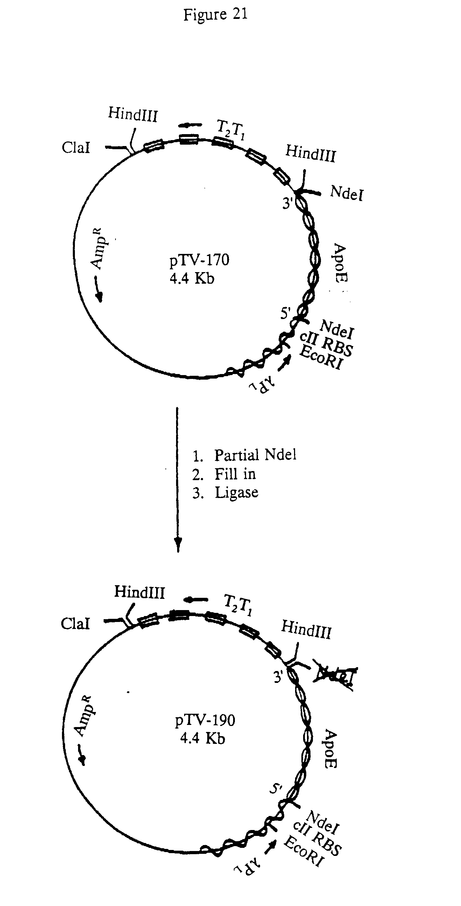

Fig. 21. Construction of pTV-190.

The plasmid pTV-170 (Fig. 20) was partially digested with NdeI and filled in with DNA polymerase I (Klenow). The isolated linear form DNA was religated to yield the plasmid pTV-190 which was analyzed and found to have only one NdeI site at the 5' end of the ApoE gene.

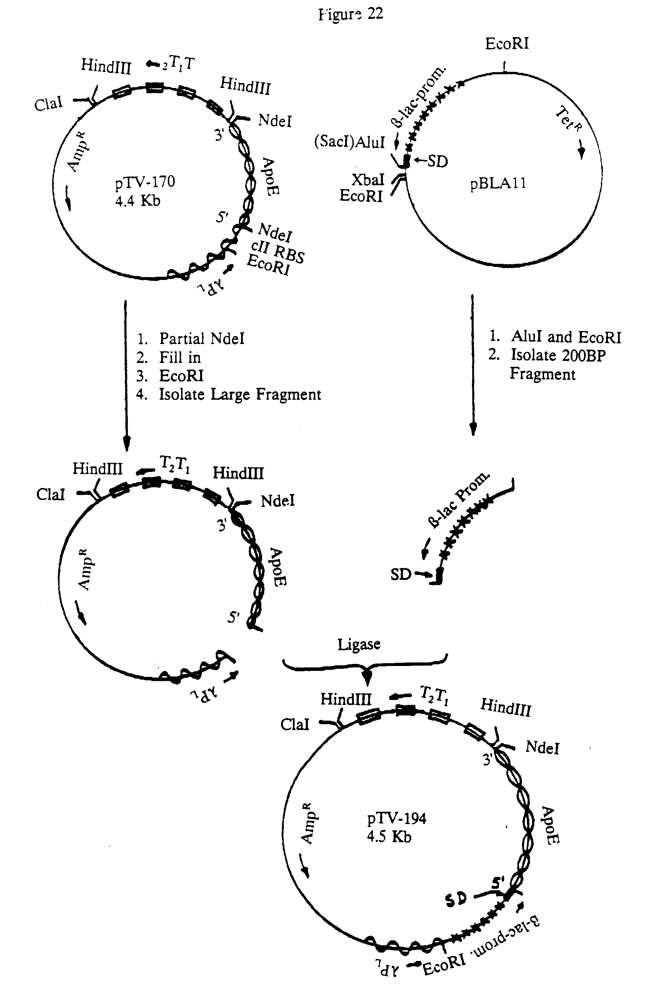

Fig. 22. Construction of pTV-194.

The β-lactamase promoter and ribosomal binding site fragment was isolated from plasmid pBLA11 (ATCC No. 39788) after digestion with EcoRI and AluI. This fragment was ligated to the large fragment of pTV-170 (Fig. 20) plasmid which had been digested with NdeI, filled in with DNA polymerase I (Klenow) and then digested with EcoRI.

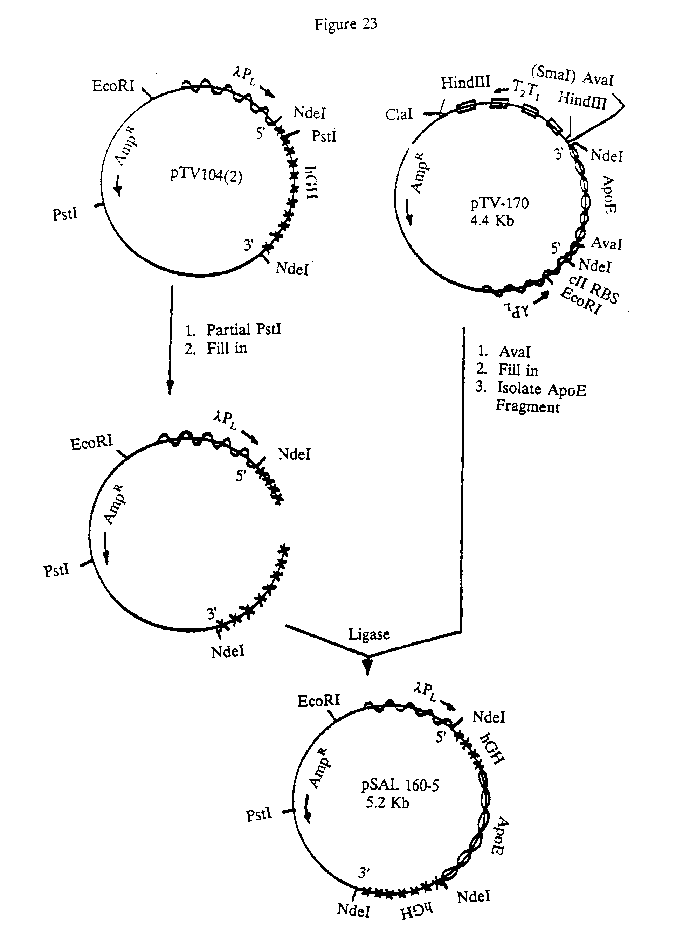

Fig. 23. Construction of pSAL 160-5.

An AvaI-AvaI fragment containing the ApoE DNA sequence was isolated from pTV-170 (Fig. 21) which was digested with AvaI. The fragment was filled in with DNA polymerase I (Klenow) and isolated on agarose gel. The purified ApoE fragment was incerted into the PstI site of the pTV 104(2) plasmid (ATCC No. 39384) which was partially digested with PstI and filled in with DNA Polymerase I (Klenow). The resulting plasmid is designated pSAL 160-5.

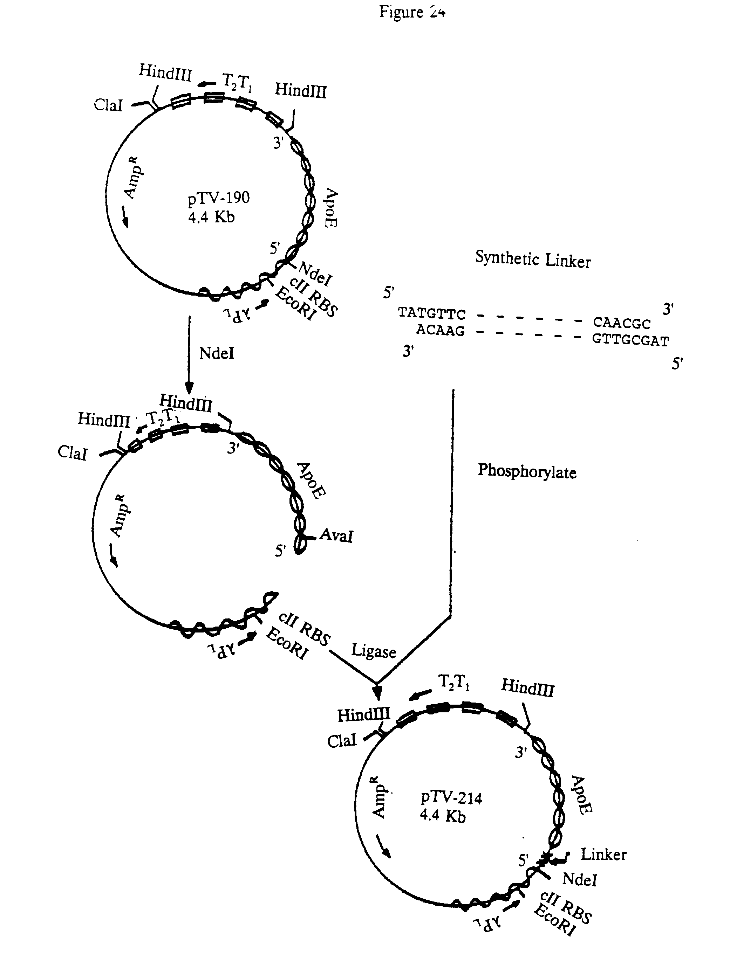

Fig. 24. Construction of pTV-214.

A synthetic fragment containing the methionine followed by the first 13 amino acids of human growth hormone with the sequence:

**TATGTTCCCAACCATTCCATTATCCCGTCTGTTCGACAACGC**

**ACAAGGGTTGGTAAGGTAATAGGGCAGACAAGCTGTTGCGAT**

was phosphorylated using $\gamma^{-32}$P-ATP and polynucleotide kinase. The phosphorylated linker was inserted into the unique NdeI site of pTV 190 plasmid which had been digested with NdeI.

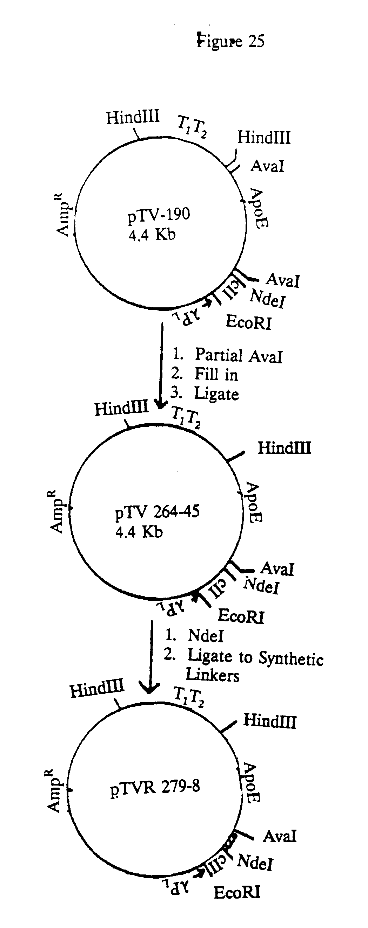

Fig. 25. Construction of pTVR 279-8.

pTVR 279-8 was constructed from pTV 264-45 which was constructed from pTV 190.

Plasmid pTV 190, which directs the expression of Met-ApoE3 analog was partially cleaved with AvaI, "filled in" using the Klenow fragment of DNA polymerase I and religated. The resulting plasmid, designated pTV 264-45 is deleted of the AvaI site at the 3' end of the gene.

Plasmid pTV 264-45 was digested to completion with NdeI and ligated to phosphorylated synthetic linkers of the sequence:

**5'-TATGCTGCTGCT**

**ACGACGACGAAT-5'**

The resulting plasmid designated pTVR 279-8 directs the expression of a met-leu-leu-leu-met-ApoE3 analog.

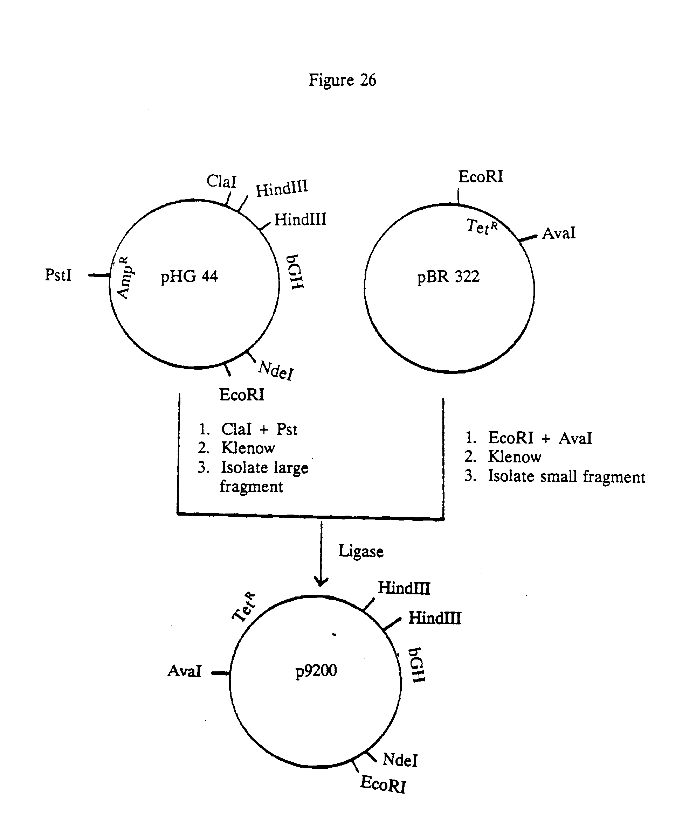

Fig. 26. Construction of p9200.

Plasmid p9200 was constructed by eliminating most of the ampicillin resistance gene from pHG44 and

EP 0 173 280 B1

replacing it with the tetracycline resistance gene of pBR 322.

pHG44 was cleaved with ClaI and PstI treated with the Klenow fragment of DNA polymerase I, and the large DNA fragment was isolated. This fragment was ligated to the small DNA fragment of pBR322 isolated after cleaving pBR322 with EcoRI and AvaI and treating with the Klenow fragment. The plasmid resulting from the ligation was designated p9200.

Plasmid p9200 directs the expression of met-asp-gln-bGH analog and confers tetracycline resistance to its host cell.

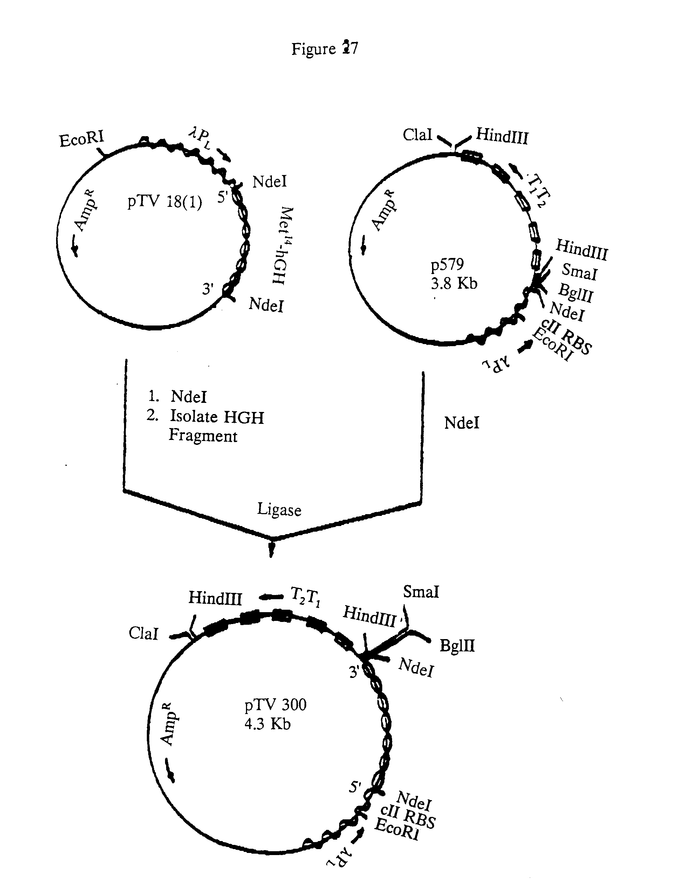

Fig. 27. Construction of pTV 300.

pTV 300 directs the expression of met[14]-hGH analog.

pTV 300 was constructed by cleaving pTV 18(1) with NdeI, isolating the met[14]-hGH DNA and ligating it to p579 (Fig. 19) cleaved with NdeI.

pTV 18(1) may be obtained as described in European Patent Application Publication No. 0 131 843 A1, published January 23, 1985 or as described in corresponding U.S. patent application Serial No. 514,188, filed July 15, 1983, the latter of which is hereby incorporated by reference.

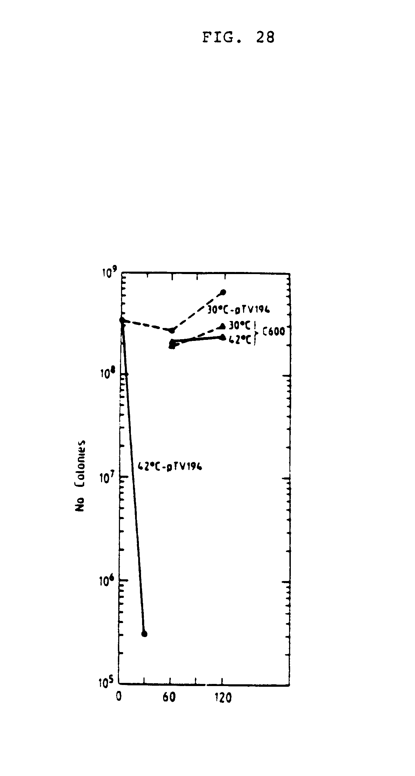

Fig. 28. Cellular Toxicity Associated With Intracellular Accumulation of ApoE Analog.

Cultures of C600 cells (5 ml) containing pTV 194 or non-transfected control cells were induced by raising the incubation temperature from 30 to 42°C. At the indicated times a 1 ml aliquot of the culture was removed, rapidly cooled with ice, serially diluted in growth medium, plated on agar in the presence of appropriate antibiotics and incubated overnight at 30°C. The number of colonies was determined from the average of duplicate plates. Parallel cultures of pTV 194 transfected and nontransfected C600 cells maintained at 30° served as noninduced controls.

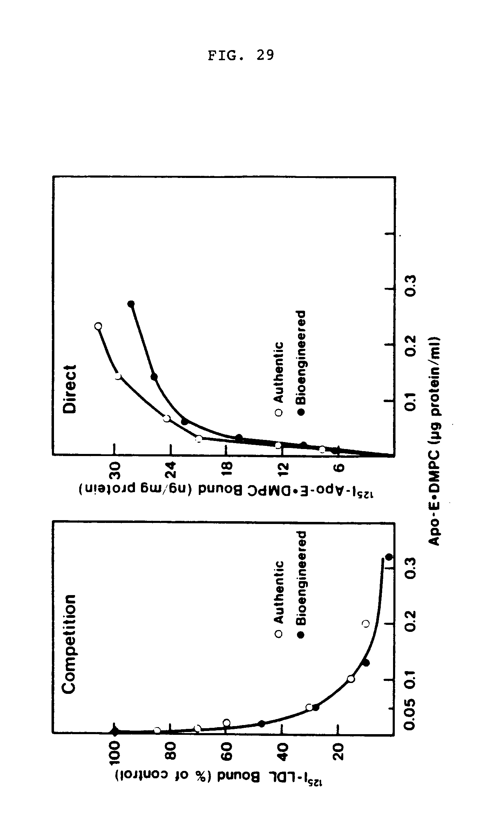

Fig. 29. Binding of ApoE DMPC Complexes to Apo-B,E(LDL) Receptors on Fibroblasts.

Phospholipid complexes of bioengineered (met-ApoE3 analog) and authentic ApoE were prepared by incubation of the proteins and DMPC at 22°C. The complexes were separated from noncomplexed material by density gradient ultracentrifugation as described (30, following Example 38). Left: Ability of bioengineered and authentic ApoE•DMPC complexes to compete with $^{125}$I-labeled human LDL for binding to cultured fibroblast receptors at 4°C. Right: Ability of $^{125}$I-labeled ApoE analog and authentic ApoE DMPC complexes to bind directly to cultured fibroblasts.

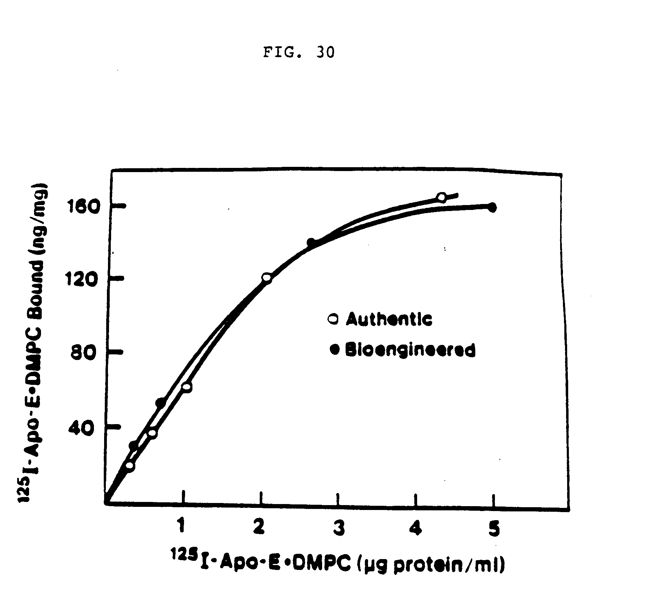

Fig. 30. Binding of $^{125}$I-ApoE DMPC Complexes to ApoE Receptors on Hepatic Membranes.

Phospholipid (DMPC) complexes were prepared as described in Fig. 29. Hepatic membranes from adult cholesterol-fed dogs served as the source for the ApoE receptors and were prepared as described (32, following Example 38). The binding of the $^{125}$I-labeled bioengineered and authentic ApoE DMPC complexes to the membranes was performed at 4°Cas described (32, following Example 38).

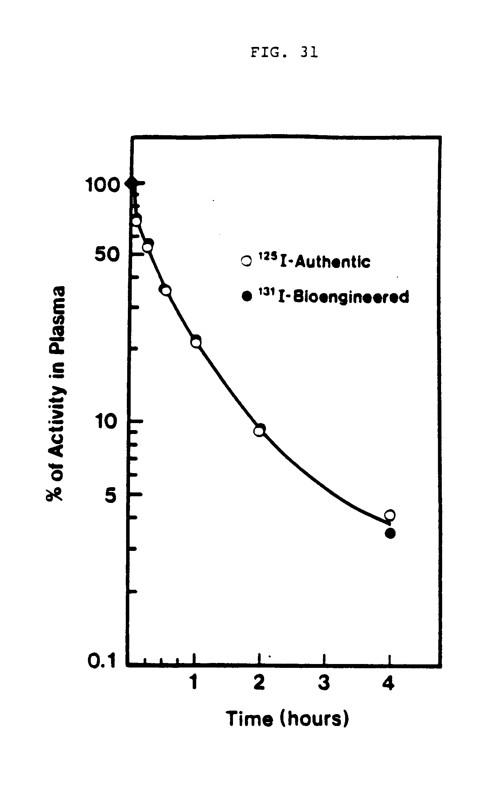

Fig. 31. Clearance of Iodinated ApoE from Rabbit Plasma.

Bioengineered and authentic ApoE were incubated at 37° for 30 minutes with 1 ml of rabbit plasma prior to injection of the mixture into a vein of a rabbit. Approximately 2 ml of blood was removed into a vial, containing EDTA, at the indicated time points and plasma prepared for counting. Counts are corrected for TCA soluble degradation products as described (33, following Example 38).

Detailed Description of the Invention

The vector included by the plasmid of the invention also includes a DNA sequence which contains an origin of replication from a bacterial plasmid capable of automomous replication in the host cell and a selection means selected from the group consisting of DNA sequences which contain a gene associated with a selectable or identifiable phenotypic trait which is manifested when the vector is present in the host cell and DNA sequences which contain the fragment designated cl[4 34], such fragment including the gene for the cl[4 34] repressor protein and its associated promoter and operator sequence. cl[4 34] represses a cl[4 34]-lysogen; loss of the plasmid will result in cell lysis. The distance between the 3' end of the $P_LO_L$ promoter

13

and operator sequence and the 5' end of the N utilization site is less than about 80 base pairs and the distance between the 3' end of the N utilization site and the 5' end of the ribosomal binding site is less than about 300 base pairs.

An optional additional component of the vector is a $T_1 T_2$ rRNA transcription termination sequence located 3' of the second restriction enzyme site. Preferably, the $T_1 T_2$ rRNA transcription termination sequence is less than about 100 base pairs from the 3' end of the second restriction enzyme site. More preferably, the $T_1 T_2$ rRNA transcription termination sequence is less than about 20 base pairs from the 3' end of the second restriction enzyme site.

The vector includes either a DNA sequence containing a gene associated with a selectable or identifiable phenotypic trait which is manifested when the vector is present in the host cell or a DNA sequence containing the $cI^{434}$ repressor gene which represses a $\lambda imm434cI^-$ lysogen or both.

Suitable genes associated with a selectable or identifiable phenotypic trait include those associated with temperature sensitivity or drug resistance, e.g., resistance to ampicillin, chloroamphenicol or tetracycline. When the $cI^{434}$ repressor gene is contained within a host, the host is prevented from $\lambda$ imm434cI⁻ prophage induction. Thus, there is no need to use expensive antibiotic selection salines when $cI^{434}$ is present.

Preferably, the vector includes both a DNA sequence which contains a gene associated with a selectable or identifiable phenotypic trait and a DNA sequence which contains a fragment designated $cI^{434}$.

Desirably, when the $cI^{434}$ gene is included on the vector, it is located after the 3' end of the $T_1 T_2$ rRNA sequence.

The vector also includes an origin of replication from a bacterial plasmid capable of autonomous replication in the host cell. Suitable such origins of replication may be obtained from a number of sources, e.g., from pBR322 or pR1.

Preferably, the vector has an origin of replication from a constitutive high copy number bacterial plasmid capable of autonomous replication in the host cell of at least 400 constitutive copies of the vector. More preferably, this origin of replication is from ColE1. Most preferably, the origin is from plasmid pOPIΔ6 which has a restriction map shown in Fig. 7.

Another component of the vector is a first restriction enzyme site permitting replacement of the DNA sequence containing the ribosomal binding site which follows thereafter. Numerous such sites may be used. Suitable sites include EcoRI.

Another component of the vector is a second restriction enzyme site for insertion of the desired gene into the plasmid in phase with the ATG initiation codon. Numerous such sites may be used. Suitable sites include NdeI, ClaI, HindIII, SmaI, BglII, XbaI, SacI and AluI.

Generally it is desirable that the second restriction enzyme site also functions as the second restriction site necessary to permit replacement of the DNA sequence containing the ribosomal binding site. If the second restriction site is not also used for this purpose then the vector of this invention must also include a third restriction enzyme site after the ribosomal binding site but prior to the second restriction site.

Preferably, the vector contains two unique restriction enzyme sites. The first site permits replacement of the DNA sequence containing the ribosomal binding site. The second site permits insertion of the desired gene into the plasmid in phase with the ATG initiation codon. The term "unique restriction enzyme" site as employed herein means a restriction enzyme site which occurs only once in the plasmid. In a presently preferred embodiment, EcoRI is the first restriction enzyme site and NdeI is the second restriction enzyme site.

Preferably, the vector is a covalently closed circular double-stranded molecule. However, it is not essential that the plasmid be covalently closed.

The plasmid achieves its enhanced expression levels after the host cell is heated to a temperature at which the $C_I$ repressor protein is inactivated. A temperature above about 38°C is effective for this purpose and since it is desired that unnecessary heat damage to the host cells be avoided to as great an extent as possible, it is generally desirable that the temperature not exceed 42°C by more than a few degrees.

One important component of the vector is the ribosomal binding site. Suitable sites are $C_{II}$ from lambda bacteriophage having the sequence:

**TAAGGAAATACTTACAT**

**ATTCCTTTATGAATGTA;**

a mutant of $C_{II}$ from lambda bacteriophage having the sequence:

<div align="center">

**TAAGGAAGTACTTACAT**

**ATTCCTTCATGAATGTA;**

</div>

the major head protein gene of bacteriophage lambda having the sequence:

<div align="center">

**TTTTTTTACGGGATTTTTTTATG**

**AAAAAAATGCCCTAAAAAAATAC;**

</div>

the natural $\beta$-lactamase ribosomal binding site derived from pBR322;
a synthetic oligonucleotide having the sequence:

<div align="center">

**AATTCGAGCGCAAGGAAACAGGCTCA**

**GCTCGCGTTCCTTTGTCCGAGTAT;**

</div>

a synthetic oligonucleotide having the sequence:

<div align="center">

**AATTCAATAATATTGAAAAAGGAAGAG**

**GTTATTATAACTTTTTCCTTCTCAT;**

</div>

and
a natural ribosomal binding site derived from Bacillus thurengensis.

Relative to vectors described previously, the vectors of this invention may be used to obtain enhanced expression of a wide variety of genes encoding desirable polypeptide products. Suitable genes include those encoding growth hormones, e.g., bovine, porcine, chicken or human growth hormones; superoxide dismutase; apolipoprotein E or analogs of any of the preceding. By analog is meant a polypeptide having the same activity as the naturally occurring polypeptide but having one or more different amino acids added or deleted, or both, at the N-terminus of the polypeptide. However, SOD analogs described have an amino acid sequence identical to that of mature human SOD. The preferred host for use with the plasmids of this invention is Escherichia coli. The presently preferred strains are A1637, A1645, A2602, A2097, A1563 and A1645 ($\lambda$i434cl$^-$ mini Tn10).

A1645 is presently the most preferred strain for expression of superoxide dismutase or an analog thereof.

A1645 and A1645 ($\lambda$ i434cl$^-$ mini Tn10) are presently the more preferred strains for expression of animal growth hormone genes.

A2097 is presently the most preferred strain for the expression of the gene which produces:

1) an analog of bGH having the amino acid sequence met-asp-gln added to the amino-terminus of the phenylalanine form of authentic bGH; or
2) an analog of cGH having the amino acid methionine added to the amino-terminus of the phenylalanine form of natural cGH.

A1637 was obtained from C600 by inserting transposon containing tetracycline resistance gene within the galactose operon as well as the lambda system for expression which is close to galactose operon. C600 is available from the American Type Culture Collection, as ATCC Accession No. 23724.

A1645 was obtained from A1637 by selection for Gal$^+$ (ability to ferment galactose) as well as loss of tetracycline resistance. It still contains the lambda expression system but part of the transposon has been removed by selection. Its phenotype is C600 r$^-$ m$^+$ gal$^+$ thr$^-$ leu$^-$ lacZ$^-$ bl ($\lambda$c1857 $\Delta$HI $\Delta$ BamHl N$^+$). A1645 has been deposited with the American Type Culture Collection in Rockville, Maryland, U.S.A. containing various plasmids as described more fully hereinafter.

A1645 ($\lambda$i434cl$^-$ mini Tn10) was derived by infecting Escherichia coli strain A1645 containing a plasmid with imm434 cl3008 mini Tn10$\Delta$16$\Delta$17 at 30°C. Tetracycline resistant colonies were isolated and purified.

This strain containing plasmid pHG50 has been deposited with the American Type Culture Collection under ATCC Accession No. 39805.

A2602 and A1563 are derived from SA500. Their phenotypes are SA500 his⁻ ile⁻ gal⁺ Δ 8 (λcI857 ΔHI Δ Bam N⁺) and SA500 his⁻ ile⁻ gal⁺ Δ8 lacZ⁻ A21 (λ cI857 int2 xisI nutL₃Δ HI), respectively. A2097 is derived from A1645. Its phenotype is A1645 lacΔ$_\chi$A21 proC::Tn10.

Prototrophic strains of Escherichia coli which enable high level polypeptide expression even when grown in a minimal media may also be used as hosts for the vectors of this invention. Preferred prototrophic strains include A4200 and A4255. Strain A4255 containing the plasmid p9200 has been deposited with the ATCC under Accession No. 53215. Even more preferred are biotin independent prototrophic strains much as A4346 containing the plasmid pHG44 which has been deposited with the ATCC under Accession No. 53218.

Lytic strains of Escherichia coli may also be used as hosts for the vectors of this invention. Suitable lytic strains include those which produce, at the temperature at which the polypeptide is produced but at a rate slower than that at which the polypeptide is produced, a substance e.g., an enzyme like endolysin which will cause the cell to lyse. This permits the cell to produce relatively large amounts of the desired polypeptide before the amount of the lysing substance produced reaches the level which causes cell lysis. Examples of suitable lytic strains include those containing the PlcI$^{ts}$ plasmid such as strain A4048 containing pHG44 which has been deposited with the ATCC under Accession No. 53217.

All deposits were made pursuant to the Budapest Treaty on the International Recognition of the Deposit of microorganisms except that pBR322 and pBRM are fully available from the American Type Culture Collection as ATCC Accession Nos. 37017 and 37283, respectively, and D4 was deposited under ATCC Accession No. 31826 in connection with the filing of a U.S. patent application.

The vector may be formed by methods well known to those of ordinary skill in the art to which the invention relates. Such methods are described in greater detail in various publications identified herein, the contents of which are hereby incorporated by reference into the present disclosure in order to provide complete information concerning the state of the art.

One presently preferred vector is pJH200 which has the restriction map shown in Fig. 2. This vector was introduced into Escherichia coli strain A1645 using a conventional transformation method. The resulting host vector system has been deposited under ATCC Accession No. 39783. A gene encoding a desired polypeptide, e.g. bovine growth hormone, may be inserted into pJH200.

A second preferred vector, pRO211, was constructed from a partial NdeI digest of pJH200. pRO211 has the restriction map shown in Fig. 2. Bovine growth hormone cDNA has been inserted into pRO211 by digesting the vector with NdeI and HindIII, isolating the large fragment and ligating to it bGH cDNA obtained from pAL500 (ATCC Accession No. 39782). The resulting plasmid is designated pRO12. Its restriction map is also shown in Fig. 2.

Plasmid pRO12 has been partially digested with PvuII followed by NdeI. A synthetic DNA fragment coding for the first 24 amino acids of the N-terminus of authentic bGH has been ligated to the digested pRO12. The resulting plasmid, designated pSAL 5200-6, has the restriction map shown in Fig. 3.

The vectors of this invention may also be engineered to yield plasmids which produce human superoxide dismutase (SOD) or analogs thereof. A fragment of pJH200 (ATCC Accession No. 39783) containing the λP$_L$ promoter and C$_{II}$ ribosomal binding site was isolated and then inserted into a plasmid pSOD NH-10 which contains the gene for human SOD to form a plasmid designated pSOD NH-550 as shown in Fig. 13. A fragment containing both the λP$_L$ promoter and the SOD gene was isolated from pSOD NH-550 following digestion with AluI. After the addition of BamHI linkers and subsequent restriction with BamHI, the fragment was inserted into the unique BamHI site of pBRM. pBRM is a high copy number plasmid which has been deposited under ATCC Accession No. 37283. The resulting plasmid is designated pSODα2. It has the restriction map shown is Fig 13. This plasmid has been deposited in E. coli strain A2097 under ATCC Accession No. 39786.

Plasmid pSODα2 (ATCC Accession No. 39786) contains the C$_{II}$ ribosomal binding site. This ribosomal binding site has been replaced with a fragment containing the β-lactamase promoter and Shine-Dalgarno ribosomal binding site isolated from an EcoRI-AluI digest of pBLA11. (Plasmid pBLA11 has the restriction map shown in Fig. 14 and has been deposited in Escherichia coli strain A1645 under ATCC Accession No. 39788.) The C$_{II}$ ribosomal binding site is removed from plasmid pSODα2 as shown in Fig. 14. pSODα2 is partially restricted with EcoRI, filled in with DNA polymerase I (Klenow) and religated, so that the only remaining EcoRI site in the plasmid is located at the 5' end of the C$_{II}$ RBS. The resulting plasmid, designated pSOD 13 was digested with NdeI, filled in with DNA polymerase I (Klenow) and then digested with EcoRI. The large fragment was isolated and ligated to the fragment containing the β-lactamase promoter and ribosomal binding site isolated from pBLA11 to form plasmid pSODβ1.

16

pSODβ1 may be modified to include a tetracycline resistance gene fragment (Tet$^R$) instead of an ampicillin resistance gene fragment (Amp$^R$). The Amp$^R$ fragment was removed from pSODβ1 by digestion with PstI followed by partial BamHI. The resulting plasmid was filled in with DNA polymerase I (Klenow). The Tet$^R$ gene fragment was separately isolated from an EcoRI-AvaI digest of pBR322, filled in and ligated to the filled in plasmid. (Plasmid pBR322 is widely available, e.g. from the American Type Culture Collection as ATCC Accession No. 37017). The then resulting plasmid is designated pSODβ$_1$T$_{11}$. It has the restriction map shown in Fig. 15.

One further plasmid which may be used to produce human superoxide dismutase is designated pSODβ$_1$-BA2. Its construction from pSODα13 is shown in Fig. 17.

The vector e.g. pR0211 may also be engineered to produce porcine or chicken growth hormones. Thus, as shown in Fig. 4, porcine growth hormone cDNA was isolated from an NdeI digest of ppGH-NdeI/RI. The resulting fragment containing the pGH gene was ligated to an NdeI digest of pR0211. The resulting plasmid, designated p3008, has been deposited in E. coli strain A2097 under ATCC Accession No. 39804.

In another embodiment of the invention two chicken growth hormone fragments were isolated from NdeI-BanII digest of pcGH-NdeI/RI as shown in Fig. 5. The two cGH fragments were ligated to a phosphorylated synthetic linker which codes for the first 18 amino acids of the N-terminus of authentic cGH. The sequence of the linker was:

```
TATGTTCCCTGCCATGCCCCTCTCCAACCTGTTTGCCAACGCTGTGCTGAGGGCT
ACAAGGGACGGTACGGGGAGAGGTTGGACAAACGGTTGCGACACGACTC.
```

The resulting fragment was then ligated to a NdeI digest of pR0211 to form the plasmid designated p5002 which has the restriction map shown in Fig. 5.

The vectors may also be engineered to produce human apolipoprotein E. The gene for human apoplipoprotein E (ApoE) may be isolated from plasmid pApoE-EX2 by NdeI digestion. pApoE-Ex2 has the restriction map shown in Fig. 18. It has been deposited in E. coli strain A1645 under ATCC Accession No. 39787.

The ApoE gene (cDNA) may be placed in various plasmids. Among the preferred embodiments is plasmid pTV-188 which has the restriction map shown in Fig. 18. pTV-188 was constructed by ligation of the ApoE gene isolated from pApoE-Ex2 to a StuI digest of plasmid pSODβ$_1$T$_{11}$. pTV-188 contains the Tet$^R$ fragment, the P$_L$ promoter sequence, the β-lactamase promoter and Shine-Dalgarno sequence. This plasmid expresses an ApoE fused protein.

Another preferred embodiment of a plasmid which contains the ApoE gene is pSAL 160-5 which has the restriction map shown in Fig. 23. pSAL 160-5 was constructed from pTV 104(2) (ATCC Accession No. 39384) and plasmid pTV-170, (see also Fig. 20). The ApoE gene was isolated from pTV-170 and inserted into pTV 104(2) at the PstI site within the human growth hormone gene sequence. The resulting plasmid pSAL 160-5 contains the Amp$^R$ fragment and the p$_L$ promoter sequence.

One presently preferred vector is p579 which has the restriction map shown in Fig. 19. This vector can be introduced into suitable Escherichia coli strain, e.g., A1637, A2602, A1563, A1645 or A2097, using a conventional transformation method known to those of ordinary skill in the art. A gene encoding a desired polypeptide, e.g., porcine growth hormone or chicken growth hormone may be inserted into p579.

Porcine growth hormone cDNA has been inserted into p579 by digesting the vector with NdeI and ligating the open strand to pGH cDNA obtained from p3008 (ATCC Accession No. 39804). The resulting plasmid is designated p3009. Its restriction map is shown in Fig. 11.

Chicken growth hormone cDNA has been inserted into p579 by digesting the vector with NdeI and ligating the open strand to cGH cDNA obtained from p5002. The resulting plasmid is designated p5003 and has a restriction map shown in Fig. 12. p5003 has been deposited in Escherichia coli strain A2097 under ATCC Accession No. 39792.

The gene for the production of human apopliprotein E (ApoE3), presumably with the amino acid methionine added to the amino terminus in the final product, can also be inserted into p579. The construction of the resulting plasmid, designated pTV-170, is shown in Fig. 20. This plasmid contains the C$_{II}$ ribosomal binding site derived from pJH200 (ATCC Accession No. 39783).

Plasmid pTV-170 can be modified by removal of one of the NdeI sites bounding the ApoE gene. The resulting plasmid, designated pTV-190, is shown in Fig. 21.

pTV-170 can also be modified by replacement of the C$_{II}$ ribosomal binding site with the β-lactamase promoter and Shine-Dalgarno ribosomal binding site sequence isolated from PBLA11 (ATCC Accession No.

17

39788). The resulting plasmid, designated pTV-194, has the restriction map shown in Fig. 22.

pTV-190 (Fig. 21) can be modified so that it produces an analog of human ApoE3 which has at its amino terminus methionine followed by the 13 amino acid amino terminus sequence of human growth hormone, followed by methionine, attached to the sequence of mature human ApoE3. Such a plasmid is designated pTV-214 and has a restriction map shown in Fig. 24.

Another preferred embodiment of a plasmid which contains the ApoE3 gene is pTVR 279-8 which has the restriction map shown in Fig. 25. pTVR 279-8 was constructed from pTV 264-45 which was constructed from pTV 190. Plasmid pTV 190, (Fig. 21), was partially cleaved with AvaI, "filled in" using the Klenow fragment of DNA polymerase I and religated. The resulting plasmid, designated pTV 264-45 is deleted of the AvaI site at the 3' end of the gene. Plasmid pTV 264-45 was digested to completion with NdeI and ligated to phosphorylated synthetic linkers of the sequence:

$$5'-TATGCTGCTGCT$$

$$ACGACGACGAAT-5'$$

The resulting plasmid designated pTVR 279-8 has been deposited in the ATCC under Accession No. 53216.

The vectors used in this invention may also be engineered to form plasmids capable of producing human growth hormone. An example of such a plasmid is pTV 300 which has the restriction map shown in Fig. 27. pTV 300 was constructed by cleaving pTV 18(1) with NdeI, isolating the $met^{14}$-bGH DNA and ligating it to p579 (Fig. 19) cleaved with NdeI. pTV 18(1) may be obtained as described in European Patent Application Publication No. 0 131 843 A1, published January 23, 1985 or as described in corresponding U.S. patent application Serial No. 514,188, filed July 15, 1983, the latter of which is hereby incorporated by reference.

The vectors used in this invention may also be engineered to yield plasmids which produce an analog of human Cu-Zn superoxide dismutase (SOD) which differs from natural human SOD in that the amino terminus is not acetylated. Such a plasmid has been constructed according to Fig. 16 and has been designated $pSOD\beta_1 TT-1$.

The vectors used in this invention may be engineered to yield plasmids which produce a recombinant bovine growth hormone. One example is the production of an analog of bGH which has the amino acid sequence met-asp-gln added to the amino terminus of the phenylalanine form of authentic bGH which is also referred to as rec bGH. Plasmid pHG44, which produces such a hormone, was constructed according to the scheme in Fig. 6 and was deposited in strain A2097 under ATCC Accession No. 39806.

Another plasmid which produces the met-asp-gln bovine growth hormone is p9200. The plasmid p9200 is similar to pHG44 (Figure 6) however the plasmid confers tetracycline resistance instead of ampicillin resistance. The construction of p9200 is shown in Figure 26. pHG44 was cleaved with ClaI and PstI, "filled in" using the Klenow fragment of DNA polymerase I and then the large DNA fragment was isolated. This fragment as ligated to a DNA fragment containing the tetracycline resistance gene of pBR322 which was isolated by cleaving pBR322 with RI and AvaI and then "filling in" using the Klenow fragment of DNA polymerase I. The resulting plasmid p9200 was deposited in the ATCC under Accession No. 53215.

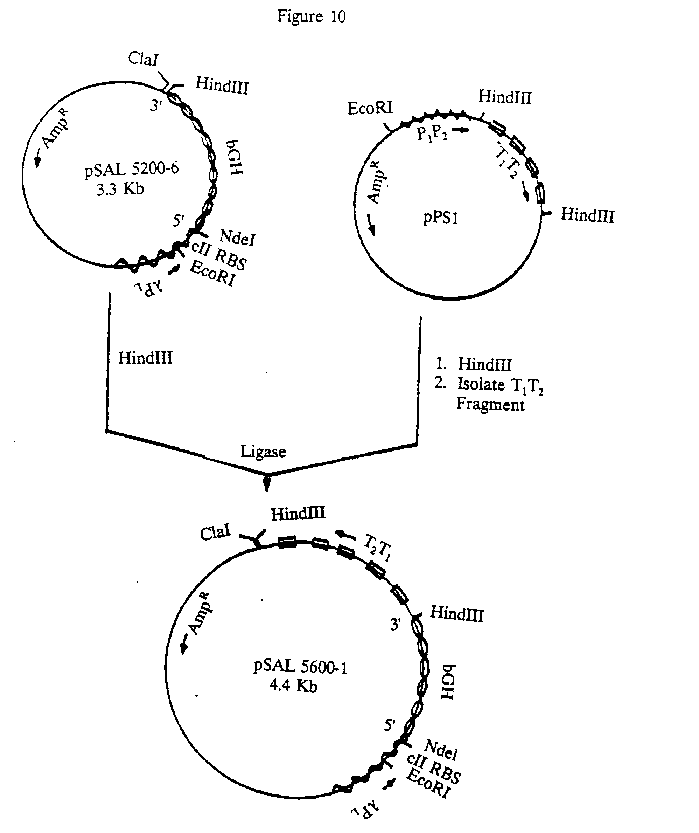

Another example is the production of an analog of bGH having the amino acid methionine added to the amino terminus of the phenylalanine form of natural bGH. Plasmid pSAL 5600-1, which produces such a hormone, was constructed according to the scheme in Fig. 10. Plasmid p7200-22 also produces such a hormone. This plasmid has a restriction map shown in Fig. 7.

One presently preferred vector is the vector p579 with a DNA sequence containing the $cl^{434}$ fragment cloned into the ClaI site. p579 has the restriction map shown in Fig. 19.

Plasmids which express an analog of bovine growth hormone having the amino acid sequence met-asp-gln added to the amino terminus of the phenylalanine form of natural bovine growth hormone (also referred to as rec-bovine growth hormone), have been constructed. One such plasmid is pHG50 which has a restriction map shown in Fig. 6. This plasmid has been deposited in strain A1645 ($\lambda i434cl^-$mini Tn10) with the American Type Culture Collection under ATCC Accession No. 37805.

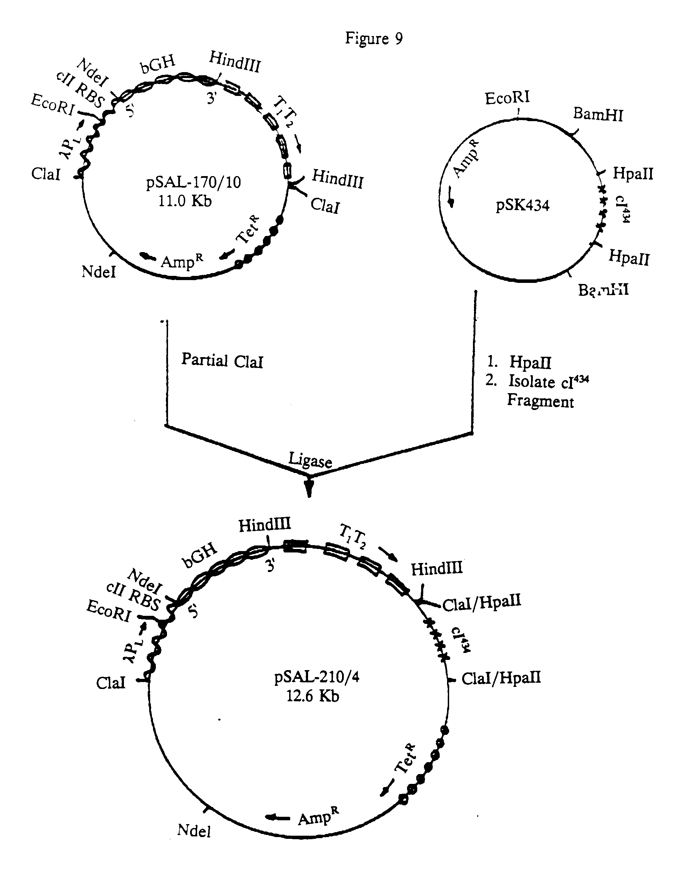

Another such plasmid is pSAL-210/4 which has the restriction map shown in Fig. 9.

One presently preferred vector is derived by removing the met-phe bGH gene from plasmid p8300-10A. The plasmid has the restriction map shown in Fig. 7 and has been deposited in strain A2097 with the American Type Culture Collection under ATCC Accession No. 39785.

Another presently preferred vector is derived by removing the rec bGH gene from plasmid pSAL-170/10. The plasmid as the restriction map shown in Fig. 8.

A third presently preferred vector is derived by removing the rec bGH gene from plasmid pSAL-210/4. The plasmid has the restriction map shown in Fig. 9.

The vectors used in this invention, upon introduction into a host, may also be engineered to yield plasmids which produce analogs of bovine growth hormone. p8300-10A (ATCC Accession No. 39785), one example of such a plasmid, was constructed according to the scheme shown in Fig. 7. The analog it produces has a methionine residue added to the amino-terminus of the phenylalanine form of natural bGH.

Other plasmids produce analogs which have the amino acid sequence met-asp-gln added to N-terminus of the phenylalanine form of natural bGH. These plasmids include pSAL-130/4 (Fig. 8), pSAL-170/10 (Fig. 8) and pSAL-210/4 (Fig. 9).

Using the same approach other plasmids may be prepared by replacing the gene encoding the desired polypeptide at the second restriction enzyme site of the plasmid.

Various host vector systems involve E. coli A1637, A1645, A2606, A2097 or A1563 if the plasmid does not contain the $cI^{434}$ fragment and strain A1645 ( i434cI⁻ mini Tn10) if the plasmid contains the $cI^{434}$ fragment. Host vector systems of this invention also involve prototrophic E. coli such as A4200, A4255 and include biotin independent host vector systems such as A4346. Lytic host vector systems of this invention include those wherein the host is A4048, particularly A3111.

The host vector systems and the plasmid described herein may be used to produce different polypeptides such as bovine, porcine, chicken and human growth hormones, human superoxide dismutase and human apoliprotein E. To do so, the host vector system is grown under suitable conditions permitting production of polypeptide which is then recovered.

Suitable conditions involve growth of the host vector system for an appropriate period of time at about 42°C. Desirably, the period of growth at 42°C for all host vector systems except those designed to produce human apolipoprotein E is about 1 to 5 hours. The period of growth for host vector systems designed to produce human apolipoprotein E is desirably about 15 minutes. Suitable media include casein hydrolysate.

By means of the preceding method a number of bGH, pGH, cGH, hGH, ApoE and SOD analogs have been prepared.

ApoE analogs have been prepared which have an amino acid sequence identical to that of natural ApoE except for variations at the N-terminus. Examples include the following:

1) amino acid methionine added to N-terminus of natural human apolipoprotein E;

2) natural human apolipoprotein E to the N-terminus of which is attached the 42 amino acid N-terminal sequence of human superoxide dismutase and then methionine;

3) natural human apolipoprotein from which the 11 N-terminal amino acids have been deleted and replaced by the 45 amino acid N-terminal sequence of mature human growth hormone followed by methionine;

4) amino acid sequence of natural human apolipoprotein E to the N-terminus of which the 14 amino acid N-terminal sequence of human growth hormone is attached, followed by methionine; and

5) natural human apolipoprotein E3 having the amino acid sequence met-leu-leu-leu-met attached to the N-terminus.

A pGH analog has been prepared in which the amino acid methionine is added to the N-terminus of natural porcine growth hormone.

A cGH analog has been prepared in which the amino acid methionine is added to the N-terminus of natural chicken growth hormone.

A hGH analog has been prepared which is deleted of the first 13 amino acids of natural human growth hormone, i.e., $met^{14}$ hGH.

SOD analogs have been prepared which have an amino acid sequence identical to that of natural SOD except for variations at the N-terminus. Examples include the following:

1) natural human SOD which is non-acetylated; and

2) natural human SOD which is non-acelylated and non-glycosylated.

These SOD analogs may be used to catalyze the dismutation or univalent reduction of the superoxide anion in the presence of proton to form hydrogen peroxide as shown in the following equation:

$$2O_2^{\cdot -} + 2H^+ \xrightarrow{\text{SOD}} H_2O_2 + O_2$$

Veterinary compositions may be prepared which contain effective amounts of one or more bGH, cGH or pGH analogs and a suitable carrier. Such carriers are well known to those of ordinary skill in the art. The analogs may be administered directly or in the form of a composition to a bovine in order to increase milk or meat production, to a chicken in order to increase meat production or to a pig in order to increase milk or meat production.

Pharmaceutical compositions may be prepared which contain effective amounts of one or more analogs of ApoE or hGH and a suitable carrier. Such carriers are well known to those skilled in the art. The analogs may be administered directly or in the form of a composition to a human subject, e.g., in the case of ApoE to treat deficiencies in ApoE production by the subject, or to treat aterioscelerosis or in the case of hGH to treat deficiencies in hGH production by the subject.

Veterinary and pharmaceutical compositions may also be prepared which contain effective amounts of SOD or one or more SOD analogs and a suitable carrier. Such carriers are well-known to those skilled in the art. The SOD or analog may be administered directly or in the form of a composition to the animal or human subject, e.g., to treat a subject afflicted by inflammations or to reduce injury to the subject by oxygen-free radicals on reperfusion following global ischemia or organ transplantation e.g., kidney transplantation. The SOD or analog may also be added directly or in the form of a composition to the perfusion medium of an isolated organ, e.g., to reduce injury to an isolated organ by oxygen-free radicals on perfusion after excision, thus prolonging the survival period of the organ, e.g. cornea. Additionally, the SOD or analog may be used to reduce neurological injury.

A method of producing enzymatically active eucaryotic superoxide dismutase (SOD) or an analog thereof in a bacterial cell has been discovered. The bacterial cell contains and is capable of expressing a DNA sequence encoding the superoxide dismutase or analog. The method comprises maintaining the bacterial cell under suitable conditions and in a suitable production medium. The production medium is supplemented with an amount of $Cu^{++}$ so that the concentration of $Cu^{++}$ in the medium is greater than about 2 ppm.

The bacterial cell is a bacterium in which a DNA sequence encoding eucaryotic superoxide dismutase has been introduced by recombinant DNA techniques. The bacterium must be capable of expressing the DNA sequence and producing the protein product. The suitable conditions and production medium will vary according to the species and strain of bacterium.

The bacterial cell may contain the DNA sequence encoding the superoxide dismutase or analog in the body of a vector DNA molecule such as a plasmid. The vector or plasmid is constructed by recombinant DNA techniques to have the sequence encoding the SOD incorporated at a suitable position in the molecule.

According to the invention the bacterial cell is an Escherichia coli cell. The preferred strain of E. coli is strain A1645. The E. coli cell of this invention contains a plasmid which encodes for the SOD or its analog. In a preferred embodiment of the invention the SOD is human superoxide dismutase or an analog thereof.

The preferred embodiments of the invention concern E. coli strains which contain the plasmids pSOD $_{\beta 1}$, pSOD $\alpha 2$, pSOD$\beta_1 T_{11}$, pSOD $_{\beta 1}$-BA2 or pSOD $_{\beta 1}T\overline{T1}$. Methods of constructing these plasmids are described in the Description of the Figures and the plasmids themselves are described in Example 3. These plasmids can be constructed from available starting materials by persons of ordinary skill in the art. E. coli strains containing the various plasmids which are useful in constructing plasmids encoding eucaryotic superoxide dismutase have been deposited with the American Type Culture Collection, Rockville, Maryland 20852, pursuant to the provisions of the Budapest Treaty on the International Recognition of the Deposit of Microorganisms for the purposes of Patent Procedure. These plasmids and their ATCC accession numbers are: pBRM ATCC 37283; pSODα2 ATCC 39786; pBR322 ATCC 37017; pBLA11 ATCC 39788; pJH200 ATCC 39783 and pPSI ATCC 39807.

In a specific embodiment of the invention an enzymatically active human superoxide dismutase analog is produced by an E. coli strain A2097 cell containing the plasmid pSODα2. This cell has been deposited with the American Type Culture Collection under accession number ATCC 39786.

The suitable production medium for the bacterial cell can be any type of acceptable growth medium such as casein hydrolysate or LB (Luria Broth) medium. Suitable growth conditions will vary with the strain of E. coli and the plasmid it contains, for example E. coli A1645 containing plasmid pSOD $_{\beta 1}$T11 are induced at 42°C and maintained at that temperature from about 1 to about 5 hours. The suitable conditions of temperature, time, agitation and aeration for growing the inoculum and for growing the culture to a desired density before the production phase as well as for maintaining the culture in the production period are described in Example 26.

The concentration of $Cu^{++}$ ion in the medium that is necessary to produce enzymatically active SOD will vary with the type of medium used. In a casein hydrolysate medium the $Cu^{++}$ ion concentration of 200 ppm has been found effective.

20

In a LB medium a $Cu^{++}$ concentration of 75 ppm has been found effective. It is preferred that in all complex types of growth mediums the concentration of $Cu^{++}$ in the medium is from about 50 to about 250 ppm.

Most eucaryotic superoxide dismutases are Cu/Zn metalloproteins. In a specific embodiment of the invention $Zn^{++}$ is added as a supplement to the medium, so that the concentration of $Zn^{++}$ in the medium is greater than about 2 ppm. In a preferred embodiment of the invention $Cu^{++}$ and $Zn^{++}$ concentrations are supplemented by adding 0.8 g/l of $CuSO_4 \cdot 5H_2O$ and 10 mg./l of $ZnSO_4 \cdot 7H_2O$.

The specific ingredients of the suitable stock, culture, inoculating and production mediums may vary and are known to those of ordinary skill in the art. Specific examples of suitable mediums are described in Example 26.

The invention also concerns a method of recovering purified enzymatically active eucaryotic SOD or an analog thereof produced in a bacterial cell in accordance with the methods of this invention.

The bacterial cell is first isolated from the production medium after the culture has been chilled. The cell may be isolated by any nondisruptive method such as centrifugation or filtration. The cell is then suspended in a suitable solution having a pH from about 7.0 to about 8.0. It is preferred that a 50mM sodium phospate solution of a pH of about 7.8 be used. The cell wall is disrupted by a suitable means such as a blender or a cell disrupter and the resulting homogeneous suspension is sonicated under suitable conditions. The sonication may be by means of a continuous flow cell. The resulting sonicated solution is then centrifuged under suitable conditions so as to separate the cell debris from the protein supernatant solution.

The supernatant is then heated for about 2 hours at about 65ºC, cooled and centrifuged under the same conditions as were used in the previous centrifugation step in order to result in a clear protein solution as a supernatant. The supernatant is then concentrated to an appropriate volume, e.g. concentrated to 1 liter in an ultrafiltration device using a 10,000 molecular weight cutoff.

The concentrated protein solution is then subjected to ion exchange chromatography on a suitable anion exchanger equilibrated at a pH from about 7.0 to about 8.0. It is preferred that the chromatography should be carried out on a DEAE-Sephacel column equilibriated with 150mM sodium phosphate buffer having a pH of about 7.8. The resulting flow through solution containing the superoxide dismutase or analog is then collected, concentrated to an appropriate volume and dialyzed against a buffered solution with a pH from about 7.0 to about 8.0. This can be done in an ultrafiltration device against a 20mM Tris-HCl solution of a pH of about 7.8.

This concentrated solution is then subjected to ion exchange chromatography on a suitable anion exchanger equilibrated at a pH from about 7.0 to about 8.0. A QAE-Sepharose column equilibrated with 20mM Tris-HCl having a pH of about 7.8 is suitable. The protein bound to the anion exchanger is subjected to a suitable salt gradient e.g. 0-200mM NaCl in 20mM Tris HCl pH 7.8. The resulting fractions containing the SOD or analog are collected, concentrated e.g. with an ultrafiltration device, dialyzed against distilled water and the adjusted to a pH from about 4.0 to about 5.0 with a suitable buffer. In a preferred embodiment the solution of interest is brought to 100mM Sodium Acetate by adding 1M Sodium Acetate having a pH of about 4.8.

This buffered solution is again subjected to ion exchange chromatography but with a cation exchanger. A CM-Sepharose column equilibrated with a 100mM sodium acetate buffer having pH of about 4.7 is suitable. The protein bound to the cation exchanger is subjected to a suitable salt gradient, such as 100 to 500mM NaCl in 100mM sodium acetate pH 4.7 and the resulting fractions containing the purified SOD or analog are collected.

The fractions containing the purified SOD may be concentrated e.g. by ultrafiltration and lyophilized for storage.

By the methods of this invention a purified enzymatically active eucaryotic superoxide dismutase or analogs thereof can be produced and purified. Preferably enzymatically active human superoxide dismutase and analogs thereof are prepared and purified by the methods of this invention.

Examples

The examples which follow are set forth to aid in understanding the invention but are not intended to, and should not be construed to, limit its scope in any way. The examples do not include detailed descriptions for conventional methods employed in the construction of vectors, the insertion of genes encoding polypeptides of interest into such vectors or the introduction of the resulting plasmids into bacterial hosts. Such methods are well-known to those of ordinary skill in the art and are described in numerous publications including by way of example the following:

T. Maniatis, E.F. Fritsch and J. Sambrook, Molecular Cloning; A Laboratory Manual, Cold Spring Harbor Laboratory, New York (1982).

Methods in Enzymology, vol. 65, "Nucleic Acids (Part 1)," edited by Lawrence Grossman and Kivie Moldave, Academic Press, New York (1980).

Methods in Enzymology, vol. 68, "Recombinant DNA," edited by Ray Wu, Academic Press, New York (1981).

Methods in Enzymology, vol. 100, "Recombinant DNA (Part B)," edited by Ray Wu, Lawrence Grossman and Kivie Moldave, Academic Press, New York (1983).

Methods in Enzymology, vol. 101, "Recombinant DNA (Part C)," edited by Ray Wu, Lawrence Grossman and Kivie Moldave, Academic Press, New York (1983).

Principles of Gene Manipulation, An Introduction to Genetic Engineering, 2nd Edition, edited by R.W. Old and S.B. Primrose, University of California Press (1981).

H.V. Bernard, et al., Gene (1979) 5, 59.

A.B. Oppenheim, et al., J. Mol. Biol. (1982) 158, 327.

E. Remaut, et al., Gene (1981) 15, 81.

EXAMPLE 1

Expression Vectors

As used herein the term "expression vector" refers to a group of plasmids useful for expressing desired genes in bacteria, particularly in E. coli. The desired gene may be inserted into the expression vector or alternatively, the promoters on the expression vector may be excised and placed in front of the desired gene.

pJH200

pJH200, shown in Fig. 2, is composed of a DNA inserted into the multicopy plasmid pBR322. The salient features of the λDNA are that it contains the λ$P_L$ promoter, the leftward N utilization site (nut$_L$), an EcoRI restriction site, the t$_{RI}$ termination site, followed by the C$_{II}$ ribosomal binding site and an ATG initiation codon which is part of the NdeI restriction site. One hundred and sixteen (116) base pairs downstream of the NdeI restriction site are four unique restriction sites as shown in Fig. 2. The restriction sites enable facile insertion of the desired gene. The C$_{II}$ ribosomal binding site differs from the natural ribosomal binding site by a single point mutation.

pJH200 was constructed from pOG11 (A. Oppenheim, et al., J. Mol. Biol. (1982) 158; 327) and contains the λ$P_L$ promoter and the C$_{II}$ ribosomal binding site found in pOG11. However, 346 bp of λDNA located between the λ$P_L$ promoter and the C$_{II}$ ribosomal binding site have been deleted, and an EcoRI restriction site has been introduced at the junction between these two elements. Also, a multi-restriction site linker was introduced "downstream" of the ribosome binding site. pJH200 has been deposited with the American Type Culture Collection under ATCC No. 39783.

pRO211

pRO211, shown in Fig. 2 and described in detail in the Description of Figures, was derived from pJH200 by eliminating one of the two NdeI restriction sites.

pJH200, pRO211 and derivatives thereof containing eucaryotic genes may be maintained in suitable E. coli hosts. The most important feature of a suitable host is that it provide the thermosensitive repressor cI857 and the anti-termination N protein. (M.E. Gottesman, et al., J. Mol. Biol. (1980) 140; 57-75).

pRO211 has numerous advantages over previously described expression vectors including:

1. extremely high levels of expression

The vector is capable of directing expression of foreign proteins in E. coli at levels as high as 35% of the total cellular protein.

2. replaceable ribosomal binding site

pRO211 contains a unique EcoRI site which is located "upstream" of the ribosomal binding site, and an NdeI site located "downstream" of the ribosomal binding site. Thus, the ribosomal binding site is bounded by two unique restriction sites. This enables facile excision of the present ribosomal binding site (the $\lambda C_{II}$ ribosomal binding site) and substitution of virtually any other natural or synthetic ribosomal binding site without altering other features of the plasmid. This greatly facilitates optimal expression of desired polypeptides.

3. thermoinducible regulation of expression

The $\lambda P_L$ promoter is inactive when the $C_I$ repressor is bound to it. The cl857 repressor is thermosensitive, that is, it binds to the promoter at 30°C but is inactivated at 42°C. Thus, by increasing the temperature of fermentation to 42°C the host bacteria are induced to produce the desired protein.

The advantages of such a system include the following:

(a) A foreign protein which is toxic to Escherichia coli can be produced late in the fermentation process thus avoiding early cell death,

(b) Overproduction of a protein may stabilize the protein and prevent proteolytic degradation. (Cheng, Y.E., et al., Gene (1981) 14, 121). Thus, "instantaneous" overproduction using a tightly regulated promoter such as $\lambda P_L$ may be preferable to continuous low level production.

4. simplified induction protocol

Protein production by the plasmids described in this patent application and in copending, coassigned U.S. patent application Serial No. 514,188 is regulated by the thermosensitive cl857 repressor.

The induction protocol required by the plasmids described in the copending, coassigned application involved induction at 42°C followed by growth at 38°C. In contrast, the optimal induction of protein synthesis when using the vectors pJH200, pRO211 or their plasmid derivatives involved induction at 42°C followed by growth at the same temperature, i.e. 42°C. This eliminates the need to cool the fermentor.

5. copy number

The $\lambda P_L$ promoter in pJH200 and pRO211 is found on a plasmid with a copy number higher than the $\lambda$ transducing phage vectors which are present in E. coli. This increases expression levels.

6. ribosome binding site and initiation codon

This expression vector contains a strong procaryotic ribosomal binding site (RBS) as well as a translation initiation codon (ATG). Thus, any eucaryotic gene may be cloned without adding the initiation codon. Furthermore, the efficient RBS increases levels of expression. The ribosome binding site is the $\lambda C_{II}$ ribosomal binding site. The sequence of the ribosomal binding site is:

**TAAGGAAGTACTTACAT**

**ATTCCTTCATGAATGTA**

One base pair is different from the ribosomal binding site round in the wild type $\lambda$.

7. convenient restriction site

The expression vector has a unique NdeI restriction site which contains within the site the ATG initiation codon. This permits proper positioning of the desired gene. The unique NdeI site is found immediately after the ribosomal binding site.

8. convenient restriction sides for gene insertion

Located 116 base pairs downstream of the NdeI restriction site are 4 other unique restriction sites in the following order: BglII, SmaI, HindIII and ClaI. The multiplicity of unique restriction sites enables facile insertion of desired genes.

9. nut site

N protein, which is provided by the host, binds the Nut site on the expression vector and thereby prevents termination of transcription at the $t_{RI}$ site or premature transcription termination within the cloned gene.

Strains

Suitable hosts for the described vectors and plasmids are strains of E. coli suitable for transformation, including A1637, A2602, A1563, A1645 (c600 r⁻m⁺ gal⁺ thr⁻ leu⁻ lac⁻ bI (λcI857ΔHI ΔBamHI N⁺)) and A2097 (A1645 lac Δ$_\chi$A21 proC::Tn 10).

EXAMPLE 2

Animal Growth Hormones

I. pRO12

The construction of pRO12 is shown in Fig. 2 and described in the Description of the Figures. bGH cDNA from pAL500 whose construction is shown in Fig. 1, was manipulated prior to insertion into pRO211 to provide the correct reading frame and an NdeI restriction site.

pRO12 was introduced into Escherichia coli strain A1645 by transformation using methods known to those of ordinary skill in the art. This strain produces upon growth and induction an analog of bovine growth hormone (bGH) having the amino acid sequence met-asp-gln added to the N-terminus of the phenylalanine form of natural bGH. The amount of bGH analog produced by pRO12 was about 30 - 36% of the total protein produced by the bacteria as calculated by scanning Coomasie blue-stained SDS polyacrylamide gels (Table I).

II. pSAL 5200-6

The construction of pSAL 5200-6 is shown in Fig. 3 and described in the Description of the Figures. The DNA sequence coding for met-phe bGH was obtained by restricting pRO12 with PvuII and NdeI and inserting a synthetic DNA fragment formed from two single-stranded synthetic oligonucleotides having 10 base pair overlapping segments.

pSAL 5200-6 was introduced into Escherichia coli strain A1645 by transformation using known methods. This strain produces upon growth and induction an analog of bGH having a methionine added to the amino terminus of phe bGH. The amount of the met-phe bGH analog produced by pSAL 5200-6 was about 18 - 20% of the total protein produced by the bacteria as calculated from scanning Coomasie-stained SDS polyacrylamide gels. The methods used to grow the strain, recover the bGH produced and purify the bGH are the same as those described hereinafter in Example 5 for bGH production from pRO12.

III. p3008

The construction of P3008 is shown in Fig. 4 and described in the Description of the Figures. p3008 has been deposited with the American Type Culture Collection under ATCC No. 39804. The DNA sequence coding for met-phe pGH (porcine growth hormone) was obtained by inserting pGH cDNA into pRO211.

p3008 was introduced into Escherichia coli strain A1645 by transformation using methods known to those of ordinary skill in the art. This strain produces upon growth and induction pGH having a methionine added to the amino terminus of phe pGH. The amount of the met-phe pGH analog produced by p3008 was about 18 - 20% of the total protein produced by the bacteria as calculated from scanning Coomasie-stained SDS polyacrylamide gels. The methods used to grow the strain, recover the pGH produced and purify the pGH are the same as those described hereinafter in Example 5 for bGH production from pRO12.

IV. p5002

The construction of p5002 is shown in Fig. 5 and described in the Description of the Figures. The DNA sequence coding for met-phe cGH (chicken growth hormone) was obtained by inserting cGH cDNA into pRO211 and completing the 5' end of the gene with synthetic oligonucleotide linkers.

p5002 was introduced into Escherichia coli strain A1645 by transformation using known methods. This strain produces upon growth and induction cGH having a methionine added to the amino terminus of phe cGH. The amount of the met-phe cGH analog produced by p5002 was about 18 - 20% of the total protein produced by the bacteria as calculated from scanning the Coomasie-stained SDS polyacrylamide gels. The methods used to grow the strain, recover cGH produced and purify the cGH are the same as those described hereinafter in Example 5 for bGH production from pRO12.

TABLE I[1]

| Plasmid | Host | %bGH[2] | Remarks |
|---|---|---|---|
| pRec 2/3 | A1637 | 23 | $Amp^R$ |
| PRO11 | A1637 | 28 | $Amp^R$ |
| pRO12 | A1645 | 30-36 | $Amp^R$ |
| pHG44 | A2097 | 37-42 | $Amp^R, T_1 T_2$ |
| pHG50 | A1645 | 37-42 | $Amp^R, T_1 T_2; cI^{434}$ |
| pSAL-130/5 | A1645 | 39-44 | $Amp^R; CHCN; T_1 T_2$ |
| pSAL-170/10 | A1645 | 40-46 | $Tet^R; CHCN; T_1 T_2$ |

1. The table summarizes the bGH expression levels of various plasmids derived from pRO211 and also of plasmids pRec 2/3 and pRO11 both of which are described in copending, coassigned U.S. patent application Serial No. 514,188, filed July 15, 1983.
2. Amount of bGH produced as percentage of total bacterial protein.

ABBREVIATIONS

CHCN = Constitutive high copy number
$Amp^R$ = Ampicillin resistance
$Tet^R$ = Tetracycline resistance
$T_1 T_2$ = Transcription termination sequences
$cI^{434}$ = Plasmid stabilization $cI^{434}$ system

EXAMPLE 3

Human Cu-Zn Superoxide Dismutase (SOD)

The starting point for Cu-Zn SOD cDNA modifications is the plasmid pS61-10 described in Lieman-Hurwitz, J., et al., PNAS (1982), 79: 2808. The SOD cDNA is also described in copending U.S. patent application Serial No. 489,786, filed April 29, 1983. The SOD cDNA was modified to introduce an NdeI restriction site at the 5' end of the gene and a HindIII restriction site at the 3' end of the gene. The resulting plasmid, pSOD NH-10, contains SOD cDNA bounded by unique restriction sites.

I. pSODα2

The construction of pSODα2 is shown in Fig. 13 and described in the Description of the Figures. pSODα2 has been deposited with the American Type Culture Collection under ATCC No. 39786. To construct pSODα2, the λP_L promoter, the Nut_L and the C_II ribosomal binding site were excised from the expression vector pJH200 and placed in front of the SOD gene of plasmid pSOD NH-10. Then, the fragment containing both the promoter, the RBS and the SOD gene was inserted into the vector pBRM (Hartman,J.R.,et al., PNAS 79: 233-237 (1982). pBRM has been deposited with the American Type Culture Collection under ATCC No. 37283.

pSODα2 was introduced into Escherichia coli strain A2097 by transformation using known methods. The clones obtained produce upon growth and induction an SOD analog protein. The amount of SOD analog produced by pSODα2 was about 0.1 - 0.3% of the total protein produced by the bacteria as calculated from scanning of Coomasie-stained SDS polyacrylamide gels (Table II). The SOD analog produced is probably identical to that produced by pSODβ1 described in the following paragraph.

II. pSOD*β*1

The construction of pSOD*β*1 is shown in Fig. 14 and described in the Description of the Figures. To construct pSOD*β*1, the $C_{II}$ RBS of pSOD*α*2 was replaced with the *β*-lactamase promoter and RBS derived from pBLA11. pBLA11 has been deposited with the American Type Culture Collection under ATCC No. 39788.

pBLA11 contains the promoter and ribosomal binding site of the *β*-lactamase gene found in pBR322 between coordinates 4157 and 4353. An EcoRI linker was added upstream of the promoter and a multi-restriction site linker was added immediately after the initiation codon ATG. Thus, the sequence of the coding strand beginning with the initiation codon is ATGAGCTCTAGAATTC.

pSOD*β*1 was introduced into Escherichia coli strain A1645 by transformation using known methods. The clones obtained produce upon growth and induction an SOD analog. The human Cu-Zn SOD analog produced differs from natural human Cu-Zn SOD in that the amino terminus alanine is not acetylated, as demonstrated by amino acid sequencing stoichiometry while the natural human SOD is acetylated at the amino terminus alanine (Hartz, J.W. and Deutsch, H.F., J. Biol. Chem. (1972) 234:7043-7050; Jabusch, J.R., et al., Biochemistry (1980) 19:2310-2316; Barra, et al., FEBS Letters (1980) 120:53 and Oberley, L.W., Superoxide Dismutase, Vol. I, (1982), CRC Press, Florida, pp. 32-33.). Furthermore, the natural human SOD is glycosylated (Huber, W., U.S. Patent No. 3,579,495, issued May 18, 1971) while bacterial-produced human SOD is almost certainly not glycosylated, because Escherichia coli does not glycosylate proteins which it produces. The amino acid sequence of the bacterial-produced SOD analog is identical to that of mature human SOD and does not contain a methionine residue at its N-terminus.

The amount of SOD produced by pSOD*β*1 was about 3-8% of the total protein produced by the bacteria as calculated from scanning of Coomasie-stained SDS polyacrylamide gels (Table II). The methods used to grow the strain, recover the SOD produced and purify the SOD are the same as those described hereinafter in Example 7 for pSOD*β*1T11.

III. pSOD*β*1T11

The construction of pSOD*β*1T11 is shown in Fig. 15 and described in the Description of the Figures. The *β*-lactamase gene of pSOD*β*1 was replaced with the gene coding for tetracycline resistance derived from pBR322.

The amount of SOD analog produced by pSOD*β*1T11 was about 8-13% of the total protein produced by the bacteria as calculated from scanning of Coomasie-stained SDS polyacrylamide gels (Table II). The SOD analog produced is identical to that produced by pSOD*β*1.

IV. pSOD*β*1-BA2

The construction of pSOD*β*1-BA2 is shown in Fig. 17 and described in the Description of the Figures. The $C_{II}$ ribosomal binding site of pSOD*α*13 was replaced by a synthetic DNA fragment with the sequence:

**AATTCAATAATATTGAAAAAGGAAGAG**

**GTTATTATAACTTTTTCCTTCTCAT**

which is similar to the sequence of the natural *β*-lactamase RBS.

pSOD *β*1-BA2 was introduced into Escherichia coli strain A1645 by transformation using methods known to those of ordinary skill in the art. The clones obtained produce upon growth and induction an analog of human SOD. The amount of SOD produced by pSOD*β*1-BA2 was about 2 - 4% of the total protein produced by the bacteria as calculated from scanning of Coomasie-stained SDS polyacrylamide gel (Table II). The SOD analog produced is identical to that produced by pSOD*β*1.

26

## TABLE II

| Plasmid | RBS | Host | %SOD[3] | Remarks |
|---|---|---|---|---|
| pSOD$\alpha$2 | C$_{II}$ | A2097 | 0.1-0.3 | Amp$^R$ |
| pSOD$\beta_1$ | BLA[1] | A1645 | 3-8 | Amp$^R$ |
| pSOD$\beta_1$T$_{11}$ | BLA[1] | A1645 | 8-13 | Tet$^R$ |
| pSOD$\beta_1$TT-1 | BLA[1] | A1645 | 10-15 | Tet$^R$;T$_1$T$_2$ |
| pSOD$\beta_1$-BA2 | BLA[2] | A1645 | 2-4 | Amp$^R$ |

1. Promoter and ribomosal binding site of $\beta$-lactamase gene.
2. Synthetic ribosomal binding site corresponding to that of the $\beta$-lactamase gene.
3. Amount of SOD analog produced expressed as percentage of total bacterial protein.

ABBREVIATIONS

Amp$^R$ =      Ampicillin resistance
Tet$^R$ =      Tetracycline resistance
T$_1$T$_2$ =      Transcription termination sequences

EXAMPLE 4

Human Apolipoprotein E (ApoE3)

The starting point for ApoE3 cDNA modifications was the plasmid pNB178 provided by Dr. John Taylor of the Gladstone Foundation, San Francisco, California. This plasmid contains a full length cDNA copy of the human ApoE3 gene. The cDNA in pNB178 was modified to remove noncoding DNA at the 5' end of the gene and to add NdeI restriction sites at both ends of the gene. This ApoE3 cDNA fragment was inserted into the vector pND5 (described in copending, coassigned U.S. patent application Serial No. 514,188, filed July 15, 1983). The resulting plasmid, pApoE-Ex2, shown in Fig. 18, has been deposited with the American Type Culture Collection under ATCC No. 39787.

I. pTV-188

The construction of pTV-188 is shown in Fig. 18 and described in the Description of the Figures. The plasmid pTV-188 was obtained by insertion of the NdeI - NdeI filled-in, ApoE3 fragment into the unique blunt end Stu I site of pSOD$\beta_1$T$_{11}$ (shown in Fig. 14 and described in the Description of the Figures.)

pTV-188 was introduced into Escherichia coli strain A1645 by transformation using methods known to those of ordinary skill in the art. The clones obtained produce upon growth and induction an analog of human ApoE3 having 42 amino acids of the N-terminal sequence of human superoxide dismutase attached to the N-terminus of authentic human ApoE3 followed by methionine at the N-terminus of the analog. The ApoE3 analog produced was about 10% of the total protein produced by the bacteria as calculated by scanning Coomasie-stained SDS polyacrylamide gels. The method used to grow the strain is the same as that described in Example 5 for bGH production from pRO12 except that 12.5 mg/liter tetracycline is used instead of ampicillin.

II. pSAL 160-5

The construction of pSAL 160-5 is shown in Fig. 23 and described in the Description of the Figures. The plasmid pSAL 160-5 was obtained by insertion of the AvaI-AvaI ApoE3 gene fragment from pTV-170 (see Fig. 20).

pSAL 160-5 was introduced into Escherichia coli strain A1645 by transformation using methods known to those of ordinary skill in the art. The clones obtained produce upon growth and induction and analog of ApoE3 which contains at its amino terminus methionine and then 45 amino acids from the N-terminus of human growth hormone fused to ApoE3 from which the 11 N-terminal amino acids have been deleted. The amount of ApoE3 analog produced by pSAL 160-5 was about 5% of the total protein produced by the bacteria as calculated by scanning Coomasie-stained SDS polyacrylamids gels. The method used to grow the strain is the same as that described in Example 5 for bGH production from pRO12.

EXAMPLE 5

Growth of pRO12

I. Stock Cultures

Stock cultures of pRO12 were grown on casein medium (see Inoculum), then diluted two-fold with freezing medium and stored at -80°C. Freezing medium contains per 500 ml:

| | |
|---|---|
| $K_2HPO_4$ | 6.3 gr |
| $KH_2PO_4$ | 1.8 gr. |
| Na Citrate | 0.45 gr |
| $MgSO_4 \cdot 7H_2O$ | 0.09 gr |
| $(NH_4)_2SO_4$ | 0.9 gr |
| Glycerol | 44.0 gr |

II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed, subsequent stages in inoculum propagation were carried out in stirred aerated fermenters. Sterile medium was inoculated with 2-10% inoculum and incubated 15 hours at 30°C, pH 7± 0.5 with agitation and aeration to maintain a dissolved oxygen level above 20% air saturation.

III. Production

The production medium contains:

| | |
|---|---|
| Casein hydrolysate | 20 g/l |
| Yeast extract | 10 g/l |
| $K_2HPO_4$ | 2.5 g/l |
| $MgSO_4 \cdot 7H_2O$ | 1 g/l |
| NaCl | 5 g/l |
| Biotin | 0.1 mg/l |
| Thiamine | 1 mg/l |
| Trace elements solution | 3 ml/l |

The medium also contains 100 mg/liter ampicillin. The ampicillin is optional for production but is always found in the medium used for growing the inoculum.

Biotin, thiamine and ampicillin in concentrated solution were filter sterilized separately and added to the sterile production medium before inoculation. Sterile gulucose solution was added initially to supply 10 g/l. At the induction step another 10 g/l of glucose was added.

The trace elements solution contains

| FeCl$_3$ | 16 g/l |
|---|---|
| ZnCl$_2$ • 4H$_2$O | 2 g/l |
| CoCl$_2$ • 6H$_2$O | 2 g/l |
| Na$_2$MoO$_4$ • 2H$_2$O | 2 g/l |
| CaCl$_2$ • 2H$_2$O | 1 g/l |
| CuCl$_2$ | 1 g/l |
| H$_3$BO$_3$ | 0.5 g/l |
| Conc. HCl | 100 ml/l |

The medium is inoculated with 0.5 - 10% inoculum culture and incubated at 30°C. Agitation-aeration rates are set to maintain a dissolved oxygen level above 20% air saturation. The pH is maintained at 7±0.2 with NH$_3$. Once cell concentration reaches about 3.5 g/l (OD$_{660}$ = 10) induction is started.

The temperature is raised to 42°C and maintained at 42°C for 1-5 hours. The culture is then chilled and cells are recovered by centrifugation for hormone purification.

Recovery of bGH

Thirteen kilograms of bacterial cells (wet cake) are resuspended in 5 volumes of a solution containing 50 mM sodium phosphate buffer (pH 7.4), 50 mM EDTA and 100 mM NaCl, using a Polytron (Kinematica) blender, while controlling the blender's speed to minimize foaming. The homogenous suspension is continuously passed through a Dynomill cell disruptor KDS (Willy A. Bachofen, Basel) at a rate of 80 liter per hour and the homogeneous suspension of disrupted cells clarified by centrifugation in a CEPA 101 centrifuge at a flow rate of 45 liter per hour. The precipitate from the centrifugation step is collected and resuspended in 15.5 liters of 50 mM sodium phosphate buffer (pH 7.4) containing 50 mM EDTA. Lysoszyme is added to a final concentration of 0.05 mg/ml and the suspension incubated for 16 hours at 37°C. Triton X-100 is added to a final concentration of 1%. The suspension is then incubated for 30 minutes at room temperature, sonicated in a continuous flow cell sonificator (Heat System) at a rate of 18 liters per hour and centrifuged in a CEPA 101 centrifuge. The precipitate is collected, resuspended in 50 mM sodium phosphate buffer (pH 7.4), sonicated as above, and centrifuged in a CEPA 101 centrifuge. The cells are resuspended in 15.5 liters of 50 mM sodium phosphate buffer (pH 7.4) containing 50 mM EDTA and 100 mM NaCl and twice precipitated and resuspended in 15.5 liters of distilled water. The precipitate is collected by centrifugation and stored at -20°C.

Purification Of bGH

The precipitate is resuspended in 30 - 40 liters distilled water and solubilized by titration with 0.5 N NaOH to pH 11.8. The solution is then continuously sonicated and clarified by centrifugation in CEPA 101 centrifuge if necessary, or filtered through Whatman No. 1 paper.

The clarified protein solution (32.6 liters containing 297,000 OD's at 280 nm) is divided into separate portions (6 X 5.4 liters) each containing 50,000 - 60,000 OD's. Each portion is ultrafiltered separately through a Millipore Pellicon ultrafilter equipped with three 100,000 molecular weight cutoff cassettes (type PTHK) of 5 ft$^2$ area each. A 5.4 liter portion is concentrated to 1 liter retentate volume. The ultrafiltrate is collected and saved. The retentate is diluted back to its original volume with fresh 10 mM Borate buffer, pH 11.8, and mixed well. The batch is concentrated again to 1 liter retentate volume. The ultrafiltrate is collected and combined with the first ultrafiltrate. When the running total of the OD's in the ultrafiltrates equals 20% of the OD's initially charged to the ultrafilter, the retentate volume on the next concentration step is taken to 0.5 liters instead of 1 liter. The cycle of concentration and dilution with 10 mM Borate buffer is continued until the ultrafiltrate from a retentate volume of 0.5 liters has an absorbance at 280 nm (1-cm cell) of less than 0.1. This normally takes between 9 and 12 cycles of concentration and dilution. The final retentate is discarded.

All ultrafiltrates are combined and adjusted to pH 9.0 with 6N HCl. The other 5.4-liter portions are ultrafiltered in the same fashion, and all pH adjusted ultrafiltrates are combined. A typical run produces a total of 380 liters of ultrafiltrates with an absorbance of 0.26 equivalent to 100,000 OD's and requires 24 to 40 hours to complete.

The combined ultrafiltrates (380 liters containing 100,000 OD's at 280 nm) from the 100K ultrafiltration step are loaded onto a Sepharose CL-6B DEAE ion-exchange column at a linear flow velocity of 23 cm/hr (25 liter/hr). The 37-cm diameter 15-cm high column is washed with two bed volumes (32 L) of 10 mM Borate buffer at pH 9.0. The eluate from the loading and washing steps is discarded. A step change in eluent to 10 mM Borate, 100 mM sodium chloride, pH 9, displaces the bGH off the column. The elution flow velocity is 23 cm/hr. The progress of the run is monitored by following absorbance of the eluate at 280 nm. The bGH peak is collected in 4 to 5 bed volumes (84 litres containing 43,000 OD's at 280 nm) and then concentrated to approximately 10 mg/ml using a Millipore Pellicon ultrafiltration device with 10,000 molecular weight cutoff cassettes. The solution is then lyophilized. The yield is approximately 70 g of pure bGH.

EXAMPLE 6

Activity Of bGH Analog Produced By pRO12

1. Radioimmunoassay Comparison of bGH Analog with Natural bGH

A solution containing 100 ng/ml bGH analog was prepared in phosphate buffered saline (including 1% BSA). This solution was diluted serially to concentrations of 50, 25, 12.5, 6.25, 3.12, 1.56 and 0.78 ng/l. Duplicate 0.1 ml aliquots of these solutions were submitted to RIA using a double antibody procedure. The dilution curve was comparable to that obtained with natural bGH.

2. Radioreceptor Binding Assay

A radioreceptor binding assay was performed with rabbit liver membranes as described by Tushima, T. and Freisen, H.G., (Y. Chin., Endocr. Metab. (1973), 37, 3) using $^{125}$I-bGH as the tracer and authentic bGH solutions for the construction of calibration curves. Samples were incubated in triplicate for two hours at room temperature in 0.3 ml of assay buffer (50 mM Tris, 15 mM CaCl$_2$ and 5 mg/ml bovine serum albumin, pH 7.6). The tubes contained $^{125}$I-bGH (20,000 cpm of preparation of 30 - 60 $\mu$ci/$\mu$g), 150 - 250 $\mu$g liver membrane protein and either natural bGH (1 - 100 ng) or extracts of bacterial bGH. The result demonstratred that the bGH activity of the bGH analog is comparable to that of natural bGH.

3. Tibia Test

The bioactivity of the pRO12 produced bGH analog recovered from bacterial cells according to Example 5 was evaluated by a tibia test. (Parlow, A.F., et al., Endocrinology (1965) 77, 1126). Rats were hypophysectomized at 28 - 30 days of age, then kept for 10 - 14 days without treatment. Bovine growth hormone derived from bovine pituitaries or from recombinant Escherichia coli was dissolved in 0.15 M NaCl + 0.01 M borate, pH 10.0. Rats (4 - 7 per group) received daily subcutaneous injections of bGH solutions (5 - 125 $\mu$g/day in 0.2 cc) for 5 days while kept on a normal diet (Purina Rat-Chow and water adlibitum). The animals were sacrificed on the 6th day, their foreleg knee-bones taken out, cut longitudinally, fixed with acetone and stained with 2% AgNO$_3$. The width of the epiphyseal plates was measured by observation through a dissecting binocular (Nikon). Mean values (40 readings per rat) were used for the constructon of long dose-response curves. The results demonstrated that the bGH activity of the pRO12-produced bGH analog is comparable to that of natural bGH.

EXAMPLE 7

Growth Of SOD$\beta_1$T$_{11}$

1. Stock Cultures

Stock cultures of pSOD$\beta_1$T$_{11}$ were grown on casein medium (see Inoculum), then diluted two-fold with freezing medium and stored at -80°C. Freezing medium contains per 500 ml:

| $K_2HPO_4$ | 6.3 gr |
|---|---|
| $KH_2PO_4$ | 1.8 gr |
| Na Citrate | 0.45 gr |
| $MgSO_4 \cdot 7H_2O$ | 0.09 gr |
| $(NH_4)_2SO_4$ | 0.9 gr |
| Glycerol | 44.0 gr |

II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was innoculated with 2 - 10% innoculum and incubated 15 hours at 30°C, pH 7±0.5 with agitation and aeration to maintain a dissolved oxygen level above 20% air saturation.

III. Production

The production medium contains:

| Casein hydrolysate | 20 g/l |
|---|---|
| Yeast extract | 10 g/l |
| $K_2HPO_4$ | 2.5 g/l |
| $MgSO_4 \cdot 7H_2O$ | 1 g/l |
| NaCl | 5 g/l |
| Biotin | 0.1 mg/l |
| Thiamine | 1 mg/l |
| Trace elements solution | 3 ml/l |
| $CuSO_4$ | 0.8 g/l |
| $ZnSO_4$ | 10 mg/l |

The medium also contains 12.5 mg/liter tetracycline. The tetracycline is optional for production, but is always found in the medium used for growing the inoculum.

Biotin, thiamine and tetracycline in concentrated solution were filter sterilized separately and added to the sterile production medium before inoculation. Sterile glucose solution was added initially to supply 10 g/l. At the induction step another 10 g/l of glucose was added.

The trace elements solution contains:

| $FeCl_3$ | 16 g/l |
|---|---|
| $ZnCl_2 \cdot 4H_2O$ | 2 g/l |
| $CoCl_2 \cdot 6H_2O$ | 2 gf/l |
| $Na_2MoO_4 \cdot 2H_2O$ | 2 g/l |
| $CaCl_2 \cdot 2H_2O$ | 1 g/l |
| $CuCl_2$ | 1 g/l |
| $H_3BO_3$ | 0.5 g/l |
| Conc. HCl | 100 ml/l |

The medium is inoculated with 0.5 - 10% inoculum culture and incubated at 30°C. Agitation-aeration rates are set to maintain a dissolved oxygen level above 20% air saturation. The pH is maintained at 7±0.2 with $NH_3$. Once cell concentration reaches about 3.5 g/l ($OD_{660}$ = 10) induction is started.

The temperature is raised to 42°C and maintained at 42°C for 1 - 5 hours. The culture is then chilled and cells are recovered by centrifugation for enzyme purification.

Recovery Of SOD

One and one-half kilograms of bacterial cells (wet cake) are suspended in 12 liters of 50 mM sodium phosphate (pH 7.8), in a Polytron (Kinematica) blender while controlling the speed to minimize foaming. The homogeneous suspension is continuously passed through a Dynomill cell disrupter KD5 (Willy, A. Bachofen, Basel). The homogeneous suspension of disrupted cells is sonicated using a continuous flow cell and centrifuged in a CEPA 101 centrifuge. The supernatant is heated for 2 hours at 65°C, cooled and centrifuged as before. The clear supernatant is concentrated to 1 liter in a Millipore Pellicon ultrafiltration device using 10,000 molecular weight cutoff cassettes (type PTGC). The concentrated protein solution is passed through a DEAE-Sepharose column (2 Kg DEAE Sepharose) equilibrated with 150 mM sodium phosphate buffer (pH 7.8). The flow through solution is collected, concentrated and dialyzed in a Pellicon ultrafiltration device against 20 mM Tris-HCl, pH 7.8, and then applied on to a QAE-Sepharose column equilibrated with 20 mM Tris-HCl buffer. The column is developed with a 20 mM Tris HCl buffer, pH 7.8, and a salt gradient (0 - 200 mM NaCl). SOD-containing fractions are collected, concentrated using a Pellicon ultrafiltration device, dialzed against distilled water and then brought to 100 mM sodium acetate by adding 1 M sodium acetate buffer, pH 4.8. The protein solution is then further separated on a CM-Sepharose column equilibrated with 100mM sodium acetate buffer, pH 4.7. The column is developed using the same buffer and a salt gradient (100-500mM NaCl). SOD containing fractions are collected, concentrated using a Pellicon ultrafilter device and lyophilized.

## EXAMPLE 8

### Activity Of SOD Produced By pSOD$\beta_1$T$_{11}$

The enzymatic activity of the SOD analog produced by pSOD$\beta_1$T$_{11}$ prepared in Example 7 was assayed by monitoring the inhibition of reduction of ferricytochrome-c as described by McCord and Fridovich, J. Biol. Chem. (1969), 244: 6049-6055. The results demonstrated that the activity of pSOD$\beta_1$T$_{11}$-produced SOD analog was comparable to that of natural human SOD (Sigma) and to that of bovine SOD (Orgotein: Grunenthal GMBH).

## EXAMPLE 9

### Expression Vectors

#### I. p579

The vector p579, shown in Fig. 19 and described in detail under Description of the Figures, is composed of a P$_L$ promoter, and N utilization site (Nut$_L$), the C$_{II}$ ribosomal binding site bounded by unique EcoRI and NdeI restriction sites, an ATG initiation codon and the T$_1$T$_2$ transcription termination signals derived from the end of the rrnB ribosomal RNA gene operon of Escherichia coli. These elements are cloned on pBR322 carrying the ampicillin resistance gene. Other features are shown in Fig. 19.

p579 was prepared by inserting the T$_1$T$_2$ transcription termination signals contained on the plasmid pPS1 into the HindIII site of the vector pRO211. pRO211 is shown in Fig. 2. pPS1 has been described in Sarmientos, et al., Cell (1983) 32, 1337-1346 and has been deposited with the American Type Culture Collection under ATCC Number 39807. p579 and its derivatives containing eucaryotic genes may be maintained in suitable Escherichia coli hosts. The most important feature of the host is that it provides the thermosensitive repressor cI857 and the antitermination N protein. (Gottesman, M. et al., J. Mol. Biol. (1980) 140, 57-75).

p579 has numerous advantages over previously described expression vectors including:

#### 1. extremely high levels of expressions

This vector is capable of directing expression of foreign proteins in E. coli at levels as high as 42% of the total cellular protein. This level of expression is higher than that described for other similar λP$_L$ plasmids lacking the T$_1$T$_2$ transcription termination sequences.

#### 2. transcription termination signals

The vector p579 contains the T$_1$T$_2$ transcription termination signals placed "downstream" from the λP$_L$ promoter and C$_{II}$ ribosomal binding site. The high levels of expression which are obtained when using this vector, are due in part to the presence of the $T_1T_2$ transcription terminators at the end of the inserted gene, as the $T_1T_2$ transcription terminators are capable of terminating transcription of N modified RNA polymerase. Thus the transcription terminators prevent the $\lambda P_L$ controlled transcription of undesired plasmid proteins, thereby enhancing the relative yields of the desired protein.

### 3. replaceable ribosomal binding sites

p579 contains a unique EcoRI site which is located "upstream" of the ribosomal binding site, and a unique NdeI site located at the ATG initiation codon. Thus, the ribosomal binding site is bounded by two unique restriction sites. This enables facile excision of the present ribosomal binding site (the $\lambda C_{II}$ ribosomal binding site) and substitution of virtually any other natural or synthetic ribosomal binding site without altering other features of the plasmid. This greatly facilitates optimal expression of desired polypeptides.

### 4. thermoinducible regulation of expression

The $\lambda P_L$ promoter is inactive when the $C_I$ repressor is bound to it. The cI857 repressor is thermosensitive, that is, it binds to the promoter at 30°C but is inactivated at 42°C. Thus, by increasing the temperature of fermentation to 42°C the host bacteria are induced to produce the desired protein.

The advantages of such a system include the following:

(a) a foreign protein which is toxic to Escherichia coli can be produced late in the fermentation process thus avoiding early cell death.

(b) overproduction of a protein may stabilize the protein and prevent proteolytic degradation (Cheng, Y.E., et al., Gene (1981) 14, 121). Thus, "instantaneous" overproduction using a tightly regulated promoter such as $\lambda P_L$ may be preferable to continuous low level production.

### 5. simplified induction protocol

The plasmids derived from p579 are induced at about 42°C and maintained at 42°C throughout the period of protein synthesis. The induction protocol for plasmids derived from pMG100 and pND5 described in copending, coassigned U.S. Patent Application Serial No. 514,188 requires a temperature shift to 42°C followed by an extended period of growth at 38°C. The optimal induction protocol for p579 does not require the cooling step to 38°C and is thus simplified.

### 6. high copy number

The $\lambda P_L$ promoter in p579 is found on a plasmid with a copy number higher than that of $\lambda$ transducing phage vectors which are used in Escherichia coli. This increases expression levels.

### 7. ribosome binding site and initiation codon

This expression vector contains a strong procaryotic ribosomal binding site (RBS) as well as a translation initiation codon (ATG). Thus, any eucaryotic gene may be cloned without adding an initiation codon. Furthermore, the efficient RBS increases levels of expression. The ribosome binding site is the $\lambda C_{II}$ ribosomal binding site which we previously cloned into the vector pND5. The sequence of the ribosomal binding site is

TAAGGAAGTACTTACAT

ATTCCTTCATGAATGTA.

One base pair is different from the ribosomal binding site found in wild type $\lambda$.

### 8. convenient restriction site

The expression vector has a unique NdeI restriction site which contains within it, the ATG initiation codon. This permits proper positioning of the desired gene. The unique NdeI site is found immediately after the ribosomal binding site.

9. convenient restriction sites for gene insertion

Located 116 base pairs downstream of the NdeI restriction site are unique restriction sites BglII and SmaI, in that order. These unique restriction sites enable facile insertion of desired genes.

10. nut site

N protein, which is provided by the host, binds to the Nut site on the expression vector and thereby prevents termination of transcription at the $t_{RI}$ site or premature transcription termination within the cloned gene.

Strains

Suitable hosts for the described vectors and plasmids are strains of Escherichia coli suitable for transformation, including A1637, A2602, A1563, A1645 (c600 $r^-m^+$ $gal^+$ $thr^-$ $leu^-$ $lac^-bl$ ($\lambda$c1857 $\Delta$HI $\Delta$BamHI $N^+$) and A2097 (A1645 lac $\Delta_\chi$A21 proC::Tn 10).

EXAMPLE 10

Animal Growth Hormones

I. pHG44

The construction of pHG44 is shown in Fig. 6, described in the Description of the Figures and deposited under ATCC No. 39806. The plasmid was derived from the pRO12 plasmid shown in Fig. 2 by insertion of the $T_1T_2$ transcription termination sequences from the plasmid pPS1 which is shown in Fig. 6, described in Sarmientos, et al., Cell (1983) 32, 337-1346 and deposited under ATCC No. 39807. The presence of the $T_1T_2$ termination sequences prevents long run-on mRNA transcripts, and thus prevents high-level expression of the $\beta$-lactamase gene and possibly other undesired proteins under the control of the $\lambda P_L$ promoter.

The plasmid pHG44 has been introduced into Escherichia coli strain A2097 by transformation using methods known to those of ordinary skill in the art. This strain produced upon growth and induction an analog of bovine growth hormone (bGH) having the amino acid sequence met-asp-gln added to the amino-terminus of the phenylalanine form of the authentic bGH. The amount of bGH analog produced by pHG44 was about 37 - 42% of the total protein produced by the bacteria as calculated by scanning Coomasie-stained SDS polyacrylamide gels.

The methods used to grow the strain, recover the bGH analog produced and purify the bGH analog, are described in Example 13. The level of expression is higher than that obtained from PRO12 (Table I) due to a significant reduction in $\beta$-lactamase expression effected by the introduction of the $T_1T_2$ termination sequences at the 3' terminus of the bGH gene.

II. pSAL 5600-1

The construction of pSAL 5600-1 is shown in Fig. 10 and described in the Description of the Figures. The plasmid pSAL 5600-1 was derived from pSAL 5200-6 (shown in Fig. 3) by insertion of the $T_1T_2$ termination sequences from the plasmid pPS1 (ATCC No. 39807).

The plasmid pSAL 5600-1 was introduced into Escherichia coli strain A1645 by transformation using methods known to those of ordinary skill in the art. This strain produced upon growth and induction an analog of bGH having the amino acid methionine added to the amino-terminus of the phenylalanine form of natural bGH. The amount of bGH analog produced by pSAL 5600-1 strains was about 22 - 28% of the total protein produced by the bacteria as calculated by scanning Coomasie-stained SDS polyacrylamide gels. The methods used to grow the strain, recover the bGH analog produced and purify the bGH analog, are the same as those described for pHG44 in Example 13.

The level of expression was higher than that obtained from pSAL 5200-6 strains due to a significant reduction in $\beta$-lactamase expression effected by the introduction of the $T_1T_2$ termination sequences at the 3' terminus of the bGH gene.

III. p3009

The construction of p3009 is shown in Fig. 11 and described in the Description of the Figures. The plasmid p3009 was obtained by insertion of the NdeI-NdeI porcine growth hormone cDNA fragment into the unique NdeI site of the p579 expression vector (Fig. 19). The porcine growth hormone (pGH) fragment was isolated from p3008 (ATCC No.39804) by an NdeI digestion.

The plasmid p3009 was introduced into Escherichia coli strain A1645 by transformation using methods known to those of ordinary skill in the art. This strain produced upon growth and induction an analog of pGH having the amino acid methionine added to the amino-terminus of the phenylalanine form of the natural pGH. The amount of pGH analog produced was about 30 - 35% of the total protein produced by the bacteria as calculated by scanning Coomasie-stained SDS polyacrylamide gels. The methods used to grow the strain, recover the pHG analog produced and purify the pGH analog, are the same as those described for pHG44 in Example 13.

The level of expression of p3009 was higher than that obtained from p3008 strains due to a significant reduction in $\beta$-lactamase expression effected by the introduction of the $T_1 T_2$ termination sequences at the 3' terminus of the pGH gene.

### IV. p5003

The construction of p5003 is shown in Fig. 12 and described in the Description of the Figures. p5003 has been deposited with the American Type Culture Collection under ATCC No. 39792 . The plasmid was obtained by insertion of the NdeI-NdeI chicken growth hormone cDNA fragment from p5002 into the unique NdeI site of the p579 expression vector.

The plasmid p5003 was introduced into Escherichia coli strain A2097 by transformation using methods known to those of ordinary skill in the art. This strain produced upon growth and induction an analog of chicken growth hormone (cGH) having the amino acid methionine added to the amino-terminus of the phenylalanine form of the natural cGH. The amount of cGH analog produced was about 30 - 35% of the total protein produced by the bacteria as calculated by scanning Coomasie-stained SDS polyacrylamide gels. The methods used to grow the strain recover the cGH analog produced and purify the cGH analog, are the same as those described for pHG44 in Example 13.

The level of expression of p5003 was higher than that obtained from p5002 strains due to a significant reduction in $\beta$-lactamase expression effected by the introduction of the $T_1 T_2$ termination sequences at the 3' terminus of the cGH gene.

### EXAMPLE 11

### Human Cu-Zn Superoxide Dismutase (SOD)

### I. pSOD$\beta_1$TT-1

The construction of pSOD$\beta_1$TT-1 is shown in Fig. 16 and described in the Description of the Figures. The plasmid pSOD$\beta_1$TT-1 was obtained by insertion of the $T_1 T_2$ termination sequences at the 3' end of the SOD gene found in pSOD$\beta_1 T_{11}$ (Fig. 15).

The plasmid pSOD$\beta_1$TT-1 was introduced into Escherichia coli strain A1645 by transformation using methods known to those of ordinary skill in the art. This strain produced upon growth an SOD analog. The amount of SOD analog produced was about 10 - 15% of the total protein produced by the bacteria as calculated by scanning Coomasie-stained SDS polyacrylamide gels.

The methods used to grow the strain, recover the SOD analog produced and purify the SOD analog, are described in Example 15.

The level of expression of pSOD$\beta_1$TT-1 was higher than that obtained from pSOD$\beta_1 T_{11}$ (Table II) due to the $T_1 T_2$-induced reduction in transcription and translation of non-desired DNA sequences.

The human Cu-Zn SOD analog produced differs from natural human Cu-Zn SOD in that the amino terminus alanine is not acetylated, as demonstrated by amino acid sequencing stoichiometry. The natural human SOD is acetylated at the amino terminus alanine (Hartz, J.W. and Deutsch, H.F., J. Biol. Chem. (1972) 247, 7043-7050, Jabusch, J.R., et al., Biochemistry (1980) 19, 2310-2316; Barra, et al., FEBS Letters (1980) 120, 53 and Oberley, L.W., Superoxide Dismutase, Vol. I, CRC Press, Florida, (1982), pp. 32-33). The natural human SOD is glycosylated (Huber, W., U.S. Patent No. 3,579,495, issued May 18, 1971). Bacterial-produced human SOD is almost certainly not glycosylated as Escherichia coli does not glycosylate proteins which it produces. The amino acid sequence of the bacterial-produced SOD analog is identical to that of mature human SOD and does not contain a methionine residue at its N-terminus.

### EXAMPLE 12

### Human Apolipoprotein E3 (Apo-E3)

#### I. pTV-170

The construction of pTV-170 is shown in Fig. 20 and described in the Description of the Figures. The plasmid PTV-170 was obtained by insertion of the NdeI-NdeI Apo-E3 fragment derived from pApoE-EX2 (ATCC No. 39787) into the unique NdeI site of the expression vector p579.

pTV-170 was introduced into Escherichia coli strain A1645 by transformation using methods known to those of ordinary skill in the art. The clone obtained produced upon growth human ApoE3, presumably having the amino acid methionine added to the amino-terminus of natural human ApoE3. The amount of human ApoE3 analog produced was about 1% of the total protein produced by the bacteria as calculated by scanning of Coomasie-stained SDS polyacrylamide gels. The methods used to grow the strain are described in Example 17.

#### II. pTV-194

The construction of pTV-194 is shown in Fig. 22 and is described in the Description of the Figures. pTV-194 was derived from pTV-170 (Fig. 20) by replacing the $C_{II}$ ribosomal binding site with the $\beta$-lactamase promoter and ribosomal binding site derived from pBLA11. pBLA11 contains the promoter and ribosomal binding site of the $\beta$-lactamase gene found in pBR322 between coordinates 4157 and 4353. An EcoRI linker was added upstream of the promoter and a multi-restriction site linker was added immediately after the initiation codon ATG. Thus the sequence of the coding strand beginning with the initiation codon is ATGAGCTCTAGAATTC. pBLA11 was deposited in the American Type Collection Center as ATCC No. 39788.

pTV-194 was introduced into Escherichia coli strain A1645 by transformation using methods known to those of ordinary skill in the art. The clone obtained produced upon growth an analog of human ApoE3, presumably having the amino acid methionine added to the amino-terminus of natural human ApoE3. The amount of human Apo-E analog produced was about 3% of the total protein produced by the bacteria as calculated by scanning Coomasie-stained SDS polyacrylamide gels. The methods used to grow the strain are the same as described for pTV-170 in Example 17.

#### III. pTV-214

The construction of pTV-214 is shown in Fig. 24 and is described in the Description of the Figures. pTV-214 was derived from pTV-190 (shown in Fig. 21 and described in the Description of the Figures) by insertion of a synthetic DNA fragment coding for the 14 amino acid amino-terminal sequence of human growth hormone into the unique NdeI site of pTV-190.

pTV-214 was introduced into Escherichia coli strain A1645 by transformation using methods known to those of ordinary skill in the art. The clone obtained produced upon growth and induction an analog of human ApoE3 having at its amino terminus methionine followed by the 13 amino acid amino-terminal sequence of human growth hormone, followed by methionine attached to the sequence of mature human ApoE3. The amount of human ApoE analog produced was about 2% of the total protein produced by the bacteria as calculated by scanning Coomasie-stained SDS polyacrylamide gels. The methods used to grow the strain are the same as those described for pTV-170 in Example 17.

### EXAMPLE 13

### Growth of pHG44

#### I. Stock Cultures

Stock cultures of pHG44 were grown on casein medium (see Inoculum), then diluted two-fold with freezing medium and stored at -80°C. Freezing medium contains per 500 ml:

| $K_2HPO_4$ | 6.3 g |
| $KH_2PO_4$ | 1.8 g |
| Na Citrate | 0.45 g |
| $MgSO_4 \cdot 7H_2O$ | 0.09 g |
| $(NH_4)_2SO_4$ | 0.9 g |
| Glycerol | 44.0 g |

II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was inoculated with 2-10 inoculum culture and incubated 15 hours at 30°C, pH 7 ± 0.5 with agitation and aeration to maintain a dissolved oxygen level above 20% air saturation.

III. Production

The production medium contains:

| Casein hydrolysate | 20 g/l |
| Yeast extract | 10 g/l |
| $K_2HPO_4$ | 2.5 g/l |
| $MgSO_4 \cdot 7H_2O$ | 1 g/l |
| NaCl | 5 g/l |
| Biotin | 0.1 mg/l |
| Thiamine | 1 mg/l |
| Trace elements solution | 3 ml/l |

The medium also contains 100 mg/liter ampicillin. The ampicillin is optional for production but is always found in the medium used for growing the inoculum.

Biotin, thiamine, and ampicillin in concentrated solutions were filter sterilized separately and added to the sterile production medium before inoculation. Sterile glucose solution was added initially to supply 10 g/l. At the induction step another 10 g/l of glucose was added.

The trace elements solution contains:

| $FeCl_3$ | 16 g/l |
| $ZnCl_2 \cdot 4H_2O$ | 2 g/l |
| $CoCl_2 \cdot 6H_2O$ | 2 g/l |
| $Na_2MoO_4 \cdot 2H_2O$ | 2 g/l |
| $CaCl_2 \cdot 2H_2O$ | 1 g/l |
| $CuCl_2$ | 1 g/l |
| $H_3BO_3$ | 0.5 g/l |
| Conc. HCl | 100 ml/l |

The medium is inoculated with 0.5 - 10% inoculum culture and incubated at 30°C. Agitation-aeration rates are set to maintain dissolved oxygen level above 20% air saturation. The pH is maintained at 7±0.2 with $NH_3$. Once cell concentration reaches about 3.5 g/l ($OD_{660}$ = 10) induction is started.

The temperature is raised to 42°C and maintained at 42°C for 1 - 5 hours. The culture is then chilled and cells are recovered by centrifugation for hormone purification.

Recovery of bGH

Thirteen kilograms of bacterial cells (wet cake) are resuspended in 5 volumes of a solution containing 50 mM sodium phosphate buffer (pH 7.4), 50 mM EDTA and 100 mM NaCl, using a Polytron (Kinematica) blender, while controlling the blender's speed to minimize foaming. The homogeneous suspension is continuously passed through a Dynomill cell disruptor KD5 (Willy A. Bachofen, Basel) at a rate of 80 liter per hour and the homogeneous suspenion of disrupted cells clarified by centrifugation in a CEPA 101 centrifuge at a flow rate of 45 liter per hour. The precipitate from the centrifugation step was collected and resuspended in 15.5 liters of 50 mM sodium phosphate buffer (pH 7.4) containing 50 mM EDTA. Lysozyme is added to a final concentration of 0.05 mg/ml and the suspension incubated for 16 hours at 37°C. Triton X-100 is added to a final concentration of 1%. The suspension is then incubated for 30 min at room temperature, sonicated in a continuous flow cell sonificator (Heat System) at a rate of 18 liters per hour and centrifuged in a CEPA 101 centrifuge. The precipitate is collected, resuspended in 50 mM sodium phosphate buffer (pH 7.4), sonicated as above, and centrifuged in a CEPA 101 centrifuge. The cells are resuspended in 15.5 liters of 50 mM sodium phosphate buffer (pH 7.4) containing 50 mM EDTA and 100 mM NaCl and twice precipitated and resuspended in 15.5 liters of distilled water. The precipitate is collected by centrifugation and stored at -20°C.

## Purification of bGH

The precipitate is resuspended in 30 - 40 liters distilled water and solubilized by titration with 0.5 N NaOH to pH 11.8. The solution is then continuously sonicated and clarified by centrifugation in CEPA 101 centrifuge if necessary, or filtered through Whatman No. 1 paper.

The clarified protein solution (32.6 liters containing 297,000 OD's at 280 nm) is divided into separate portions (6 x 5.4 liters) each containing 50,000 - 60,000 OD's. Each portion is ultrafiltered separately through a Millipore Pellicon ultrafilter equipped with three 100,00 molecular weight cutoff cassettes (type PTHK) of 5 ft$^2$ area each. A 5.4 liter portion is concentrated to 1 liter retentate volume. The ultrafiltrate is collected and saved. The retentate is diluted back to its original volume with fresh 10 mM Borate buffer pH 11.8, and mixed well. The batch is concentrated again to 1 liter retentate volume. The ultrafiltrate is collected and combined with the first ultrafiltrate. When the running total of the OD's in the ultrafiltrates equals 20% of the OD's initially charged to the ultrafilter, the retentate volume on the next concentration step is take: to 0.5 liters instead of 1 liter. The cycle of concentration and dilution with 10 mM Borate buffer is continued until the ultrafiltrate from a retentate volume of 0.5 liters has an absorbance at 280 nm (l-cm cell) of less than 0.1. This normally takes between 9 and 12 cycles of concentration and dilution. The final retentate is discarded.

All ultrafiltrates are combined and adjusted to pH 9.0 with 6N HCl. The other 5.4-liter portions are ultrafiltered in the same fashion, and all pH adjusted ultrafiltrates are combined. A typical run produces a total of 380 liters of ultrafiltrates with an absorbance of 0.26 equivalent to 100,000 OD's and requires 24 to 40 hours to complete.

The combined ultrafiltrates (380 liters containing 100,000 OD's at 280 nm) from the 100K ultrafiltration step are loaded onto a Sepharose CL-6B DEAE ion-exchange column at a linear flow velocity of 23 cm/hr (25 liter/hr). The 37-cm diameter 15-cm high column is washed with two bed volumes (32 L) of 10 mM Borate buffer at pH 9.0. The eluate from the loading and washing steps is discarded. A step change in eluent to 10 mM Borate, 100 mM sodium chloride, pH 9, displaces the bGH off the column. The elution flow velocity is 23 cm/hr. The progress of the run is monitored by following absorbance of the eluate at 280 nm. The bGH peak is collected in 4 to 5 bed volumes (84 liters containing 43,000 OD's at 280 nm) and then concentrated to approximately 10 mg/ml using a Millipore Pellicon ultrafiltration device with a 10,000 molecular weight cutoff cassettes. The solution is then lyophilized. The yield is approximately 70 g of pure bGH.

## EXAMPLE 14

Activity of bGH Analog Produced by pHG44

### 1. Radioimmunoassay Comparison of bGH Analog with Natural bGH

A solution containing 100 ng/ml bGH analog was prepared in phosphate buffered saline (1% BSA). This solution was diluted serially to concentrations of 50, 25, 12.5, 6.25, 3.12, 1.56 and 0.78 ng/l. Duplicate 0.1 ml aliquots of these solutions were submitted to RIA using a double antibody procedure. The dilution curve was comparable to that obtained with natural bGH.

2. Radioreceptor Binding Assay

A radioreceptor binding assay was performed with rabbit liver membranes as described by Tushima, T. and Freisen, H.G., (Y. Chin., Endocr. Metab. (1973), 37, 3) using $^{125}$I-bGH as the tracer and authentic bGH solutions for the construction of calibration curves. Samples were incubated in triplicate for two hours at room temperature in 0.3 ml of assay buffer (50 mM Tris, 15 mM CaCl$_2$ and 5 mg/ml bovine serum albumin, pH 7.6). The tubes contained $^{125}$I-bGH (20,000 cpm of preparation of 30 - 60 $\mu$ci/$\mu$g), 150 - 250 $\mu$g liver membrane protein and either natural bGH (1 - 100 ng) or extracts of bacterial bGH. The result demonstrates that the bGH activity of the bGH analog is comparable to that of natural bGH.

3. Tibia Test

The bioactivity of the pRO12 produced bGH analog recovered from bacterial cells according to Example 13 was evaluated by a tibia test. (Parlow, A.F., et al., Endocrinology (1965) 77, 1126). Rats were hypophysectomized at 28 - 30 days of age, then kept for 10 - 14 days without treatment. Bovine growth hormone derived from bone pituitaries or from recombinant Escherichia coli was dissolved in 0.15 M NaCl with 0.01 M borate, pH 10.0. Rate (4 - 7 per group) received daily subcutaneous injections of bGH solutions (5 - 125 $\mu$g/day in 0.2 cc) for 5 days while kept on a normal diet (Purina Rat-Chow and water adlibitum). The animals were sacrificed on the 6th day, their foreleg knee-bones taken out, cut longitudinally, fixed with acetone and stained with 2% AgNO$_3$. The width of the epiphyseal plates was measured by observation through a dissecting binocular (Nikon). Mean values (40 readings per rat) were used for the constructon of long dose-response curves. The results demonstrated that the bGH activity of the pHG44-produced bGH analog is comparable to that of natural bGH.

EXAMPLE 15

Growth of pSOD$\beta_1$TT-1

1. Stock Cultures

Stock cultures of pSOD$\beta_1$T$_{11}$ were grown on casein medium (see Inoculum), then diluted two-fold with freezing medium and stored at -80°C. Freezing medium contains per 500 ml:

| | |
|---|---|
| K$_2$HPO$_4$ | 6.3 g |
| KH$_2$PO$_4$ | 1.8 g |
| Na Citrate | 0.45 g |
| MgSO$_4$·7H$_2$O | 0.09 g |
| (NH$_4$)$_2$SO$_4$ | 0.9 g |
| Glycerol | 44.0 g |

II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was inoculated with 2 - 10% innoculum and incubated 15 hours at 30°C, pH 7±0.5 with agitation and aeration to maintain a dissolved oxygen level above 20% air saturation.

III. Production

The production medium contains:

| Casein hydrolysate | 20 g/l |
| Yeast extract | 10 g/l |
| $K_2HPO_4$ | 2.5 g/l |
| $MgSO_4 \cdot 7H_2O$ | 1 g/l |
| NaCl | 5 g/l |
| Biotin | 0.1 mg/l |
| Thiamine | 1 mg/l |
| Trace elements solution | 3 ml/l |
| $CuSO_4$ | 0.8 g/l |
| $ZnSO_4$ | 10 mg/l |

The medium also contains 12.5 mg/liter tetracycline. The tetracycline is optional for production, but is always found in the medium used for growing the inoculum.

Biotin, thiamine and tetracycline in concentrated solution were filter sterilized separately and added to the sterile production medium before inoculation. Sterile glucose solution was added initially to supply 10 g/l. At the induction step another 10 g/l of glucose was added.

The trace elements solution contains:

| $FeCl_3$ | 16 g/l |
| $ZnCl_2 \cdot 4H_2O$ | 2 g/l |
| $CoCl_2 \cdot 6H_2O$ | 2 g/l |
| $Na_2MoO_4 \cdot 2H_2O$ | 2 g/l |
| $CaCl_2 \cdot 2H_2O$ | 1 g/l |
| $CuCl_2$ | 1 g/l |
| $H_3BO_3$ | 0.5 g/l |
| Conc. HCl | 100 ml/l |

The medium is inoculated with 0.5 - 10% inoculum culture and incubated at 30°C. Agitation-aeration rates are set to maintain a dissolved oxygen level above 20% air saturation. The pH is maintained at 7±0.2 with $NH_3$. Once cell concentration reaches about 3.5 g/l ($OD_{660}$ = 10) induction is started.

The temperature is raised to 42°C and maintained at 42°C for 1 - 5 hours. The culture is then chilled and cells are recovered by centrifugation for enzyme purification.

Recovery Of SOD

One and half kilograms of bacterial cells (wet cake) are suspended in 12 liters of 50 mM sodium phosphate (pH 7.8), in a Polytron (Kinematica) blender while controlling the speed to minimize foaming. The homogeneous suspension is continuously passed through a Dynomill cell disrupter KD5 (Willy, A. Bachofen, Basel). The homogeneous suspension of disrupted cells is sonicated using a continuous flow cell and centrifuged in a CEPA 101 centrifuge. The supernatant is heated for 2 hours at 65°C, cooled and centrifuged as before. The clear supernatant is concentrated to 1 liter in a Millipore Pellicon ultrafiltration device using 10,000 molecular weight cutoff cassettes (type PTGC). The concentrated protein solution is passed through a DEAE-Sepharose column (2 Kg DEAE Sepharose) equilibrated with 150 mM sodium phosphate buffer (pH 7.8). The flow through solution is collected, concentrated and dialyzed in a Pellicon ultrafiltration device against 20 mM Tris-HCl, pH 7.8, and then applied on to a QAE-Sepharose column equilibrated with 20 mM Tris-HCl buffer. The column is developed with a 20 mM Tris HCl buffer, pH 7.8, and a salt gradient (0 - 200 mM NaCl). SOD-containing fractions are collected, concentrated using a Pellicon ultrafiltration device, dialzed against distilled water and then brought to 100 mM sodium acetate by adding 1 M sodium acetate buffer, pH 4.8. The protein solution is then further separated on a CM-Sepharose column equilibrated with 100mM sodium acetate buffer, pH 4.7. The column is developed using the same buffer and a salt gradient (100-500mM NaCl). SOD containing fractions are collected, concentrated using a Pellicon ultrafilter device and lyophilized.

EXAMPLE 16

Activity Of SOD Produced By pSOD$\beta_1$TT-1

The enzymatic activity of the SOD analog produced by pSOD$\beta_1$TT-1 prepared in Example 15 was assayed by monitoring the inhibition of reduction of ferricytochrome-c as described by McCord and Fridovich, J. Biol. Chem. (1969), 244, 6049-6055. The results demonstrated that the activity of pSOD$\beta_1$TT-1-produced SOD analog was comparable to that of natural human SOD and to that of bovine SOD (Orgotein: Grunenthal GMBH).

EXAMPLE 17

Growth of pTV-170

1. Stock Cultures

Stock cultures of pTV-170 were grown on casein medium (see Inoculum), then diluted two-fold with freezing medium and stored at -80°C. Freezing medium contains per 500 ml:

| | |
|---|---|
| $K_2HPO_4$ | 6.3 g |
| $KH_2PO_4$ | 1.8 g |
| Na Citrate | 0.45 g |
| $MgSO_4 \cdot 7H_2O$ | 0.09 g |
| $(NH_4)_2SO_4$ | 0.9 g |
| Glycerol | 44.0 g |

II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was inoculated with 2 - 10% innoculum and incubated 15 hours at 30°C, pH 7±0.5 with agitation and aeration to maintain a dissolved oxygen level above 20% air saturation.

III. Production

The production medium contains:

| | |
|---|---|
| Casein hydrolysate | 20 g/l |
| Yeast extract | 10 g/l |
| $K_2HPO_4$ | 2.5 g/l |
| $MgSO_4 \cdot 7H_2O$ | 1 g/l |
| NaCl | 5 g/l |
| Biotin | 0.1 mg/l |
| Thiamine | 1 mg/l |
| Trace elements solution | 3 ml/l |

The medium also contained 100 mg/liter ampicillin. The ampicillin is optional for production but is always found in the medium used for growing the inoculum.

Biotin, thiamine and ampicillin in concentrated solutions were filter sterilized separately and added to the sterile production medium before inoculation. Sterile glucose solution was added initially to supply 10 g/l. At the induction step another 10 g/l of glucose was added.

The trace elements solution contains:

| FeCl$_3$ | 16 g/l |
|---|---|
| ZnCl$_2 \cdot 4H_2O$ | 2 g/l |
| CoCl$_2 \cdot 6H_2O$ | 2 g/l |
| Na$_2$MoO$_4 \cdot 2H_2O$ | 2 g/l |
| CaCl$_2 \cdot 2H_2O$ | 1 g/l |
| CuCl$_2$ | 1 g/l |
| H$_3$BO$_3$ | 0.5 g/l |
| Conc. HCl | 100 ml/l |

The medium is inoculated with 0.5 - 10% inoculum culture and incubated at 30°C. Agitation-aeration rates are set to maintain dissolved oxygen level above 20% air saturation. The pH is maintained at 7±0.2 with NH$_3$. Once cell concentration reaches about 3.5 g/l (OD$_{660}$ = 10) induction is started.

The temperature is raised to 42°C and maintained at 42°C for 15 minutes. The culture is then chilled and cells are recovered by centrifugation for protein purification.

EXAMPLE 18

Bovine Growth Hormone

pHG50

The construction of pHG50 is shown in Fig. 6 and is described in the Description of the Figures. The plasmid pHG50 was obtained by insertion of the HpaII-HpaII λcI$^{434}$ fragment of pSK434 (ATCC No. 39784) into the unique ClaI site of pHG44 (ATCC No. 39806). The plasmid pHG51 was constructed in the same way. However, the λimm434 cI$^+$ fragment is found in the opposite orientation in the plasmid, as compared to plasmid pHG50.

pSK434 (ATCC No. 39784) was constructed by digesting λimm434 with BamHI and ligating the mixture to BamHI-digested pBR322. The ligated mixture was transformed into Escherichia coli A2097 and colonies immune to λimm434cI3003 phage were isolated. The plasmid pSK434 isolated from one of these colonies, contains a 6 kb BamHI fragment which contains the λcI434 gene and extends from beyond the N gene to beyond the P gene. pSK434 has the restriction map shown in Fig. 6.

pHG50 and pHG51 were introduced into Escherichia coli A1645 by transformation using methods known to those skilled in the art. Clones obtained, designated A3108 and A3112 respectively, produce upon growth and induction an analog of bGH having the amino acid sequence met-asp-gln added to the amino-terminus of the phenylalanine form of natural bGH. The amount of bGH analog produced was in the range of 37-42% of the total protein produced by the bacteria as calculated by scanning Coomasie-stained SDS polyacrylamide gels (Table I).

Prophage Introduction

In order to use the λcI434 selection system, the cells containing pHG50 must also contain prophage λimm434cI$^-$. We have used as a prophage λimm434 cI3003 mini Tn10Δ16Δ17. The mini Tn10Δ16Δ17 tetracycline resistance marker (Foster, et al., Cell (1981) 23, 201-213) was introduced into the phage in order to facilitate isolation of rare and stable prophage insertion events. It also permits a simple testing the presence of prophage by monitoring tetracycline resistance.

Escherichia coli strain A1645 containing the plasmid pHG50, grown in the presence of ampicillin, was infected at a low multiplicity of infection with λimm434 cI3003 mini Tn10Δ16 Δ17 at 30°C. Tetracycline resistant colonies were isolated and purified. This strain was deposited with the American Type Collection Center under ATCC No. 39805.

In an alternative method, the prophage was introduced by transforming Escherichia coli 1645 simultaneously with pHG50 and λimm434 cI3003 mini Tn10Δ16Δ17 and selecting for colonies which are both ampicillin and tetracycline resistant. Suitable hosts for the described vectors and plasmids are strains of Escherichia coli suitable for transformation, including A1637, A2602, A1563, A1645 (C600 ΔH ΔBamHI r$^-$m$^+$ gal$^+$ thr$^-$ leu$^-$ BI) or A2097 (A1645 lacΔ$_\chi$A21, proC::Tn10). The desired prophage can be introduced into all of the strains in the same manner as described above.

pHG50 and the vector which can be derived from it by excision of the bGH gene, have numerous advantages over previously described expression vectors including:

42

### 1. improved plasmid stability

The plasmid contains the $cI^{434}$ repressor gene which represses a λimm434ci⁻ lysogen. Loss of the plasmid results in bacterial cell lysis due to λimm434cI⁻ prophage induction. Thus the plasmid is stably maintained without resort to antibiotic selection schemes which are expensive. Table III demonstrates the increased stability of plasmids carrying $cI^{434}$ stabilization system.

The $cI^{434}$ plasmid stabilization system is compatible with the thermoinducible $\lambda P_L$ promoter expression system, this despite the fact that the thermolabile repressor for the $\lambda P_L$ promoter is $C_I$.

It should be noted that any λimm434cI⁻ prophage can be substituted for the λimm434cI3003 prophage. Further, any antibiotic resistance marker can substitute for the tetracycline resistance marker which we introduced on mini Tn10Δ16Δ17. The availability of λimm21 and λimm22 repressor negative mutants, allows the replacement of the λ434 system with a comparable system of phage 21 or phage 22.

### 2. extremely high levels of expression

This plasmid is capable of directing expression of foreign proteins in Escherichia coli at levels as high as 42% of the total cellular protein. This level of expression is higher than that described for other similar $\lambda P_L$ plasmids lacking the $T_1 T_2$ transcription termination sequence.

### 3. transcription termination signals

The plasmid contains the $T_1 T_2$ transcription termination signals placed "downstream" from the $\lambda P_L$ promoter and $C_{II}$ ribosomal binding site. The high levels of expression which are obtained when using this plasmid, may be in part to the presence of the $T_1 T_2$ transcription terminators at the end of the inserted gene, as the $T_1 T_2$ transcription terminators are capable of terminating transcription of N modified RNA polymerase. Thus the transcription terminators prevent the $\lambda P_L$ controlled transcription of undesired plasmid proteins, thereby enhancing the relative yields of the desired protein.

### 4. replaceable ribosomal binding site

pHG50 contains a unique EcoRI site which is located "upstream" of the ribosomal binding site, and an NdeI site located at the ATG initiation codon. Thus, the ribosomal binding site is bounded by two unique restriction sites. This enables facile excision of the present ribosomal binding site (the $\lambda C_{II}$ ribosomal binding site) and substitution of virtually any other natural or synthetic ribosomal binding site without altering other features of the plasmid. This greatly facilitates optimal expression of desired polypeptides.

### 5. thermoinducible regulation of expression

The $\lambda P_L$ promoter is inactive when the $C_I$ repressor is bound to it. The cI857 repressor is thermosensitive, that is, it binds to the promoter at 30°C but is inactivated at 42°C. Thus, by increasing the temperature of fermentation to 42°C the host bacteria are induced to produce the desired protein.

The advantages of such a system include the following:

(a) A foreign protein which is toxic to Escherichia coli can be produced late thus avoiding early cell death in the fermentation process.

(b) Overproduction of a protein may stabilize the protein and prevent proteolytic degradation. (Cheng, Y.E., et al., Gene (1981) 14, 121). Thus, "instantaneous" overproduction using a tightly regulated promoter such as $\lambda P_L$ may be preferable to continuous low level production.

### 6. simplified induction protocol

pHG50 is induced at about 42°C and maintained at 42°C throughout the period of protein synthesis. The induction protocol for plasmids derived from pMG100 and pND5 described in copending, coassigned U.S. patent application Serial No. 514,188 involved induction at 42°C followed by an extended period of growth at 38°C. The optimal induction protocol for pHG50 does not require the cooling step to 38°C and is therefore simplified.

### 7. high copy number

The λP$_L$ promoter in pHG50 is found on a plasmid with a copy number higher than the λ transducing phage vectors which are present in Escherichia coli. This increases expression levels.

### 8. ribosome binding site and initiation codon

This expression vector contains a strong procaryotic ribosomal binding site (RBS) as well as a translation initiation codon (ATG). Thus, any eucaryotic gene may be cloned without adding an initiation codon. Furthermore, the efficient RBS increases levels of expression. The ribosome binding site is the λC$_{II}$ ribosomal binding site. The sequence of the ribosomal binding site is:

$$\text{TAAGGAAGTACTTACAT}$$
$$\text{ATTCCTTCATGAATGTA}$$

One base pair is different from the ribosomal binding site found in the wild type λ.

### 9. convenient restriction site

The expression vector derived from the plasmid has a unique NdeI restriction site which contains within the site the ATG initiation codon. This permits proper positioning of the desired gene. The unique NdeI site is found immediately after the ribosomal binding site.

### 10. nut site

N protein, which is provided by the host, binds the Nut site on the expression vector and thereby prevents termination of transcription at the t$_{RI}$ site or premature transcription termination within the cloned gene.

TABLE III

| Plasmid Stabilization Resulting from the cl$^{434}$ System | | | |
|---|---|---|---|
| Strain | Plasmid | Host | % of Cells Without Plasmid |
| A3102 | pHG44 | A2097 | 10 - 20 |
| A3108 | pHG50 | A1645 | 10 - 20 |
| A3109 | pHG50 | A1645 (λi434cl$^-$ mini Tn10) | <1.0 |
| A3112 | pHG51 | A1645 | 10 - 20 |
| A3113 | pHG51 | A1645 (λi434cl$^-$ mini Tn10) | <1.0 |

Strains A3108 and A3112 were infected with λimm434I$^-$ mini Tn10 at 30°C and tetracycline resistant colonies were isolated. The colonies were purified and tested for ampicillin resistance. Single colonies were selected and grown overnight at 30°C in LB medium containing 50 μg/ml ampicillin. The cultures were diluted 1/1000 into LB medium, grown to about 5x10$^8$/ml, and further diluted 1/100,000 into fresh LB and grown overnight. Samples were spread on LB plates and on LB plates containing 50 μg/ml ampicillin. About 50 colonies from each LB plate were checked for growth on LB plates containing ampicillin. The results demonstrate plasmid stability of selected clones.

EXAMPLE 19

Growth of pHG50

I. Stock Cultures

Stock cultures of pHG50 were grown on casein medium (see Inoculum), then diluted two-fold with freezing medium and stored at -80°C. Freezing medium contains per 500 ml:

| K$_2$HPO$_4$ | 6.3 g |
|---|---|
| KH$_2$PO$_4$ | 1.8 g |
| Na Citrate | 0.45 g |
| MgSO$_4$·7H$_2$O | 0.09 g |
| (NH$_4$)$_2$SO$_4$ | 0.9 g |
| Glycerol | 44.0 g |

II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was inoculated with 2-10% inoculum and incubated 15 hours at 30°C, pH 7±0.5 with agitation and aeration to maintain a dissolved oxygen level above 20% air saturation.

III. Production

The production medium contains:

| Casein hydrolysate | 20 g/l |
|---|---|
| Yeast extract | 10 g/l |
| K$_2$HPO$_4$ | 2.5 g/l |
| MgSO$_4$·7H$_2$O | 1 g/l |
| NaCl | 5 g/l |
| Biotin | 0.1 mg/l |
| Thiamine | 1 mg/l |
| Trace elements solution | 3 ml/l |

The medium also contains 100 mg/liter ampicillin. The ampicillin is optional for production but is always found in the medium used for growing the inoculum.

Biotin, thiamine and ampicillin in concentrated solution were filter sterilized separately and added to the sterile production medium before inoculation. Sterile glucose solution was added initially to supply 10 g/l. At the induction step another 10 g/l of glucose was added.

The trace elements solution contains

| FeCl$_3$ | 16 g/l |
|---|---|
| ZnCl$_2$·4H$_2$O | 2 g/l |
| CoCl$_2$·6H$_2$O | 2 g/l |
| Na$_2$MoO$_4$·2H$_2$O | 2 g/l |
| CaCl$_2$·2H$_2$O | 1 g/l |
| CuCl$_2$ | 1 g/l |
| H$_3$BO$_3$ | 0.5 g/l |
| Conc. HCl | 100 ml/l |

The medium is inoculated with 0.5 - 10% inoculum culture and incubated at 30°C. Agitation-aeration rates are set to maintain a dissolved oxygen level above 20% air saturation. The pH is maintained at 7±0.2 with NH$_3$. Once cell concentration reaches about 3.5 g/l (OD$_{660}$ = 10) induction is started.

The temperature is raised to 42°C and maintained at 42°C for 1-5 hours. The culture is then chilled and cells are recovered by centrifugation for hormone purification.

EXAMPLE 20

p8300-10A

45

The construction of p8300-10A (ATCC No. 39785) is shown in Fig. 7 and is described in the Description of the Figures. The plasmid p8300-10A was derived from the constitutive high copy number plasmid pOPIΔ6 (Gelfand, D.H., et al., PNAS (1978) 75, 5869; Meusing, et al., Cell (1981) 24, 235-242). p7200-22, also shown in Fig. 7, was digested with ClaI and the ClaI-ClaI fragment, which contains the λP$_L$ promoter, the bGH gene and the T$_1$T$_2$ sequences was isolated. The ClaI-ClaI fragment was inserted into the unique ClaI site of pOPIΔ6. (The plasmid p7200-22 is a derivative of pSAL 5600-1 (Fig. 10) in which a synthetic ClaI linker was introduced at the BglII site "upstream" of the λP$_L$ promoter.)

Plasmid p8300-10A was found to maintain the constitutive high copy number phenotype even after induction of the λP$_L$ promoter at about 42°C. This may be due to the presence of the T$_1$T$_2$ termination sequences at the 3' end of the bGH sequence which prevents formation of long mRNA transcripts from the λP$_L$ promoter which might interfere with other mRNA transcripts at the origin of replication of pOPIΔ6.

p8300-10A was introduced into Escherichia coli strain A2097 by transformation using methods known to those of ordinary skill in the art. This strain produces upon growth and induction an analog of bovine growth hormone having a methionine residue added to the amino terminus of the phenylalanine form of natural bGH. The amount of bGH analog produced was about 37-43% of the total protein produced by the bacteria as calculated by scanning Coomasie-stained SDS polyacrylamide gels. The methods used to grow the strain, recover the bGH analog produced and purify the bGH analog, are the same as those described for pSAL-170/10 in Example 24.

EXAMPLE 21

A general utility expression vector may be derived from the plasmid p8300-10A (Fig. 7, ATCC No. 39785) by excision of the bGH gene. A vector so derived has numerous advantages over previously described expression vectors including:

1. extremely high levels of expression

The vector is capable of directing expression of foreign proteins in Escherichia coli at levels as high as 44% of the total cellular protein.

2. constitutive high copy number

The vector maintains a constitutively high copy number of about 200-300 copies per cell. This is in distinction to other λP$_L$ expression vectors which are present in lower copy numbers. The high copy number contributes to higher levels of expression.

3. transcription termination signals

The vector which may be obtained by excision of bGH sequence from p8300-10A contains the T$_1$T$_2$ transcription termination signals placed "downstream" from the λP$_L$ promoter and C$_{II}$ ribosomal binding site. The high levels of expression are due in part to the presence of the T$_1$T$_2$ transcription terminators at the end of the inserted gene, as the T$_1$T$_2$ signal terminates transcription of N-modified RNA polymerase. Thus the transcription terminators prevent the λP$_L$-controlled transcription of undesired plasmid proteins, thereby enhancing the relative yields of the desired protein. Furthermore the presence of the T$_1$T$_2$ transcription termination signals prevents long mRNA transcripts through the plasmid origin of replication. This enhances the stability of the high copy phenotype. Similar high copy number plasmids containing the λP$_L$ promoter but lacking the transcription termination sequences are unstable and tend to lose the high copy number phenotype.

4. replaceable ribosomal binding site

p8300-10A contains a unique EcoRI site which is located "upstream" of the ribosomal binding site, and an NdeI site located "downstream" of the ribosomal binding site. Thus, the ribosomal binding site is bounded by two unique restriction sites. This enables facile excision of the present ribosomal binding site (the λC$_{II}$ ribosomal binding site) and substitution of virtually any other natural or synthetic ribosomal binding site without altering other features of the plasmid. This greatly facilitates optimal expression of desired polypeptides.

## 5. thermoinducible regulation of expression

The λP$_L$ promoter is inactive when the C$_I$ repressor is bound to it. The cI857 repressor is thermosensitive, that is, it binds to the promoter at 30°C but is inactivated at 42°C. Thus, by increasing the temperature of fermentation to 42°C the host bacteria are induced to produce the desired protein.

The advantages of such a system include the following:

(a) A foreign protein which is toxic to Escherichia coli can be produced late in the fermentation process thus avoiding early cell death,

(b) Overproduction of a protein may stabilize the protein and prevent proteolytic degradation. (Cheng, Y.E., et al., Gene (1981) 14, 121). Thus, "instantaneous" overproduction using a tightly regulated promoter such as λP$_L$ may be preferable to continuous low level production.

## 6. simplified induction protocol

Protein production by the plasmids described in this patent application and in copending, coassigned U.S. patent application Serial No. 514,188 is regulated by the thermosensitive cI857 repressor.

The induction protocol required by the plasmids described in the copending, coassigned application involved induction at 42°C followed by growth at 38°C. In contrast, the optimal induction of protein synthesis when using the plasmid p8300-10A or pSAL-130/15 or their plasmid derivatives involved induction at 42°C followed by growth at the same temperature, i.e., 42°C. This eliminates the need to cool the fermentor.

## 7. ribosome binding site and initiation codon

This expression vector contains a strong procaryotic ribosomal binding site (RBS) as well as a translation initiation codon (ATG). Thus, any eucaryotic gene may be cloned without adding the initiation codon. Furthermore, the efficient RBS increases levels of expression. The ribosome binding site is the λ C$_{II}$ ribosomal binding site. The sequence of the ribosomal binding site is:

TAAGGAAGTACTTACAT

ATTCCTTCATGAATGTA

One base pair is different from the ribosomal binding site found in the wild type λ.

## 8. convenient restriction site

The expression vector has a unique NdeI restriction site which contains within the site the ATG initiation codon. This permits proper positioning of the desired gene. The unique NdeI site is found immediately after the ribosomal binding site.

## 9. nut site

N protein, which is provided by the host, binds the Nut site on the expression vector and thereby prevents termination of transcription at the t$_{RI}$ site or premature transcription termination within the cloned gene.

## Strains

Suitable hosts for the described vectors and plasmids are strains of Escherichia coli suitable for transformation, including A1637, A2602, A1563, A1645 (c600 r⁻m⁺ gal⁺ thr⁻ leu⁻ lac⁻ bI (λcI857 ΔHI ΔBamHI N⁺)) and A2097 (A1645 lac Δ$_X$A21 proC::Tn 10).

## EXAMPLE 22

pSAL-130/5

The construction of pSAL-130/5 is shown in Fig. 8 and described in the Description of the Figures. pSAL-130/5 was obtained from p8300-10A (ATCC No. 39785) by replacing the met-phe bGH gene with the met-asp-gln bGH gene. The met-asp-gln bGH gene was obtained from plasmid pHG44 (Fig. 6) (ATCC No. 39806) by NdeI and HindIII digestion.

pSAL-130/5 was introduced into Escherichia coli strain A1645 by transformation using methods known to those of ordinary skill in the art. This strain produces upon growth and induction an analog of bovine growth hormone (bGH) having the amino acid sequence met-asp-gln added to the N-terminus of the phenylalanine form of natural bGH. The amount of bGH analog produced by pSAL-130/5 was about 39 - 44% of the total protein produced by the bacteria as calculated by scanning Coomasie blue-stained SDS polyacrylamide gels (Table I). The methods used to grow the strain, recover the bGH analog produced and purify the bGH analog are the same as those described for pSAL-170/10 in Example 24.

EXAMPLE 23

pSAL-170/10

The construction of pSAL 170/10 is shown in Fig. 8 and described in the Description of the Figures.

pSAL 170/10 was introduced into Escherichia coli strain A1645 by transformation using known methods. This strain produces upon growth and induction an analog of bGH having the amino acid sequence met-asp-gln added to the amino terminus of the phenylalanine form of natural bGH. The amount of the bGH analog produced by pSAL-170/10 was about 40 - 46% of the total protein produced by the bacteria as calculated by scanning the Coomasie-stained SDS polyacrylamide gels. (Table I).

EXAMPLE 24

Growth of pSAL-170/10

I. Stock Cultures

Stock cultures of pSAL-170/10 were grown on casein medium (see Inoculum), then diluted two-fold with freezing medium and stored at -80°C. Freezing medium contains per 500 ml:

| | |
|---|---|
| $K_2HPO_4$ | 6.3 gr |
| $KH_2PO_4$ | 1.8 gr. |
| Na Citrate | 0.45 gr |
| $MgSO_4 \cdot 7H_2O$ | 0.09 gr |
| $(NH_4)_2SO_4$ | 0.9 gr |
| Glycerol | 44.0 gr |

II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C and approximately 200 r.p.m. As needed subsequent stages in inoculum propagation were carried out in stirred aerated fermenters. Sterile medium was inoculated with 2-10% inoculum and incubated 15 hours at 30 C, pH 7± 0.5 with agitation and aeration to maintain a dissolved oxygen level above 20% air saturation.

III. Production

The production medium contains:

| | |
|---|---|
| Casein hydrolysate | 20 g/l |
| Yeast extract | 10 g/l |
| $K_2HPO_4$ | 2.5 g/l |
| $MgSO_4 \cdot 7H_2O$ | 1 g/l |
| NaCl | 5 g/l |
| Biotin | 0.1 mg/l |
| Thiamine | 1 mg/l |
| Trace elements solution | 3 ml/l |

The medium also contains 12.5 mg/liter tetracycline. The tetracycline is optional for production but is always found in the medium used for growing the inoculum.

Biotin, thiamine and antibiotics in concentrated solution were filter sterilized separately and added to the sterile production medium before inoculation. Sterile gulucose solution was added initially to supply 10 g/l. At the induction step another 10 g/l of glucose was added.

The trace elements solution contains

| | |
|---|---|
| $FeCl_3$ | 16 g/l |
| $ZnCl_2 \cdot 4H_2O$ | 2 g/l |
| $CoCl_2 \cdot 6H_2O$ | 2 g/l |
| $Na_2MoO_4 \cdot 2H_2O$ | 2 g/l |
| $CaCl_2 \cdot 2H_2O$ | 1 g/l |
| $CuCl_2$ | 1 g/l |
| $H_3BO_3$ | 0.5 g/l |
| Conc. HCl | 100 ml/l |

The medium is inoculated with 0.5 - 10% inoculum culture and incubated at 30°C. Agitation-aeration rates are set to maintain a dissolved oxygen level above 20% air saturation. The pH is maintained at 7±0.2 with $NH_3$. Once cell concentration reaches about 3.5 g/l ($OD_{660}$ = 10) induction is started.

The temperature is raised to 42°C and maintained at 42°C for 1-5 hours. The culture is then chilled and cells are recovered by centrifugation for hormone purification.

Recovery of bGH

Thirteen kilograms of bacterial cells (wet cake) are resuspended in 5 volumes of a solution containing 50 mM sodium phosphate buffer (pH 7.4), 50 mM EDTA and 100 mM NaCl, using a Polytron (Kinematica) blender, while controlling the blender's speed to minimize foaming. The homogenous suspension is continuously passed through a Dynomill cell disruptor KD5 (Willy A. Bachofen, Basel) at a rate of 80 liter per hour and the homogeneous suspension of disrupted cells clarified by centrifugation in a CEPA 101 centrifuge at a flow rate of 45 liter per hour. The precipitate from the centrifugation step is collected and resuspended in 15.5 liters of 50 mM sodium phosphate buffer (pH 7.4) containing 50 mM EDTA. Lysozyme is added to a final concentration of 0.05 mg/ml and the suspension incubated for 16 hours at 37°C. Triton X-100 is added to a final concentration of 1%. The suspension is then incubated for 30 minutes at room temperature, sonicated in a continuous flow cell sonificator (Heat System) at a rate of 18 liters per hour and centrifuged in a CEPA 101 centrifuge. The precipitate is collected, resuspended in 50 mM sodium phosphate buffer (pH 7.4), sonicated as above, and centrifuged in a CEPA 101 centrifuge. The cells are resuspended in 15.5 liters of 50 mM sodium phosphate buffer (pH 7.4) containing 50 mM EDTA and 100 mM NaCl and twice precipitated and resuspended in 15.5 liters of distilled water. The precipitate is collected by centrifugation and stored at -20°C.

Purification Of bGH

The precipitate is resuspended in 30 - 40 liters distilled water and solubilized by titration with 0.5 N NaOH to pH 11.8. The solution is then continuously sonicated and clarified by centrifugation in CEPA 101 centrifuge if necessary, or filtered through Whatman No. 1 paper.

The clarified protein solution (32.6 liters containing 297,000 OD's at 280 nm) is divided into separate portions (6 X 5.4 liters) each containing 50,000 - 60,000 OD's. Each portion is ultrafiltered separately through a Millipore Pellicon ultrafilter equipped with three 100,000 molecular weight cutoff cassettes (type PTHK) of 5 ft$^2$ area each. A 5.4 liter portion is concentrated to 1 liter retentate volume. The ultrafiltrate is collected and saved. The retentate is diluted back to its original volume with fresh 10 mM Borate buffer, pH 11.8, and mixed well. The batch is concentrated again to 1 liter retentate volume. The ultrafiltrate is collected and combined with the first ultrafiltrate. When the running total of the OD's in the ultrafiltrates equals 20% of the OD's initially charged to the ultrafilter, the retentate volume on the next concentration step is taken to 0.5 liters instead of 1 liter. The cycle of concentration and dilution with 10 mM Borate buffer is continued until the ultrafiltrate from a retentate volume of 0.5 liters has an absorbance at 280 nm (l-cm cell) of less than 0.1. This normally takes between 9 and 12 cycles of concentration and dilution. The final retentate is discarded.

All ultrafiltrates are combined and adjusted to pH 9.0 with 6N HCl. The other 5.4-liter portions are ultrafiltered in the same fashion, and all pH adjusted ultrafiltrates are combined. A typical run produces a total of 380 liters of ultrafiltrates with an absorbance of 0.26 equivalent to 100,000 OD's and requires 24 to 40 hours to complete.

The combined ultrafiltrates (380 liters containing 100,000 OD's at 280 nm) from the 100K ultrafiltration step are loaded onto a Sepharose CL-6B DEAE ion-exchange column at a linear flow velocity of 23 cm/hr (25 liter/hr). The 37-cm diameter 15-cm high column is washed with two bed volumes (32 L) of 10 mM Borate buffer at pH 9.0. The eluate from the loading and washing steps is discarded. A step change in eluent to 10 mM Borate, 100 mM sodium chloride, pH 9, displaces the bGH off the column. The elution flow velocity is 23 cm/hr. The progress of the run is monitored by following absorbance of the eluate at 280 nm. The bGH peak is collected in 4 to 5 bed volumes (84 liters containing 43,000 OD's at 280 nm) and then concentrated to approximately 10 mg/ml using a Millipore Pellicon ultrafiltration device with a 10,000 molecular weight cutoff cassettes. The solution is then lyophilized. The yield is approximately 70 g of pure bGH.

EXAMPLE 25

Activity Of bGH Analog Produced By pSAL-170/10

1. Radioimmunoassay Comparison of bGH Analog with Natural bGH

A solution containing 100 ng/ml bGH analog was prepared in phosphate buffered saline (1% BSA). This solution was diluted serially to concentrations of 50, 25, 12.5, 6.25, 3.12, 1.56 and 0.78 ng/l. Duplicate 0.1 ml aliquots of these solutions were submitted to RIA using a double antibody procedure. The dilution curve was comparable to that obtained with natural bGH.

2. Radioreceptor Binding Assay

A radioreceptor binding assay was performed with rabbit liver membranes as described by Tushima, T. and Freisen, H.G., (Y. Chin., Endocr. Metab. (1973), 37; 3) using $^{125}$I-bGH as the tracer and authentic bGH solutions for the construction of calibration curves. Samples were incubated in triplicate for two hours at room temperature in 0.3 ml of assay buffer (50 mM Tris, 15 mM CaCl$_2$ and 5 mg/ml bovine serum albumin, pH 7.6). The tubes contained $^{125}$I-bGH (20,000 cpm of preparation of 30 - 60 $\mu$ci/$\mu$g), 150 - 250 $\mu$g liver membrane protein and either natural bGH (1 - 100 ng) or extracts of bacterial bGH. The result demonstrated that the bGH activity of the bGH analog is comparable to that of natural bGH.

3. Tibia Test

The bioactivity of the pRO12 produced bGH analog recovered from bacterial cells according to Example 5 was evaluated by a tibia test. (Parlow, A.F., et al., Endocrinology (1965) 77: 1126). Rats were hypophysectomized at 28 - 30 days of age, then kept for 10 - 14 days without treatment. Bovine growth hormone derived from bovine pituitaries or from recombinant Escherichia coli was dissolved in 0.15 M NaCl + 0.01 M borate, pH 10.0. Rats (4 - 7 per group) received daily subcutaneous injections of bGH solutions (5 - 125 $\mu$g/day in 0.2 cc) for 5 days while kept on a normal diet (Purina Rat-Chow and water adlibitum). The animals were sacrificed on the 6th day, their foreleg knee-bones taken out, cut longitudinally, fixed with acetone and stained with 2% AgNO$_3$. The width of the epiphyseal plates was measured by observation through a dissecting binocular (Nikon). Mean values (40 readings per rat) were used for the constructon of long dose-response curves. The results demonstrated that the bGH activity of the pSAL-170/10-produced bGH analog is comparable to that of natural bGH.

## EXAMPLE 26

The yield and activity of human superoxide dismutase produced by the SOD host-vector systems described in Example 3 may be improved by modifying the growth conditions of the host-vector systems. As the following data demonstrates supplementing the growth medium for the host-vector system with Cu and/or Zn results in a greater yield of the enzyme in active dimer form.

## Growth of Bacteria Containing $pSOD\beta_1 T11$

### I. Stock Cultures

Stock cultures of $pSOD\beta_1 T11$ were grown on casein medium (see inoculum), then diluted twofold with freezing medium and stored at -80°C. Freezing medium contains:

| | |
|---|---|
| $K_2HPO_4$ | 6.3 gr |
| $KH_2PO_4$ | 1.8 gr |
| Na Citrate | 0.45 gr |
| $MgSO_4 \cdot 7H_2O$ | 0.09 gr |
| $(NH_4)_2SO_4$ | 0.9 gr |
| Glycerol Per 500 ml | 44 gr |

### II. Inoculum

The inoculum was propagated in 20 g/l casein hydrolysate, 10 g/l yeast extract and 2 g/l NaCl. Sterile medium in a shake flask was inoculated from stock culture and incubated 15 hours on a shaker at 30°C, and approximately 200 r.p.m. If needed subsequent stages in inoculum propagation were carried out in stirred aerated fermenters. Sterile medium was inoculated with 2-10% flask culture, and incubated 15 hours at 30°C, pH 7 ± 0.5 with agitation and aeration to maintain dissolved oxygen level above 20% air saturation.

### III. Production

The production medium contains:

| | |
|---|---|
| Casein hydrolysate | 20 g/l |
| Yeast extract | 10 g/l |
| $K_2HPO_4$ | 2.5 g/l |
| $MgSO_4 \cdot 7H_2O$ | 1 g/l |
| NaCl | 5 g/l |
| Biotin | 0.1 mg/l |
| Thiamine | 1 mg/l |
| Trace elements solution | 3 ml/l |
| Tetracycline | 12.5 mg/l |

In some of the experiments we added:

| | |
|---|---|
| $CuSO_4 \cdot 5H_2O$ | 0.8 g/l |
| $ZnSO_4 \cdot 7H_2O$ | 10 mg/l |

Biotin, thiamine, and tetracycline in concentrated solutions were filter sterilized separately and added to the sterile production medium before inoculation. Sterile glucose solution was added initially to supply 10 g/l. At the induction step another 10 g/l of glucose was added.

The trace elements solution contains:

| FeCl$_3$ | 16 g/l |
| ZnCl$_2 \cdot 4H_2O$ | 2 g/l |
| CoCl$_2 \cdot 6H_2O$ | 2 g/l |
| Na$_2$MoO$_4 \cdot 2H_2O$ | 2 g/l |
| CaCl$_2 \cdot 2H_2O$ | 1 g/l |
| CuCl$_2$ | 1 g/l |
| H$_3$BO$_3$ | 0.5 g/l |
| Conc. HCl | 100 ml/l |

The medium is inoculated with 0.3 - 10% inoculum culture and incubated at 30°C. Agitation-aeration rates are set to maintain dissolved oxygen level above 20% air saturation. The pH is maintained at 7 ± 0.2 with NH$_3$. Once cell concentration reaches about 3.5 g/l (OD$_{660}$ = 10) induction is started.

The temperature is raised to 42°C and maintained at 42°C for 1-5 hours. The culture is then chilled, and cells are recovered by centrifugation for enzyme purification.

RECOVERY OF SOD

One and one-half kilograms of bacterial cells (wet cake) are suspended in 12 liters of 50 mM sodium phosphate (pH 7.8), in a Polytron (Kinematica) blender while controlling the speed to minimize foaming. The homogeneous suspension is continuously passed through a Dynomill cell disrupter KD5 (Willy, A. Bachofen, Basel). The homogeneous suspension of disrupted cells is sonicated using a continuous flow cell and centrifuged in a CEPA 101 centrifuge. The supernatant is heated for 2 hours at 65°C, cooled and centrifuged as before. The clear supernatant is concentrated to 1 liter in a Millipore Pellicon ultrafiltration device using 10,000 molecular weight cutoff casettes (type PTGC). The concentrated protein solution is passed through a DEAE-Sephacel column (2 Kg DEAE Sephacel) equilibrated with 150 mM sodium phosphate buffer (pH 7.8). The flow through solution is collected, concentrated and dialyzed in a Pellicon ultrafiltration device against 20 mM Tris-HCL, pH, 7.8 and then applied on to a QAE-Sepharose column equilibrated with 20 mM Tris-HCl buffer. The column is developed with a 20 mM Tris-HCl buffer, pH 7.8, and a salt gradient (0 - 200 mM NaCl). SOD containing fractions are collected, concentrated using a Pellicon ultrafiltration device, dialyzed against distilled water and then brought to 100 mM sodium acetate by adding 1M sodium acetate buffer, pH 4.8. The protein solution is then further separated on a CM-Sepharose column equilibrated with 100 mM sodium acetate buffer, pH 4.7. The column is developed using the same buffer and a salt gradient (100-500 mM NaCl). SOD containing fractions are collected, concentrated using a Pellicon ultrafiltration device and lyophilized.

EXAMPLE 27

Enzymatic Activity of SOD Produced by Bacteria

The purified human SOD (hSOD) produced by bacteria in Example 26 under standard growth conditions (that is without adding extra CuSO$_4$) possessed only 5% of enzymatic activity as compared to bovine Cu/Zn SOD. Analysis of the metal content reveals that the enzyme contains little Cu, and that is only 8% of the expected value. Furthermore, it seems that most of the Cu$^{++}$ sites are replaced by Zn ions since the protein contains almost twice the Zn required. However, completely metal free apoprotein, prepared from the bacterially produced hSOD, regains essentially full enzymatic activity upon reconstitution in solution. The solution contains both Cu$^{++}$ and Zn$^{++}$, each at a concentration of 1.2 mole ions per mole active site. (Table IV).

The data presented above suggested that the intracellular concentration of Cu$^{++}$ is limited and insufficient to saturate the hSOD produced. In a series of experiments we have demonstrated that elevated Cu$^{++}$ concentrations in the growth medium caused increase in the specific activity of hSOD. Indeed, E. coli grown in casein hydrolysate (Example 26) supplemented with 200 ppm Cu$^{++}$, produced, after induction, fully active hSOD with the natural composition of metals, and full enzymatic activity (Table IV). Additional experiments have demonstrated the same effect when the amount of exogenous Cu$^{++}$ added ranged from 50-250 ppm. We have seen a similar effect with LB (Luria Broth) medium supplemented with 75 ppm Cu$^{++}$.

52

TABLE IV

| Activity and Metal Content of SOD Preparations | | | |
|---|---|---|---|
| Protein | Mole/Subunit | | Activity |
| | Cu | Zn | u/mg |
| hSOD[1] | 0.07 | 1.62 | 167 |
| Apo enzyme | 0.01 | 0.02 | 0 |
| Reconstituted SOD | 0.81 | 0.88 | 2931 |
| hSOD[2] | 0.88 | 0.90 | 2730 |
| Bovine SOD | 0.97 | 1.01 | 2805 |
| hSOD (Sigma) | | | 1606 |

[1]hSOD prepared as described in Example 26. The medium used did not contain the added $Cu^{++}$ and $Zn^{++}$.
[2]hSOD prepared as described in Example 26. The medium used did contain the added $Cu^{++}$ and $Zn^{++}$.

SOD concentrations were determined by the method of Lowry (Lowry, O.H., Rosebrough, N.J., Farr, A.L. and Randall, R.J., J. Biol. Chem. 193, 265-275 (1951)) using boving serum albumin as standard. Activity measurement monitoring the inhibition of reduction of ferricytochrome-c were carried out as described by McCord and Fridovich (McCord, J.M. and Fridovich, I., J. Biol. Chem. 244, 6049-6055 (1969)). Cu and Zn content in pure SOD preparations was determined by atomic absorption. SOD-apo enzyme was prepared according to Weser and Hartmann (Weser, U. and Hartmann, H.J., FEBS Lett. 17, 78-80 (1971)) and reconstituted by simultaneous addition of Cu and Zn (Jewett, S.L., Latrenta, G.S. and Beck, C.M., Arc. Biochem. Biophys. 215, 116-128 (1982)).

EXAMPLE 28

Amino Terminal Sequence of Bacterial Produced hSOD

The sequence of 5 amino acids at the amino terminus of purified SOD prepared as described in Example 26 was determined by Edmann degradation. The amino acid sequence is identical to the N-terminus of human Cu/Zn SOD, that is Ala-Thr-Lys-Ala-Val. This confirms the authenticity of the bacterial product. It seems therefore that the soluble hSOD is accessible to E. coli processing enzymes which remove the N-terminal methionine.

Discussion

We have demonstrated that bacteria which are induced to produce large amounts of hSOD when grown on LB or casein hydrolysate media, produce an enzymatically inactive protein. The protein so produced can be activated by reconstituting and adding back the missing $Cu^{++}$, $Zn^{++}$ ions. Unexpectedly, the bacteria can be induced to produce enzymatically active hSOD by growing on media which has been supplemented with $Cu^{++}$. While not wishing to be bound by theory, we believe that certain components of rich media (such as LB and casein hydrolysate), chelate most of the available copper, such that the bacteria do not have available enough free copper to fill all the sites in the SOD molecules, which are being produced at elevated levels. The addition of 50-250 ppm of $Cu^{++}$ ions to the media apparently raises the $Cu^{++}$ concentration within the bacteria to levels which are sufficient to provide all the $Cu^{++}$ necessary for the overproduced human Cu/Zn SOD.

EXAMPLE 29

Reduction in Reperfusion Injury with Recombinant Human Superoxide Dismutase Following Global Ischemia

Human superoxide dismutase produced by the host-vector system pSOD$\beta_1$T11 in E. coli A1645 described in Example 3, grown and purified under the conditions described in Example 26 has been shown to reduce reperfusion injury following global ischemia.

53

### Isolated Perfused Rabbit Heart Preparation

Female New Zealand white rabbits, 1.2-2.0 kg were heparinized and anesthetized, their hearts removed and quickly placed into cold (4°C) perfusate. The ascending aorta was cannulated and the hearts perfused under constant pressure (110 cm of water) with a modified Krebs-Ringers bicarbonate buffer solution containing 117 mM sodium chloride, 6 mM potassium chloride, 3.0 mM calcium chloride, 1.0 mM magnesium sulphate, 0.5 mM EDTA, and 16.7 mM glucose with the final pH adjusted to 7.40 by the addition of approximately 24 mM sodium bicarbonate. The perfusate was bubbled continuously with 95% oxygen and 5% carbon dioxide. Coronary flow was removed from the NMR sample tube by vacuum aspiration. The hearts were paced at 175 beats/minute by right ventricular pacing with a wick soaked in saturated potassium chloride, encased in polyethylene tubing, and connected to a Grass SD-9 stimulator. To quantitate left ventricular contractile function a latex rubber balloon was tied to the end of a 100 cm length of PE 190 tubing, carefully purged of air bubbles and connected via a three-way stopcock to a Statham P23Db transducer. Isovolumic pressure was recorded with a Brush two channel direct writing recorder. The balloon was initially inflated via a syringe with a volume of saline sufficient to produce an end diastolic pressure of 10 mmHg. All subsequent measurements of developed pressure were at this end diastolic volume. All hearts were subjected to 30 minutes of global ischemia during which time the hearts were maintained at 37°C by a flow of warm perfusate around the heart. Total interruption of aortic in-flow was accomplished by cross clamping the perfusion line. Forty-five minutes of normothermic reperfusion (37±2°C) followed the period of ischemia. Recovery of left ventricular developed pressure was calculated as a percentage of the pre-ischemic control. At the onset of ischemia the balloon was deflated and the pacer turned off. The balloon was reinflated 15 minutes after initiating reflow, just prior to the first measurement of function with the same volume removed at the onset of ischemia. Coronary blood flow was measured volumetrically by vacuum aspiration prior to ischemia, 5 minutes after reflow and after 15, 30 and 45 minutes of reperfusion.

### Nuclear Magnetic Resonance Methods

Phosphorous-31 NMR spectra were obtained in a Brucker WH 180 spectrometer at 4.23 Telsa in a wide bore superconducting magnet. At this field strength, phosphorus resonates at 72.89 MHz. The diameter of the phosphorus probe is 25 mm. This instrument was operated in the pulsed Fourier transform mode and interfaced to a Nocolet 1280 computer and the data collected on high density magnetic disks. Because of the field stability of the superconducting magnet, field/frequency lock is not required. Five minute proton decoupled spectra were collected from transients following 45° pulses delivered at two second intervals, conditions previously documented to result in minimal spectral saturation. The data were accumulated with a 2K table at a 3,000 Hz spectral width.

### Estimation of Tissue Intracellular pH from NMR Spectra

Measurement of intracellular pH was determined from the chemical shift ($\delta_o$) of the inorganic phosphate peak by the following equation:

$$pH_l = pK - \log \frac{\delta_O - \delta_B}{\delta_A - \delta_O}$$

In order to minimize tissue inhomogeneity effects, chemical shift values were measured relative to the resonance of phosphocreatine which is relatively pH independent over the range of pH to be encountered in these studies ($pK_A = 4.6$). The constants used in this equation are $pK = 6.90$, $\delta_A = 3.290$ PPM and $\delta_B = 5.805$ PPM, as previously reported.

### Quantitation of Metabolites from NMR Spectra

Estimations of tissue phosphocreatine (PCr), adenosine triphosphate (ATP), as well as inorganic phosphate (Pi) were obtained by planimetric measurements of the areas under the individual peaks allowing for the computer determined normalization constant or scaling factor. A Hewlett-Packard digitizer was used to perform the area integrations. Quantitative data thus derived for PCr, ATP and Pi are expressed as percent of the pre-ischemia control content.

Experimental Protocol

Seventeen hearts were divided into two groups:

Group I    (n = 8) The hearts in this group were treated with 60,000 units of human recombinant superoxide dismutase (hSOD, specific activity 3,200 IU/mg) administered as a 10 ml bolus just prior to reflow, followed by a continuous infusion of 60,000 units during the first 15 minutes of reflow; hSOD was dissolved in warm 37°C Krebs-Ringers bicarbonate perfusate.

Group II   (n = 9) The hearts in this group received a 10 ml bolus of perfusate just prior to reflow followed by normothermic reperfusion.

Results

Recovery of Left Ventricular Function

The experimental ischemic model employed in the present study was specifically chosen following a series of preliminary studies to provide hearts which having suffered a moderately severe irreversible insult, still retained the potential for improvement with a therapeutic intervention. At the end of 45 minutes of reflow, the recovery of left ventricular function (as measured by percent recovery of control developed pressure) was 47±5% for the control group with an end diastolic pressure to 48±7 mmHg (compared to a pre-ischemic control value of 10mmHg). These parameters did not change appreciably between 30 and 45 minutes of reperfusion, suggesting that a relatively steady state of recovery had been achieved. The administration of hSOD just prior to reflow and for the initial 15 minutes of reperfusion resulted in significantly improved preservation of cardiac function and in a smaller increase in end diastolic pressure compared to control hearts; hSOD treated hearts recovered 71±6% of control developed pressure at an end diastolic pressure of only 27±4 mmHg (both P<.01 vs control).

Myocardial Metabolism During Ischemia and Following Reperfusion

Myocardial high-energy phosphate contents were serially measured during ischemia and following reperfusion in both control and hSOD-treated hearts. During the 30-minute ischemic period progressive decreases in myocardial creatine phosphate and ATP content were observed. Phosphocreatine content fell by the end of the ischemic period to 8±3% of the pre-ischemic baseline value in control hearts and to 10±5% of control in those hearts which were subsequently to be treated with hSOD; ATP content reached 36±6% of the baseline value in control hearts and 33±6% in hSOD treated hearts. These data clearly indicate that both groups of hearts were subjected to an equally severe degree of ischemia. These data also rule out the possibility the hearts receiving hSOD demonstrated better functional recovery due to better preservation of cellular metabolism during the ischemic period by some uncontrolled for mechanism.

At the end of 45 minute reflow period hSOD-treated hearts displayed a nearly normal content of phosphocreatine (93±9% of control) whereas control hearts recovered only 69±7% of the original value (P<.05). At the end of the reflow period ATP content was equal in both groups of hearts (41±4% in control vs 42±5% in hSOD-treated hearts). This latter result might reflect increased energy demands of hearts recovering better left ventricular function, in the presence of a limited ability to increase rates of high energy phosphate production. In contrast, poorer recovery of function in control hearts would result in less utilization of high energy phosphate metabolites, possibly masking even more severe limitation in energy production.

In conclusion, these data demonstrate, in hearts subjected to a moderately severe ischemic insult and subsequent reperfusion, the administration of hSOD just prior to and during early reperfusion results in better recovery of systolic and diastolic function, as well as in higher myocardial content of phosphocreatine. These data also suggest that reperfusion of ischemic myocardium may result in a component of structural and/or functional damage which can be avoided or reduced by the administration of an oxygen free radical scavenger such as hSOD at the time of reperfusion. Thus, by limiting that component of reflow injury resulting from reoxygenation of previously ischemic myocardium, hSOD may provide a valuable addition to thrombolytic therapy and/or coronary angioplasty in patients treated early after acute myocardial infarction.

EXAMPLE 30

Reduction in Experimental Infarct Size by Recombinant Human Superoxide Dismutase Administration During Reperfusion

Human superoxide dismutase produced by the host vector system pSOD$\beta_1$T11 in E. coli A1645 described in Example 3, grown and purified under the conditions described in Example 26 has been shown to reduce infarct size in hearts.

Timely reperfusion of the ischemic myocardium reduces infarct size (IS); however, this beneficial effect may be blunted by the simultaneous occurrence of reflow injury, mediated through the generation of toxic oxygen free radicals. To test whether scavenging of free radicals by recombinant human superoxide dismutase (hSOD) could result in a reduction of IS compared to reperfusion alone, in 16 anesthetized dogs the circumflex coronary artery was occluded before any marginal branch for 90 min; at the time of reperfusion the animals were injected with either hSOD (400,000 units as a bolus into the left atrium, followed by 300,000 units as a 1 hr i.v. infusion; n = 8), or with a similar amount of saline (controls, n = 8). The chest was then closed and the animals were allowed to recover. After 48 hrs the dogs were sacrificed and the hearts processed in a blinded fashion for the evaluation of IS by gross pathology and of the risk area by postmortem angiography. Proximal occlusion of the circumflex artery resulted in ischemia of 40.8 + 2.3% of the left ventricle (LV) in controls and 41.8 + 2.0 in treated dogs. In control dogs reperfusion was associated with infarction of 52.2 + 7% of risk area; hSOD treatment, however, resulted in a significant reduction of necrosis, IS being 33.6 + 2.1% of risk area (p < .05). Control animals developed confluent, non-transmural infarcts which extended throughout most of the risk area, whereas in treated dogs the infarcts appeared more patchy and non-confluent. In conclusion, free radical scavenging by hSOD administered at the time of reperfusion significantly reduced the extent of necrosis, possibly through a prevention of reflow injury.

EXAMPLE 31

The Role of Oxygen Free Radicals in Mediating the Reperfusion Injury of Cold Preserved Ischemic Kidneys[1]

In a new indication, human superoxide dismutase can reduce reperfusion injury following transplantation of organs. The following Example demonstrates that superoxide dismutase ameliorates injury on reperfusion following the transplantation of a kidney. The human superoxide dismutase utilized in this example was produced by the host-vector system pSOD$\beta_1$T11 is E. coli A1645 described in Example 3, and grown and purified under the conditions described in Example 26.

The parenthesized arabic numbers found throughout this Example refer to the articles listed in the Bibliography at the end of this Example.

Model of Renal Preservation and Transplantation Ischemia in Swine

Female, outbred pigs, weighing 15 to 18 kg were premedicated with acepromazine and atropine, and anesthetized with ketamine and halothane. In the donor pigs, diuresis was established 30 minutes prior to harvest by the intravenous administration of 1500 cc of Ringer's lactate, furosemide (20 mg) and mannitol (12.5 g). Phenoxybenzamine (50 mg) was given intravenously to prevent renal vasopasm, which can be seen frequently in pigs. Through a midline abdominal incision, the distal aorta and vena cava were mobilized, just proximal to their bifurcations. The ureters were dissected and divided at the level of the bladder. An inflow catheter was placed in the aorta, just above the bifurcation, and an outflow catheter was placed into the inferior vena cava. Heparin (5,000 units) was given intravenously and a continuous flush with Euro-Collins solution at 4°C was initiated through the distal aorta, coincident with the cross-clamping of the aorta just above the renal artery. After the kidneys had been cooled in situ, they were removed en block. The kidneys were then separated and one was assigned to be the control and the other to be the test kidney, thus facilitating a paired design. Each kidney was then flushed again with 4°C Euro-Collins solution. When called for by the protocol, test substances were added to the preservation fluid of the second kidney at this time. Both kidneys were packaged sterilely and stored at 4°C overnight.

After 24 hrs of cold ischemia, a fresh recipient animal was anesthetized and the preserved kidneys were transplanted to its iliac vessels. The time required for each anastomosis was 25 to 30 min. the control

[1]Abbreviations used in this example: $C_{CR}$, Crestinine clearance; ATP, adenosine triphosphate; ADP, adenosine diphosphate; AMP, adenosine monophosphate.

(untreated) kidney was always transplanted first, the test kidney second. Reperfusion of the test kidney was therefore always delayed for a total of one hour after the reperfusion of the control kidney. This means that the control kidneys were subjected to 23 hours of cold ischemia, the test kidneys to 24 hours. The SOD analog was administered intraarterially beginning one hour after reperfusion of the control kidney at the time of reperfusion of the test kidney. Thus, the control kidney was exposed to the toxic effects of rewarming and reperfusion for one hour before exposure to any possible effects of agents provided to the test kidney. Following reperfusion of the second kidney, the native kidneys of the recipient pig were removed. Additional doses (10 g) of mannitol were given prior to reperfusion of first the control, and then again of the test kidney, to mimic clinical practice. This allowed evaluation of the effect of free radical modifying agents superimposed upon optimal conventional preservation/transplantation techniques. The ureter from each kidney was brought out separately as a cutaneous ureterostomy.

Two days following transplantation, the recipient was lightly anesthetized and urine was collected for one hour from each kidney (ureterostomy) separately and assayed for volume and creatinine concentration. Serum creatinine was also determined, allowing the calculation of creatinine clearance separately for each kidney.

All results are expressed as mean ± SEM. Data were analyzed during the Student t-test (two-tailed). In most cases a paired test could be applied due to the paired design of the experiments.

Experimental Protocol

Superoxide Dismutase (SOD) and Catalase

Sigma bovine blood superoxide dismutase, a scavenger of oxygen free radicals, was administered to test kidneys in four pigs. A 5 mg bolus was given into the renal artery immediately prior to reperfusion, and a constant intraarterial infusion was maintained at 1 mg/min during the first 15 min. of reperfusion. This provided a total dose of 20 mg of SOD. In a second group of four pigs, the test kidneys received catalase (Sigma Co.), by the same dosage regimen, in addition to SOD. The other kidney in each of these pigs received no treatment, and thus served as a control.

Dose Response to SOD

In order to determine the minimal dosage for maximal protection, a dose response relationship was studied. At revascularization, one kidney received an infusion of a lesser of two doses of SOD and the other the next higher dose. Human recombinant SOD prepared as described in Example 9 was given as described above. Two comparisons were made at each dosage range. In the first, 0.2 mg of SOD was compared with saline solution as the control. Stepwise, 0.2 mg was compared to 2 mg, 2 mg was compared to 20 mg, and finally 20 mg to 100 mg. In each case, the kidney to receive the lesser dose was transplanted first, in order to avoid the possible problem of SOD retention in the circulation at the time of the second transplant.

Results

The Effect of Bovine SOD and Catalase

The creatinine clearance for a normal single kidney in pigs of the size we used, under the above conditions of anesthesia and hydration, was 25.5 + 6.3 ml/min (n = 8). The administration of SOD in a dose of 20 mg into the renal artery for the first 15 min of reperfusion substantially ameliorated the renal functional impairment after cold ischemia. The four kidneys treated with SOD alone had a mean creatinine clearance of 23.2 ± 4.5 ml/min), almost three times that of the control kidneys (8.4 ± 1.7 ml/min, p<0.05). A combination of SOD and catalase provided similar, but not greater, protection ($C_{CR}$ = 19.0 ± 4.5 ml/min). In early pilot experiments, a separate group of four pigs had undergone transplantation with anastomotic times in excess of 40 minutes. Although renal function was significantly increased by SOD and catalase, in these animals, both treated and control kidneys had very poor function ($C_{CR}$ = 4.8±0.8 vs. 1.6±0.4 ml/min). In these kidneys, presumably subjected to more severe injury due to ischemia per se prior to reperfusion, modification of free radical injury was unable to restore normal renal function.

Human SOD Analog Dose Reponse Relationships

Infusions of human SOD analog (0.2 mg and 2 mg) provided no improvement in renal function compared to the controls. However, the mean creatinine clearance of kidneys receiving 20 mg of SOD analog was 14.2 + 1.1 ml/min, significantly better than in the pairs receiving 2 mg infusions (7.7 ± 1.0 ml/min p<0.05). No further benefit was obtained from 100 mg of SOD analog ($C_{CR}$ = 16.1 ± 1.2 ml/min). Therefore, the minimal effective dose of SOD analog proved to be greater than 2 and less than 20 mg when administered in this manner. The human SOD analog was as effective as the bovine SOD.

Discussion

The paired design was employed to maximize differences due to the treatment regimens employed, and to control for confounding variables which were related to the particular donor or recipient animal. It also provided for a paired statistical analysis of results, which allowed optimal use of small numbers of relatively expensive experimental animals.

The striking finding of these studies was the magnitude of the benefit provided by ablation of free-radical mediated reperfusion injury. Despite the fact that the control kidneys received the benefits of optimal conventional methods for organ preservation, including healthy donor kidneys and recipient animals, hydration, alpha adrenergic blockage, anticoagulation, and diuresis, they demonstrated a severe functional lesion, with creatinine clearance levels depressed to values less than a third of normal. This 24 hour period of ischemic preservation, as it often does in similar clinical circumstances, exceeds conventional organ preservation capabilities. Treatment with effective doses of bovine SOD or human SOD analog dramatically prevented this injury, preserving renal function at near normal levels. Indeed, there was not a single kidney so treated that did not have a creatinine clearance at least twice that of its paired, untreated control, when measured 48 hours after transplantation. The magnitude of this benefit was therefore quantitatively greater than that seen by others following 45-60 minutes of warm ischemia. This suggests that with optimal conventional preservation techniques, the injury due to ischemia per se has been minimized, allowing the injury produced at reperfusion to become predominant. This interpretation is further supported by the studies with kidneys preserved 18 hours prior to reperfusion. In these kidneys, function was excellent in both the control and the treatment groups, suggesting that sufficient time had not elapsed to allow the accumulation of enough matabolites to set up the conditions favoring free radical generation at reperfusion. On the other hand, when the kidneys were subjected to a more severe degree of ischemia per se, as in the early pilot experiments with prolonged anastomosis (i.e., warm ischemia) times, SOD was not able to restore function to near normal levels, although a significant improvement was still seen. These kidneys would therefore be more analogous to those studied following shorter periods of warm ischemia (7,8,9). As in other organs, the benefits of obviating free radical injury are primarily related to the relative proportions of the injury that are due to ischemia itself, compared to that due to reperfusion.

Furthermore, the achievement of this increment of benefit appears to be obtained either wholly, or not at all. The dose response studies with SOD showed either maximal protection, or no protection whatsoever. Catalase provided no additional benefit when added to SOD alone. Within the quantitative limits of resolution of this study, the prevention of reperfusion injury appears to have been an all-or-nothing phenomenon.

The findings of this study, however, appear to be particularly relevant to clinical application. The 24 hour period of cold ischemia chosen corresponds well with the periods of cadaveric graft preservation necessitated by clinical circumstances. Furthermore, these studies evaluated the efficacy of free radical reperfusion injury ablation in the face of optimal conventional preservation and transplantation methods. This includes the use of mannitol, itself a potent hydroxyl radical scavenger. These studies therefore suggest that a similar degree of benefit might well be obtained by superimposing free radical ablation on current clinical practices. As SOD is a nontoxic compound, this approach seems promising.

BIBLIOGRAPHY

1. Parks D.A., Bulkley G.B., Granger D.N., Hamilton S.R., McCord J.M. Ischemic Injury to the Cat Small Intestine: Role of Superoxide Radicals. Gastroenterology 82:9 (1982).
2. Manson P.N., Anthenelli R.M., Im M.J., Bulkley G.B., Hoopes J.E. The Role of Oxygen-Free Radicals in Ischemic Tissue in Island Skin Flaps. Ann Surg 198:87 (1983).
3. Shlafter M., Kane P.F., Kirsh M.M. Superoxide Dismutase Pluse Catalase Enhance the Efficacy of Hypothermic Cardioplegia to Protect the Globally Ischemic, Reperfused Heart. J Torac Cardiovasc Surg 83:830 (1982).
4. Stuart R.S., Baumgartner W.A., Borkon A.M., et al. Five-Hour Hypothermic Lung Preservation with Oxygen Free-Radical Scavengers. Transplant Proc 17:1454 (1985).

5. Sanfey H., Bulkley G.B., Cameron J.L. The Role of Oxygen Derived Free Radicals in the Pathogenesis of Acute Pancreatitis. Ann Surg 200:405 (1984).

6. Hansson R., Gustavsson B., Jonsson O., et al. Effect of Xanthine Oxidase Inhibition on Renal Circulation After Ischemia. Transplant Proc 14:51 (1982).

7. Ouriel K., Smedira N.G., Ricotta J.J. Protection of the Kidney After Temporary Ischemia: Free Radical Scavengers. J Vasc Surg 2:49 (1985).

8. Peller M.S., Hoidal Jr, Ferris T.F. Oxygen Free Radicals in Ischemic Acute Renal Failure in the Rat. J Clin Invest 74:1156 (1984).

9. Im M.J., Shen W.H., Pak C.I., Manson P.N., Bulkley G.B., Hoopes J.E. Effect of Allopurinol on the Survival of Hyperemic Island Skin Flaps. Plast Reconstr Surg 73:276 (1984).

10. Parks D.A., Bulkley G.B., Granger D.N. Role of Oxygen Free Radicals in Shock, Ischemia, and Organ Preservation. Surgery 94:428 (1983).

11. Toledo-pareyra L.H., Simmons R.L., Najarian J.S. Effect of Allopurinol on the Preservation of Ischemic Kidneys Perfused with Plasma or Plasma Substitutes. Ann Surg 180:780 (1974).

12. Vasco K.A., DeWall R.A., Riley A.M. Effect of Allopurinol in Renal Ischemia. Surgery 71:787 (1972).

13. Owens M.L., Lazarus H.M., Wolcott M.W., Maxwell J.G., Taylor J.B. Allopurinol nd Hypoxanthine Pretreatment of Canine Kidney Donors. Transplantation 17:424 (1974).

14. Granger D.N., Rutilli G., McCord J. Superoxide Radicals in Feline Intestinal Ischemia. Gastroenterology 81:22 (1981).

15. Roy R.S., McCord J.M. Superoxide and Ischemia: Conversion of Xanthine Dehydrogenease to Xanthine Oxidase. In: Greenwald R., Cohen G., eds. Oxyradical and Their Scavenger Systems (Vol. 2). Cellular and Molecular Aspects. New York: Elsevier Science 145 (1983).

16. Toledo-pareyra L.H., Simmons R.L., Olson L.C., Najarian J.S. Clinical Effect of Allopurinol on Preserved Kidneys: A Randomized Double-Blind Study. Ann Surg 185:128 (1977).

17. Parks D.A., Granger D.N., Bulkley G.B. Superoxide Radicals and Mucosal Lesions of the Ischemic Small Intestine (Abstract). Fed Proc 41:1742 (1982).

18. Casale A.S., Bulkley G.B., Bulkley B.H., Flaherty J.T., Gott V.L., Gardner T.J. Oxygen Free-Radical Scavengers Protect the Arrested, Globally Ischemic Heart Upon Reperfusion. Surg Forum 34:313 (1983).

EXAMPLE 32

Survival of Isolated Rabbit Cornea and Free Radical Scavengers

Human superoxide dismutase produced by the host vector system pSOD $\beta_1$T11 is E. coli A1645 described in Example 3, grown and purified under the conditions described in Example 26 was shown to prolong the survival period of excised isolated corneas.

The parenthesized arabic numbers found throughout this Example refer to the articles listed in the Bibliography at the end of this Example.

Previous studies established the beneficial effect of low concentration of adenosine on the rabbit corneal endothelial pump in vitro (1). Activation of the fluid pump was obtained by perfusing the isolated cornea with physiological concentration of glucose (5mM) and adenosine ($10^{-6}$M) in a balanced salt solution; the survival time was about 7 hours.

Our next goal was to increase the survival time. Superoxide dismutase (SOD) (2), scavenges the superoxide free radicals by catalyzing the reaction $O_2^- + O_2^- + 2H^+ \rightarrow H_2O_2 + O_2$. Administration of SOD is known to significantly increase the survival of ischemic tissues after partial anoxia (3).

Substantial degree of tissue damage resulting from ischemia occurs during the period of reperfusion and reoxygenation of the isolated tissues. Most of that injury is mediated through the superoxide radical and its descendants. The purpose of the present study is to determine if superoxide dismutase (SOD) or catalase are able to prolong the survival of the isolated cornea.

We isolated corneas from albino rabbit weighing 2.0-3.0 kg. The details of the techniques used have been previously described (1). Briefly, the eyes were excised and the corneal epithelia scraped off; then the corneas were isolated as described, and subjected to perfusion.

Results

The addition of 2 $\mu$/ml of SOD analog to the Basal Salt solution containing 5 mM glucose and 1$\mu$ adenosine prolonged the survival time of the isolated cornea from 7 hours to 14 hours. If the adenosine was eliminated, the survival time was prolonged to only 12 hours.

In summary, the SOD analog was demonstrated to be useful in prolonging the survival time of isolated corneas. The SOD analog may be important in prolonging the survival of other isolated organs as well. Similar data using bovine SOD has been published in Experimental Eye Research 4:153-154 (1985).

BIBLIOGRAPHY

1. Neuwirth, O. and Dikstein, S. (1983). The effect of cyclic AMP on the rabbit corneal endothelial fluid pump. Current Eye Res. 2(8), 565-567.

2. Fridovich, I. (1975). Superoxide dismutases. Ann. Rev. Biochem. 44, 147-159.

3. Manson, P.N., Robert, M., Anthenelli, M.M., Michael, J., Bulkley, G.B. and Hoopes, J.E. (1983). The role of oxygen-free radicals in ischemic tissue injury in Island skin flaps. Ann. Surg. 198, 87-90.

4. Michaelson, A.M. and Puget, K. (1980). Cell penetration by exogenous superoxide dismutase. Acta Physiol. Scand. Suppl. 492, 67-80.

5. Perlman, M. and Baum, J.L. (1974). The mass culture of rabbit corneal endothelial cells. Arch. Ophthalmol. 92, 235-237.

6. Klyce, S. and Maurice, D.M. (1976). Automatic recording of corneal thickness in vitro. Invest. Ophthalmol. 15, 550-553.

7. Neuwirth Lux, O. (1984). Survival of rabbit corneal endothelial pump. Ph.D. thesis, submitted to the Senate of the Hebrew University of Jerusalem.

EXAMPLE 33

Construction of p9200 and Production of bGH Using p9200 in E. coli A1645 and A4255

The construction of p9200 is shown in Figure 26 and described in the Description of the Figures. pHG44 was cleaved with ClaI and PstI, "filled in" using the Klenow fragment of DNA polymerase I and then the large DNA fragment was isolated. This fragment as ligated to a DNA fragment containing the tetracycline resistance gene of pBR322 which was isolated by cleaving pBR322 with RI and AvaI and then "filling in" using the Klenow fragment of DNA polymerase I. The resulting plasmid p9200 was deposited in the ATCC under Accession Number ATCC 53215.

The plasmid p9200 is similar to pHG44 (Figure 6) however the plasmid confers tetracycline resistance instead of ampicillin resistance. The plasmid p9200 has been introduced into E. coli strains A1645 and A4255 by transformation using methods known to those of ordinary skill in the art. These strains produced upon growth and induction and analog of bovine growth hormone (bGH) having the amino acid sequence met-asp-gln added to the amino-terminus of the phenylalanine form of the authentic bGH. The amount of bGH analog produced by these strains was roughly equivalent to that produced by pHG44.

The methods used to grow strain A1645/p9200 (host strain A1645 transformed with p9200), recover the bGH analog and purify the bGH analog are identical to that described in Example 13 for pHG44 except that 12.5 mg/l of tetracycline was added instead of 100 mg/l of ampicillin.

The methods used to grow and induce strain A4255/p9200, which has been designated A4320, are described in Example 36. The methods used to purify the bGH analog are identical to those described for pHG44 in Example 13.

The strain A4320 (host strain A4255 transformed with the plasmid p9200) was deposited in the ATCC under Accession Number ATCC 53215.

Replacement of the ampicillin gene with the tetracycline gene is advantageous in that ampicillin can now be eliminated from the production process, thereby eliminating possible contamination of the final product with ampicillin which can cause severe allergic reactions.

EXAMPLE 34

Production of met-leu-leu-leu-met Human ApoE Analog

Plasmid pTVR 279-8 directs expression of a novel apolipoprotein E3 analog. The N-terminal sequence of this analog is met-leu-leu-leu-met followed by the sequence of mature apolipoprotein E3.

Construction of pTVR 279-8

The construction of pTVR 279-8 is shown in Figure 25 and is described in the Description of the Figures. The plasmid pTVR 279-8 has been deposited in the ATCC under Accession No. 53216.

Plasmid pTV 190 (Figure 21) was partially digested with AvaI, "filled in" using the klenow fragment of DNA polymerase I, ligated and transformed into E. coli. The resulting plasmid in which the 3' AvaI site had been eliminated, designated pTV 264-45, was digested to completion with NdeI and ligated to the following synthetic oligonucleotides:

```
# 1217            5'-TATGCTGCTGCT
# 1218                 ACGACGACGAAT-5'
```

The resulting plasmid designated pTVR 279-8 was transformed into E. coli A1645 using methods known to those of ordinary skill in the art. Cells containing the plasmid were identified by colony hybridization using $^{32}$P-labelled oligonucleotide No. 1218 as a probe. The identity of the plasmid was confirmed by DNA sequencing. Strain A1645 containing pApoE Ex2 has been deposited in the ATCC under Accession No. 39787.

pTVR 279-8 produces upon growth and induction an analog of human apolipoprotein E3 having the amino acid sequence met-leu-leu-leu-met added to the N-terminus of natural apolipoprotein E3. The methods used to grow the strain are identical to those described for pTV-170 in Example 17 except that the period of induction at 42°C was 1-5 hours rather than 15 minutes.

pTVR 279-8 produces an ApoE3 analog which is less toxic to the bacteria than the met-ApoE3 analog produced by pTV 170 and pTV 190. The met-leu-leu-leu-met-ApoE3 analog continues to accumulate in the cell even after 60 minutes of induction at 42°C, reaching levels of 400-600 mg/liter culture.

EXAMPLE 35

Production of Human Growth Hormone Analog

Construction of pTV 300

The construction of pTV 300 is shown in Figure 27 and described in the Description of the Figures. The plasmid was constructed by inserting the hGH gene derived from the plasmid pTV 18(1) into the NdeI site of P579 (Figure 19). The construction of pTV 18(1) is disclosed in European Patent Publication No. 0 131 843 A1, published January 23, 1985 and also in the corresponding U.S. patent application Serial No. 514,188, filed July 15, 1983 which is hereby incorporated by reference.

Synthesis of hGH Analog

pTV 300 was introduced into E. coli strain A1645 by transformation using methods known to those of ordinary skill in the art. This strain produced upon growth and induction an analog of human growth hormone which is deleted of the first 13 amino acids of natural growth hormone. The methods used to grow the strain and purify the hGH analog are identical to those described in Example 13.

EXAMPLE 36

Construction of Prototrophic Strains and Production of bGH Using Prototrophic Hosts

Prototrophic strains of E. coli have been constructed which enable high level protein expression by many of the previously described plasmids even when grown in a minimal media. The advantages of a bacterial growth protocol using minimal media are:
    a) the bacteria can be grown to a higher cell density;
    b) it is easier to duplicate growth conditions as the media components are "simpler" and therefore of a higher quality; and
    c) the media is more economical.

The preferred prototrophic strains of this invention are designated A4200, A4255, and A4346. Strain A4255 containing the plasmid p9200 has been deposited with the ATCC under Accession No. 53215, and is referred to as A4320.

Selection and Construction of the Prototrophic Strains

The following strains were screened for high growth rates on minimal media, and sensitivity to phage$\lambda$, $\lambda$434 and phage PI:

## Strain

1. ATCC 12435
2. ATCC 3716
3. ATCC 27662

4. ATCC 25404
5. ATCC 11775
6. ATCC 25254
7. HfrC=A4134
8. W3350=A2509
9. A1645

Based on results of these studies, we focused the development of a prototrophic strain based on strains ATCC 12435 and ATCC 25404 which were sensitive to the above-listed phage and showed superior growth rates. Using these two strains, we constructed new strains containing the $\lambda$ cl857 repressor by transducing them with PI containing $\lambda$cl857 $\Delta$HI $\Delta$Bam HI:Tn10. Tetracycline resistant colonies were purified and cured of PI if necessary.

The resulting strains and their genotypes are:

A4200 = ATCC 12435 ($\lambda$cl857 $\Delta$HI $\Delta$Bam HI):Tn10

A4206 = ATCC 25404 ($\lambda$cl857 $\Delta$HI $\Delta$Bam HI):Tn10

Both strains were transformed with pHG44 yielding strains A4202 and A4207 respectively.

Growth and Induction

Strains A4202 and A4207 were grown and induced under the following conditions and assayed for production of bovine growth hormone analog.

Media

| Component | Concentration |
|---|---|
| $KH_2PO_4$ | 13.6 gm/liter |
| $(NH_4)_2SO_4$ | 2 gm/liter |
| $MgSO_4 \cdot 7H_2O$ | 0.2 gm/liter |
| $CaCl_2$ | 0.01 gm/liter |
| $FeSO_4 \cdot 7H_2O$ | 0.5 g/liter |
| pH 7.4 | |

62

| Supplements | |
|---|---|
| Glucose 20% solution | 25 ml/liter |
| Ampicillin 20 mg/ml solution | 1 ml/liter |
| B1 0.3% solution | 1 ml/liter |
| Biotin 0.3% solution | 1 ml/liter |

Both A4202 and A4207 grow well in minimal media; however, only A4202 expresses significant levels of the bGH analog. A4202 expresses the bGH analog at roughly the same level as pHG44 grown in rich media as described in Example 13.

Elimination of Tetracycline Resistance From A4200

In order to utilize the prototrophic strain A4200 with plasmids carrying only a tetracycline resistance marker, we constructed a strain cured of the Tn10 marker. Strain A4200 was streaked on MacConky galactose plates, gal$^+$ revertants were selected and tested for sensitivity to tetracycline and immunity to lambda phage. This strain was designated A4255.

Construction of Biotin Independent Prototrophic Strains

All of the above prototrophic strains contain the lambda cI857 delta HI delta Bam HI defective prophage. The delta HI deletion extends to the bio uvr B region, removing the biotin biosynthetic operons. Thus the strains require the addition of biotin to the growth media.

To eliminate the biotin requirement of strains derived from A4200 and A4255, we have introduced an F' episome from strain A89 into these strains.

Strain A89 carries an F' gal plasmid. We have demonstrated that this F' plasmid carries all the genes necessary for the endogenous synthesis of biotin. Several properties make this plasmid a convenient source of the bio operons:

1. F' gal is a unit copy plasmid.
2. The plasmid is extremely stable in E. coli.
3. F' plasmids are compatible with colE1 plasmid, on which our expression vectors are based.
4. F' gal is a conjugative plasmid. It can, therefore, be easily transferred from cell to cell.
5. One can easily screen for biotin independent strain.

Strain A4202 was conjugated for 30 minutes with A89, and biotin independent colonies were selected. The resulting strain A4346 yields high levels of bovine growth hormone analog following induction in minimal glucose medium in the absence of biotin. No other growth factor is required.

A4346 can be cured of pHG44 using standard methods known to those skilled in the art. The resulting prototrophic host strain can be transformed by all the plasmids described in this application.

Minimal Media

The minimal media standardly used for production purposes with the prototrophic strains was:

| | For Fermenters | For Shake Flasks |
|---|---|---|
| $K_2HPO_4$ | 6 g/l | 6 g/l |
| $KH_2PO_4$ | 4 g/l | 4 g/l |
| $NH_4Cl$ | 1 g/l | 1 g/l |
| $MgSO_4 \cdot 7H_2O$ | 3 g/l | 0.2 g/l |
| 10% $FeNH_4$ Citrate | 0.3 ml/l | 0.1 ml/l |
| Trace Elements Solution | 3 ml/l | 1 ml/l |
| Antifoam, Silicone | 0.5 ml/l | |

Autoclave.

Ampicillin (100 mg/l) or tetracycline (12.5 mg/l) may be added to the media depending on whether the strain is ampicillin or tetracycline resistant, respectively.

50% glucose was autoclaved separately and added to 20 gm/l. 50% glucose was fed during the fermentation at a rate of approximately 1.08 gm/glucose per O.D. unit for strains without the F' episome, or at a rate of 1.8 gm/glucose per 2.0 unit for strain A4346. The pH was controlled by feeding 25% $NH_4$. Antifoam was added as needed. Biotin was added at 15 mg/l for strains based on A4200, A4255 and A4206.

The trace elements solution contains (Biotechnol. Bioeng. 16:933-941 (1974)):

|  | g/l |
|---|---|
| $H_3BO_3$ | 0.57 |
| $CuCl_2$ ($CuSO_4 \cdot 5H_2O$) | 1.0 (0.04) |
| $CaCl_2 \cdot 2H_2O$ | 1.0 |
| $MnSO_4 \cdot 4H_2O$ | 0.81 |
| $Na_2MoO_4 \cdot 2H_2O$ | 2.0 |
| $CaCl_2 \cdot 6H_2O$ | 2.0 |
| $ZnCl_2 \cdot 4H_2O$ ($ZnSO_4 \cdot 7H_2O$) | 2.0 (2.78) |
| Concentrated HCl | 100 ml |

The compounds in parenthesis are alternate compounds which may be used in place of the compounds preceding them. The parenthesized amounts refer to appropriate amounts of such alternative compounds. Many of the previously described plasmids have been introduced into the prototrophs A4200 and A4255. A partial list of these strains is described in the table below:

| Strain Designation | Host Strain/Plasmid |
|---|---|
| A4202 | A4200/pHG44 |
| A4256 | A4255/pHG44 |
| A4320 | A4255/p9200 |
| Z1803 | A4255/pSOD $_1$ T11 |
| A4500 | A4255/pTV 194 |
| A4346 | A4200/pHG44, F'Gal |

## EXAMPLE 37

### Construction of Lytic Hosts and Production of bGH Using These Lytic Hosts

#### 1. Construction of Strains A4048 and A3111

The strain W3350 has been used extensively for growing phage lambda (see, for example, Oppenheim and Salomon (1970), 41 Virology, 151-159). Strain A2509, a prototrophic derivative of W3350, was transduced by Plclts grown on A1637, and also transduced with a λcl857 ΔHlΔBam Hl defective prophage carrying the Tn10 marker. The resulting strain A4048 was selected as a tetracycline resistant clone carrying the defective prophage λcl857Δ Hl Δ Bam Hl. This strain also carried a Plclts plasmid. This strain was transformed by pHG44 to yield strain A3111.

Similarly, strain A4085 was also constructed from A2509 by the insertion of λ cl857 Δ Hl Δ Bam Hl, the removal of the Tn10 transposon and the curing of the Plclts plasmid prior to transformation with pHG44. Strain A4085 carries the defective prophage λcl857ΔHlΔ Bam Hl and serves as a control for measuring bGH production and the autolysis affected without the presence of the PI plasmid. Strain A3111 has been deposited in the ATCC under Accession No. 53217.

#### 2. Synthesis of Bovine Growth Hormone (bGH).

Stock Cultures: Stock cultures of strain A3111 (pHG44 in A4048 cells) were grown overnight at 30°C in LB medium containing 50 $\mu$g/ml ampicillin (Amp). the cultures were diluted two-fold with 50% glycerol and stored at -20°C.

Inoculum: The inoculum was obtained from a single colony of A3111 grown on an LB agar plate containing 100$\mu$ g/ml Amp. The LB plates, in turn, were spread with material taken from the stock cultures.

Sterile 3 ml LB medium containing 50 $\mu$g/ml Amp was inoculated with a single colony of A3111 and grown for 18 hours at 30°C in a shaker bath.

Production: Production of bGH was carried out in BHI medium (37 $\mu$g/l brain heart infustion (Difco)) containing 50 $\mu$g/ml Amp. The inoculum was diluted 1:100 into a flask containing fresh BHI + 50 $\mu$g/ml Amp, and grown in a shaker bath at 30°Cuntil the cell concentration reached about $4 \times 10^8$ cells/ml (OD.$_{600}$ = 0.5).

For the induction of bGH production, the flask was transferred to a shaker bath set at 42°C. Cell samples were taken at time 0 and at 90 minutes after the beginning of induction.

Analysis of bGH Production: bGH production was analyzed on a 10-26% gradient acrylamide gel. The cell samples were spun in a microfuge, the supernatant was removed, and the pellets were dissolved in sample buffers (2% SDS, 50mM Tris pH 7.0, 3% sucrose, 5% $\beta$-Mercaptoethanol) and loaded on the gel. After electrophoresis at 200 volts for 2-1/2 hours, the gels were stained with Coomassie blue and the amount of bGH was determined by scanning with a gel scanner.

After 90 minutes of induction at 42°C, bGH comprises about 20% of the total protein of A3111.

## 3. Autolysis

Strain A3111 carries pHG44, the defective prophage λcI857Δ HI ΔBam HI, and a stable Plclts plasmid. After prolonged induction at 42°C, the cells will start to lyse due to the production of endolysin directed by PI. Complete lysis of the culture was achieved after 2.5-3 hrs.

Test of Controlled Autolysis: 5 ml of A3111 culture were taken before and after induction at 42°C (90 min.) and spun down in a Sorvall centrifuge at 7000 rpm for 7 minutes. The supernatants were removed and the pellets were frozen at -20°C overnight. The pellets were then resuspended in 0.5 ml of T.E. buffer (10 mM Tris pH 8.0, 1 mM EDTA) and 0.1 ml was diluted 1:10 with T.E. and the OD.$_{600}$ determined.

As a control for this experiment we used strain A4085 which, similarly to A3111, contains pHG44 and the defective prophage λ cI857 Δ HI Δ Bam HI but does not carry the Plclts plasmid.

The results of such an experiment appear in Table V which shows that cells containing the Plclts plasmid (A3111) lyse immediately upon thawing. Inspection of the thawed mixture revealed that over 95% of the cells were lysed following this treatment.

TABLE V

| Strain | Plclts | OD.$_{600}$ | | % Loss |
|--------|--------|-------------|-------------|--------|
| | | Before Freezing | After Thawing | |
| A4085 | - | 0.980 | 0.851 | 14 |
| A3111 | + | 0.543 | 0.08 | 86 |

The lysis procedure simplifies the extraction of bGH from the induced cells without affecting bGH production.

## EXAMPLE 38

### Biological Activity of the Met-ApoE3 Analog.

Bacteria pTV 194 were grown as described in Example 17.

The numbers in parenthesis refer to the references found at the end of this example.

### Analysis of Bacterial Extracts

Bacterial cells were harvested by centrifugation and suspended in 50 mM potassium phosphate buffer, pH 7.5, containing 5 mM EDTA and 2 mM phenylmethyl sulfonyl fluoride. Aliquots were lysed in 1.5 X sample buffer (15% glycerol, 4.5% NaDodSO, 1 mM $\beta$-mercaptoethanol, 93.5 mM Tris•HCl, 0.25% Bromophenol blue, pH 6.8), heated at 100°C for 10 minutes and proteins analyzed on 10% NaDodSO$_4$ polyacrylamide gels (26). Proteins separated on polyacrylamide gels were either stained with Coomassie brilliant blue or were electrophoretically transferred to nitrocellulose sheets (27) and reacted with $^{125}$I-labeled anti-human ApoE monoclonal or polyclonal IgG. The immunoblots were washed, air dried and exposed to X-ray film.

### Isolation of ApoE

Authentic ApoE was isolated from the d< 1.02 plasma lipoproteins of a hypertriglyceridemic subject (E3/3 phenotype) by Sephacryl S-300 column chromatography as previously described (14). The E3 isoform was obtained by preparative Immobiline isoelectric focusing (LKB Instruments, Bromma, Sweden, pH range 4.9 to 5.9) (28).

The ApoE analog was isolated from 33 g of lyophilized cells, which represented the cell mass from a five liter fermentation. The cells were ground to a fine powder with the aid of 22 g of alumina (Buehler Ltd., Evanston, Illinois) in a chilled (4º) mortar and pestle. The ground cells were extracted with 300 ml 6 M urea (freshly deionized), containing 0.1 M $NH_4HCO_3$, 2 mM PMSF, 0.1% Trasylol (Mobay Chemical Corp., New York, NY) (pH 7.8). The insoluble cellular residue was sedimented by ultracentrifugation at 4º in a Beckman SW28 rotor (25,000 rpm for 50 minutes) and reextracted with 200 ml of 6 M urea buffer. The combined supernatant fractions were dialysed against three changes of 2 M urea, containing 25 mM $NH_4HCO_3$, 2 mM PMSF, 2 mM EDTA, 0.1% Trasylol, 0.1% $\beta$-mercaptoethanol (pH 7.4). Following dialysis, the extract supernatant was added to ~ 200 ml heparin-Sepharose, which was prepared as described (29) and equilibrated with the 2 M urea buffer. The gel-supernatant mixture was incubated overnight at 4º on a rotating platform and then packed into a glass column (4.0 x 3.5 cm, Kontes Glass, Vineland, NJ). The material not bound to the heparin-Sepharose was washed from the column by pumping ~300 ml of 2 M urea buffer at a rate of 25 ml/h through the column. The bound material was then eluted from the column with 50 ml of 1.0 M $NH_4HCO_3$ in 2 M urea and then dialyzed against 5 mM $NH_4HCO_3$ and lyophilized. This semi-purified ApoE was solubilized in 15 ml 6 M guanidine, containing 0.1 M Tris•HCl, 1 mM EDTA, 1.0% $\beta$-mercaptoethanol (pH 7.4) and applied to a Sephacryl S-300 (Pharmacia Fine Chemicals, Uppsala, Sweden) column (2.5 x 300 cm), equilibrated with 4 M guanidine, 0.1 M Tris•HCl, 1 mM EDTA, 0.1% $\beta$-mercaptoethanol (pH 7.4). The fractions containing ApoE were pooled, exhaustively dialyzed against 5 mM $NH_4HCO_3$ and lyophilized. Final purification was accomplished by preparative isoelectric focusing on an immobiline gel (28).

### Structural Characterization of the ApoE Analog

Protein or peptide samples for amino acid analysis were hydrolyzed in 6 N HCl for 20 h at 110ºC in sealed, evacuated tubes. Analyses were performed on a Beckman 121 MB Analyzer equipped with a model 126 Data System.

Peptides for amino acid and sequence analyses were generated by digesting 3 mg of Sephacryl S-300 column-purified ApoE with 90 mg CNBr in 600 $\mu$l 70% HCOOH for 30 h at room temperature. Resultant peptides were separated on a Sephadex G-50 column as previously described for authentic ApoE (4).

Sequence analyses were performed on an updated Beckman 890C Sequencer using a standard 0.1 M Quadrol program. The intact protein was degraded in the presence of 3 mg polybrene and 0.5% $NaDodSO_4$; peptides were degraded in the presence of polybrene only. Phenylthiohydantoin amino acids were identified and quantified by high performance liquid chromatography as previously described (14).

Analytical isoelectric focusing and $NaDodSO_4$ polyacrylamide gel electrophoresis were performed (14). Charge modification with cysteamine ($\beta$-mercaptoethanolamine) was done (14).

### Biological Characterization of the ApoE Analog

Phospholipid complexes of ApoE analog and dimyristoylphosphatidylcholine (DMPC) were prepared and isolated as previously described (30). Lipoprotein receptor binding assays were performed as described for fibroblasts (31) and hepatic membranes (32). Iodinations of ApoE were performed in 0.10 M $NH_4HCO_3$ with Iodo-Beads (Pierce) according to manufacturer's directions.

For rabbit and rat in vivo studies, iodinated authentic and ApoE analog (90 $\mu$g each) were incubated for 30 minutes at room temperature with 1 ml of rabbit or rat plasma prior to injection into male New Zealand white rabbits. Plasma radioactivity is reported as TCA-precipitable protein using the precipitation method previously described (33). Calculation of the percentage of the injected dose remaining in plasma at the various time intervals was based on a plasma volume estimate of 4.5% of the body mass.

### Results and Discussion

### Expression of Human ApoE

Following induction of cells transfected with pTV 194, a protein with an apparent molecular weight identical to that of ApoE was specifically induced. This induced protein reacted with anti-human ApoE antibodies (not shown).

With induction periods of 30 minutes or longer, lysis of the cells was observed (Fig. 28). This cell lysis was associated with the intracellular accumulation of ApoE. Noninduced cells maintained at 30ºC were stable (Fig. 28). This cellular toxicity lysis was not a general feature of this expression vector as this same expression system containing a human growth hormone cDNA did not show this effect. ApoE was destroyed by proteolysis following cell lysis. The problem of toxicity caused by ApoE accumulation was overcome by inducing the cells for short periods of time (~20 minutes) and then cooling the cells by addition of ice to the fermenter.

As determined by solid-phase radioimmunoassay, the ApoE levels in cells induced for short periods of time were approximately 1% of soluble cellular protein. The ApoE was isolated and purified from cell extracts by heparin-Sepharose and Sephacryl S-300 chromatography. This two-step process resulted in an ApoE preparation that was greater than 90% pure, with a yield representing approximately 20% of the ApoE present in the cell extract. Final purification for characterization was accomplished by the preparative immobiline isoelectric focusing. The purification scheme used in these studies was not optimized for total recovery but, rather, was designed to obtain pure material for characterization.

Structural Characterization of the ApoE Analog

The Immobiline-purified ApoE analog migrated as a single band on SDS gels with an apparent $M_r$ identical to that of authentic ApoE. On isoelectric focusing gels the bioengineered ApoE focused as one major band with a pI identical to that of Immobiline-purified ApoE3. Consistent with the presence of one residue of cysteine, the ApoE analog was shifted one charge unit toward the anode after cysteamine modification. Amino acid analysis of the Immobiline-purified product was compared to authentic human ApoE3 purified by the same method. As shown in Table VI, the analyses of the ApoE analog and authentic ApoE were nearly identical to each other and to the theoretical composition derived from previous sequence analysis of human ApoE. The analyses suggested, however, that the ApoE analog product contained an additional residue of methionine compared to the authentic ApoE. In addition, the presence of one cysteine residue, suggested by cysteamine treatment, was confirmed.

Sequence analysis of the intact ApoE analog (ca. 6 nmole) demonstrated that the extra methionine residue was at the $NH_2$-terminus of the protein and yielded a single sequence of Met-Lys-Val-Glu-Gln-Ala-Val-Glu-Thr-Glu-Pro-Glu-Pro-Glu-Leu-Arg-Gln-Gln-. The sequence following the methionine corresponds to residues 1-17 of human ApoE. These results established that the synthetic oligonucleotide used to reconstruct the $NH_2$-terminal coding portion of ApoE was correctly translated and that the extra methionine (whose codon was added for bacterial translation initiation) was not removed by any processing mechanism. The initial yield in the sequence analysis was unexpectedly low (ca. 20%), but this was probably not due to a portion of the $NH_2$-terminal methionine being formylated, because the formylated protein would have a pI distinctly different from the non-formylated polypeptide (and from authentic ApoE) and this was not observed.

Several of the CNBr peptides were characterized as to their amino acid compositions and were found to be no different from those of authentic ApoE (not shown). In addition, sequence analysis of CB4 (residues 109-125 in authentic ApoE) and partial sequence analysis of CB5 (through the residue corresponding to position 164 in ApoE) established that the sequence of the ApoE analog was identical to authentic ApoE3 in the crucial receptor binding domain. All these data indicated that, except for the additional methionine at the $NH_2$-terminus, the ApoE analog was identical in structure to authentic ApoE3.

In Vitro and In Vivo Metabolic Characterication of the ApoE Analog

Comparison of the receptor binding of ApoE•DMPC complexes demonstrated that the ApoE analog possessed binding properties essentially identical to those of authentic ApoE. In competition studies using $^{125}$I-LDL bound to ApoB, E (LDL) receptors on cultured fibroblasts, the 50% displacement concentration for the ApoE analog was 0.019 $\mu$g/ml compared to 0.024 $\mu$g/ml for authentic ApoE (Fig. 29). In direct binding studies to fibroblasts both ApoE preparation bound in a similar manner (Fig. 29). Scatchard analysis of the direct binding data revealed that the Kds and maximum amount bound for bioengineered and authentic ApoE were 0.93 and 0.96 x $10^{-10}$ M and 29.4 and 34.2 $\mu$g/mg of cell protein, respectively. In addition, both ApoE preparations also bound similarly and effectively to ApoE receptors on canine hepatic membranes (Fig. 30).

Comparison of the in vivo metabolic properties of the ApoE analog and authentic ApoE also demonstrated that both preparations behaved in an identical manner. When $^{131}$I-ApoE analog and $^{125}$I-authentic ApoE were mixed and incubated with normal rabbit plasma and then the mixture injected into a normal rabbit, both labels were removed from circulation with identical kinetics (Fig. 31). Clearance of 50% of the injected dose of both labels occurred at approximately 20 minutes after the injection. Identical results were obtained with reciprocally labeled proteins and also when turnover studies were done in rats (not shown). Thus, in both in vitro and in vivo studies, the bioengineered and authentic ApoE exhibited similar properties and behaved in essentially identical manners.

In summary, structural characterization of the isolated ApoE analog demonstrated that with the exception of an additional methionine residue at the amino terminus the structure of the ApoE analog was identical to authentic ApoE. In addition, both the in vitro and in vivo metabolic properties of the ApoE analog and authentic ApoE were identical.

TABLE VI

| Amino Acid Composition of the ApoE Analog and Authentic ApoE3 [1] | | | |
|---|---|---|---|
| | ApoE3 Analog | Authentic ApoE3 | ApoE3 Sequence |
| Lys | 12.1 | 12.0 | 12 |
| His | 2.0 | 2.0 | 2 |
| Arg | 33.3 | 33.3 | 34 |
| Cys | 0.8 | 0.8 | 1 |
| Asp | 12.3 | 12.4 | 12 |
| Thr | 10.5 | 10.5 | 11 |
| Ser | 12.7 | 12.6 | 14 |
| Glu | 72.0 | 71.9 | 71 |
| Pro | 8.5 | 8.4 | 8 |
| Gly | 17.3 | 17.3 | 17 |
| Ala | 35.6 | 35.5 | 35 |
| Val | 21.9 | 22.3 | 22 |
| Met | 7.7 | 6.5 | 7 |
| Ile | 1.9 | 1.9 | 2 |
| Leu | 37.0 | 37.3 | 37 |
| Tyr | 3.8 | 3.9 | 4 |
| Phe | 3.2 | 3.2 | 3 |
| Trp | n.d. | n.d. | 7 |

[1]Results are from duplicate determinations and are expressed as residues per mole. Cysteine was determined separately after performic acid oxidation. Threonine and serine values were not corrected for hydrolytic loss. Tryptophan was not determined. ApoE3 sequence is from reference 4.

BIBLIOGRAPHY

1. Mahley R.W. (1978) in Disturbances in Lipid and Lipoprotein Metabolism, eds. Dietschy J.M., Gotto A.M. Jr. and Ontko J.A. (American Physiological Society, Bethesda, MD), pp. 181-197.
2. Mahley R.W., Innerarity T.L., Rall S.C. Jr. & Weisgraber K.H. (1984) J. Lipid Res. 25, 1277-1294.
3. Mahley R.W. & Innerarity T.L. (1983) Biochim. Biophys. Acta 737, 197-222.
4. Rall S.C., Jr., Weisgraber K.H. and Mahley R.W. (1982) J. Biol. Chem. 257, 4171-4178.
5. McLean J.W., Elshourbagy N.A., Chang D.J., Mahley R.W. and Taylor J.M. (1984) J. Biol. Chem. 259, 6498-6504.
6. Olaisen B., Teisberg P. and Gedde-Dahl T. Jr. (1982) Hum. Genet. 62, 233-236.
7. Paik Y. K., Chang D.J., Reardon C.A., Davies G.E., Mahley R.W. and Taylor J.M. Proc. Natl. Acad. Sci. USA, in press.
8. Utermann G., Langenbeck U., Beisiegel U. and Weber W. (1980) Am. J. Hum. Genet. 32, 339-347.
9. Zannis V.I. and Breslow J.L. (1981) Biochemistry 20, 1033-1041.
10. Zannis V.I., Breslow J.L., Utermann G., Manley R.W., Weisgraber K.H., Havel R.J., Goldstein J.L.,

Brown M.S., Schonfeld G., Hazzard W.R., Blum C. (1982) J. Lipid Res. 23, 911-914.

11. Utermann G., Steinmetz A. and Weber W. (1982) Hum. Genet. 60, 344-351.

12. Havel R.J. (1982) Med. Clin. North Am. 66, 441-454.

13. Menzel H.J., Kladetzky R.G. and Assmann G. (1983) J. Biol. Chem. 256, 9077-d9083.

14. Weisgraber K.H., et al., (1981) J. Biol. Chem. 256, 9077-9083.

15. Rall S.C. Jr., Weisgraber K.H., Innerarity T.L. and Mahley R.W. (1982) Proc. Natl. Acad. Sci. USA 79, 4696-4700.

16. Rall S.C. Jr., Weisgraber K.H., Innerarity T.L., Mahley R.W. and Assmann G. (1983) J. Clin. Invest. 71, 1023-1031.

17. Weisgraber K.H., Rall S.C. Jr., Innerarity T.L., Mahley R.W., Kuusi T. and Ehnholm C. (1984) J. Clin. Invest. 73, 1024-1033.

19. Mahley R.W., Innerarity T.L., Rall S.C. Jr. and Weisgraber K.H. Ann. NY Acad. Sci., in press.

20. Innerarity T.L., Friedlander E.J., Rall S.C. Jr., Weisgraber K.H. and Mahley R.W. (1983) J. Biol. Chem. 258, 12341-12347.

20a. Innerarity T.L., Weisgraber K.H., Arnold K.S., Rall S.C. Jr. and Mahley R.W. (1984) J. Biol. Chem. 259, 7261-7267.

21. Weisgraber K.H., Innerarity T.L., Harder K.J., Mahley R.W., Milne R.W., Marcel Y.L. and Sparrow J.T. (1983) J. Biol. Chem. 258, 12348-12354.

26. Laemmli U.K. (1970). Nature (London) 227:680-685.

27. Towbin H., Staehelin T. and Gordon J. (1979). Proc. Natl. Acad. Sci. USA 148:107-127.

28. Manzel, H.J., et al., (1984) J. Biol. Chem. 254: 3070-3076 (1984).

29. Weisgraber K.H. and Mahley R.W. (1980) J. Lipid Res. 21, 316-325.

30. Innerarity T.L., Pitas R.E. and Mahley R.W. (1979) J. Biol. Chem. 254, 4186-4190.

31. Innerarity T.L. and Mahley R.W. (1978) Biochemistry 17, 1440-1447.

32. Hui D.Y., Innerarity T.L. and Mahley R.W. (1981) J. Biol. Chem. 256, 5646-5655.

33. Mahley R.W., Innerarity T.L., Weisgraber K.H. and Oh S.Y. (1979) J. Clin. Invest. 64, 743-750.

EXAMPLE 39

Biological Activity of the met-asp-gln-bGH Analog

A recent study (P.J. Eppard and D.E. Bauman, Proceedings of 1984 Cornell Nutrition Conference for Feed Manufacturers, pp. 5-12) indicated that continued administration of methionyl-bovine growth hormone over a 12-week period increased milk yields from 10 to 40% as compared with controls. Similar results have been obtained using the met-asp-gln-bGH analog encoded by the pHG44 plasmid upon growth, recovery and purification as described in Example 13.

EXAMPLE 40

Biological Activity of the met-pGH Analog

A recent study (C.A. Spence et al., Abstract entitled "Effect of Exogenous Growth Hormone On Fetal Energy Storage and Lactation Performance in Sows," from The Annual Meeting of The American Society of Animal Science, Univ. of Missouri, Aug. 7-10, 1984) indicate that administration of pituitary-derived porcine growth hormone increases sow lactation and piglet litter survival. In a study of lactation performance, administration of the met-pGH analog to pregnant sows improved sow lactation and piglet litter survival.

EXAMPLE 41

Use of Recombinant SOD in Treating Spinal Cord Ischemia

Anesthetized dogs were connected to the Nicolet Compact 4 evoked potential system, and we obtained the baseline SEP (Somatosensory Evoked Potentials) by applying 250 stimuli consecutively at the rate of 4.7 stimuli per second to the posterior tibial nerve. The evoked potential of 250 stimuli were recorded from 2 electrodes over the Fpz and Cz (two specific points over the scalp), averaged by signal averager to reduce the signal to noise ratio, and the SEP was displayed on a screen.

A left thoracotomy was then performed, the descending aorta just distal to the left subclavian artery was dissected and isolated in preparation for the application of the crossclamp. A purse string was inserted proximal to the proposed site of the aortic crossclamp and a size 20 gauge cannula inserted for monitoring the proximal aortic pressure and infusion of the medication as in the experimental group. A size 14 gauge cannula was inserted into the right femoral artery for BP monitoring and removal of blood to control BP after the aortic crossclamp is applied. Serial blood gases were taken and the respirator was adjusted to maintain the blood gases within normal limits.

The aortic crossclamp was then applied just distal to the left subclavian artery. SEP is repeated at one minute intervals. The proximal aortic hypertension was controlled by removing blood from the femoral artery to maintain BP at 90-110 mm Hg mean. The aortic crossclamp was maintained for 10 more minutes after the SEP disappear. Disappearance of the SEP tracing signifies that the ischemia within the spinal cord produced by the crossclamping of thoracic aorta is severe enough to compromise the conduction of afferent impulses within the dorsal column of the spinal cord. The crossclamp was removed 10 minutes after the disappearance of SEP. The dogs would become hypotensive which responded to infusion of blood, Ringer's lactate and sodium bicarbonate.

In control dogs (n = 8), the animals did not receive any recombinant SOD. In the experimental animals, one group (n = 8) received a bolus of 25,000 units of recombinant SOD prior to removal of the crossclamp followed by 5,000 units per minute for 10 minutes; the second experimental group (n = 7) received 5,000 units of recombinant SOD prior to removal of the crossclamp followed by 10,000 units per minute for 10 minutes.

Postoperatively, the neurological status of the hind limbs was accessed by Tarlov's criteria: 0 = no movement in hind limbs; 1 = slight movement of hind limbs; 2 = good movement of hind limbs, but unable to stand; 3 = able to stand but not normally; and 4 = complete recovery. On the seventh postoperative day, SEP were repeated and recorded for comparison to the baseline. The animals were then sacrificed.

The results are:

Neurological status on the seventh postoperative day (POD):

Control animals (n = 8)
- 4 animals were grade 0
- 4 animals were either grade 2 or 3

Experimental animals (I) - 25,000 units SOD bolus and 5,000 units/minute X 10 minutes.
- 6 animals showed complete recovery
- 2 animals were in either grade 2 or 3.

Experimental animals (II) - 50,000 units SOD bolus and 10,000 units/minute X 10 minutes.
- all 7 animals showed complete recovery.

Time taken for SEP to disappear after application of aortic crossclamp varies from 12 to 19 minutes. Since the crossclamp was maintained for 10 more minutes after SEP disappear; the total crossclamp time will be more than 20 minutes.

In the immediate postoperative period after the closure of thoracotomy wound, repeat SEP were taken. In the control animals, there was no SEP tracing discernible, in contrast, the treated animals showed a return of SEP tracing with delay in the latency of the waveform.

In summary, recombinant SOD proved to be useful in preventing neurologic injury due to spinal cord ischemia. This method of treatment is especially important in surgery of the aneurysms of the thoracic aorta.

**Claims**

**Claims for the following Contracting States : BE, CH, DE, DK, ES, FR, GB, GR, IT, LI, LU, NL, SE**

1. A plasmid which upon introduction into a suitable bacterial host cell containing the thermolabile repressor $C_1$ renders the host cell capable, upon increasing the temperature of the host cell to a temperature at which the repressor is inactivated, of effecting expression of a desired eucaryotic gene inserted into the plasmid and production of a eucaryotic polypeptide encoded by the gene comprising:

   a double-stranded DNA molecule which includes in 5' to 3' order the following:

   a DNA sequence which contains the promoter and operator $P_LO_L$ from $\lambda$ bacteriophage;

   an N utilization site for binding antiterminator N protein;

   a first restriction enzyme site permitting replacement of the DNA sequence containing the ribosomal binding site which follows thereafter;

a ribosomal binding site for rendering the mRNA of the desired gene capable of binding to ribosomes within the host cell selected from the group consisting of:

a mutant of $C_{II}$ from λ bacteriophage having the sequence

TAAGGAAGTACTTACAT
ATTCCTTCATGAATGTA;

a natural β-lactamase ribosomal binding site derived from pBR322 (ATCC Accession No. 37017);

a synthetic oligonucleotide having the sequence

AATTCGAGCGCAAGGAAACAGGCTCA
GCTCGCGTTCCTTTGTCCGAGTAT;

and a synthetic oligonucleotide having the sequence

AATTCAATAATATTGAAAAAGGAAGAG
GTTATTATAACTTTTTCCTTCTCAT;

an ATG initiation codon; and

a second restriction enzyme site having inserted therein the desired gene encoding the eucaryotic polypeptide or analog thereof in phase with the ATG initiation codon;

and which additionally includes a DNA sequence which contains an origin of replication from a bacterial plasmid capable of autonomous replication in the host cell and a selection means selected from the group consisting of DNA sequences which contain a gene associated with a selectable or identifiable phenotypic trait which is manifested when the vector is present in the host cell and DNA sequences which contain the fragment designated $cI^{434}$, such fragment including the gene for the $cI^{434}$ repressor protein and its associated promoter and operator sequence, and wherein the distance between the 3' end of the $P_L O_L$ promoter and operator sequence and the 5' end of the N utilization site is less than about 80 base pairs and the distance between the 3' end of the N utilization site and the 5' end of the ribosomal binding site is less than about 300 base pairs.

2. A plasmid of claim 1, which additionally comprises a DNA sequence which contains a $T_1 T_2$ rRNA transcription termination sequence located 3' of the second restriction enzyme site.

3. A plasmid of claim 1, wherein the selection means is a DNA sequence which contains a gene associated with a selectable or identifiable phenotypic trait.

4. A plasmid of claim 3, wherein the phenotypic trait is drug resistance.

5. A plasmid of claim 4, wherein the drug resistance is resistance to ampicillin or tetracycline.

6. A plasmid of claim 2, wherein the $T_1 T_2$ rRNA transcription termination sequence is less than about 100 base pairs from the 3' end of the second restriction enzyme site.

7. A plasmid of claim 6, wherein the $T_1 T_2$ rRNA transcription termination sequence is less than about 20 base pairs from the 3' end of the second restriction enzyme site.

71

**8.** A plasmid of claim 1, wherein the selection means is a DNA sequence which contains the fragment designated $cI^{434}$.

**9.** A plasmid of claim 8, wherein the $cI^{434}$ fragment is located after the 3' end of the $T_1 T_2$ rRNA transcription termination sequence.

**10.** A plasmid of claim 1, wherein the origin of replication is derived from pBR322 (ATCC Accession No. 37017).

**11.** A plasmid of claim 1, wherein the origin of replication is from a bacterial plasmid capable of autonomous reproduction in the host cell and production of at least 400 constitutive copies of the plasmid.

**12.** A plasmid of claim 11, wherein the origin of replication is derived from ColE1.

**13.** A plasmid of claim 12, wherein the origin of replication is pOP1Δ6.

**14.** A plasmid of claim 1, further comprising a third restriction enzyme site located between the DNA sequence containing the ribosomal binding site and the ATG initiation codon.

**15.** A plasmid of claim 1, wherein the first restriction enzyme site is a unique site within the plasmid.

**16.** A plasmid of claim 15, wherein the first unique restriction enzyme site is EcoRI.

**17.** A plasmid of claim 1, wherein the second restriction enzyme site is a unique site within the plasmid.

**18.** A plasmid of claim 17, wherein the second unique restriction enzyme site is NdeI, BglII or SmaI.

**19.** A plasmid for production of bovine growth hormone or an analog thereof which comprises the plasmid of claim 1, wherein the DNA gene sequence encoding the eucaryotic polypeptide is DNA encoding bovine growth hormone or an analog thereof, inserted into the second restriction enzyme site.

**20.** A plasmid of claim 19, wherein the plasmid is pRO12 having the restriction map shown in Fig. 2 and being 3.3 kb in length; pSAL 5200-6 having the restriction map shown in Fig. 3 and being 3.3 kb in length; pHG44 having the restriction map shown in Fig. 6 and deposited under ATCC Accession No. 39806; pSAL 5600-1 having the restriction map shown in Fig. 10 and being 4.4 kb in length; p7200-22 having the restriction map shown in Fig. 7 and being 4.9 kb in length; pHG50 having the restriction map shown in Fig. 6 and deposited under ATCC Accession No. 39805; pSAL-210/4 having the restriction map shown in Fig. 9 and being 12.6 kb in length; p8300-10A having the restriction map shown in Fig. 7 and deposited under ATCC Accession No. 39785; pSAL-130/5 having the restriction map shown in Fig. 8 and being 9.6 kb in length; pSAL-170/10 having the restriction map shown in Fig. 8 and being 11.0 kb in length; or p9200 having the restriction map shown in Fig. 26 and deposited under ATCC Accession No. 53215.

**21.** A plasmid for production of human growth hormone or an analog thereof which comprises the plasmid of claim 1, wherein the DNA gene sequence encoding the eucaryotic polypeptide is DNA encoding human growth hormone or an analog thereof inserted into the second restriction enzyme site.

**22.** A plasmid of claim 21, wherein the plasmid is pTV300, has the restriction map shown in Fig. 27, and is 4.3 kb in length.

**23.** A plasmid for production of superoxide dismutase or an analog thereof which comprises the plasmid of claim 1, wherein the DNA gene sequence encoding the eucaryotic polypeptide is DNA encoding superoxide dismutase or an analog thereof inserted into the second restriction enzyme site.

**24.** A plasmid of claim 23, wherein the plasmid is pSODα2 having the restriction map shown in Fig. 13 and deposited under ATCC Accession No. 39786; pSODβ1 having the restriction map shown in Fig. 14 and being 3.0 kb in length; pSODβ$_1$T$_{11}$ having the restriction map shown in Fig. 15 and being 3.7 kb in

EP 0 173 280 B1

length; pSOD$\beta_1$-BA2 having the restriction map shown in Fig. 17; or pSOD$\beta_1$TT-1 having the restriction map shown in Fig. 16 and being 4.8 kb in length.

25. A plasmid for production of porcine growth hormone or an analog thereof which comprises the plasmid of claim 1, wherein the DNA gene sequence encoding the eucaryotic polypeptide is DNA encoding porcine growth hormone or an analog thereof inserted into the second restriction enzyme site.

26. A plasmid of claim 25, wherein the plasmid is p3008, has the restriction map shown in Fig. 4, and is deposited under ATCC Accession No. 39804, or the plasmid is p3009, has the restriction map shown in Fig. 11, and is 4.3 kb in length.

27. A plasmid for production of chicken growth hormone or an analog thereof which comprises the plasmid of claim 1, wherein the DNA gene sequence encoding the eucaryotic polypeptide is DNA encoding chicken growth hormone or an analog thereof inserted into the second restriction enzyme site.

28. A plasmid of claim 27, wherein the plasmid is p5002, has the restriction map shown in Fig. 5, and is 3.3 kb in length, or the plasmid is p5003, has the restriction map shown in Fig. 12, and is deposited under ATCC Accession No. 39792.

29. A plasmid for production of human apolipoprotein E or an analog thereof which comprises the plasmid of claim 1, wherein the DNA gene sequence encoding the eucaryotic polypeptide is DNA encoding human apolipoprotein E or an analog thereof inserted into the second restriction enzyme site.

30. A plasmid of claim 29, wherein the plasmid is pTV-188 having the restriction map shown in Fig. 18 and being 4.2 kb in length; pSAL 160-5 having the restriction map shown in Fig. 23 and being 5.2 kb in length; pTV-170 having the restriction map shown in Fig. 20 and being 4.4 kb in length; pTV-190 having the restriction map shown in Fig. 21 and being 4.4 kb in length; pTV-194 having the restriction map shown in Fig. 22 and being 4.5 kb in length; pTV-214 having the restriction map shown in Fig. 24 and being 4.4 kb in length; pTV 264-45 having the restriction map shown in Fig. 25 and being 4.4 kb in length; or pTVR 279-8 having the restriction map shown in Fig. 25 and deposited under ATCC Accession No. 53216.

31. A host plasmid system comprising the plasmid of claim 1 in a suitable Escherichia coli host.

32. A host plasmid system of claim 31, wherein the host is Escherichia coli strain A1637 (ATCC Accession No. 39385: containing plasmid pRec 2/3); Escherichia coli strain A1645 (ATCC Accession No. 39787: containing plasmid pApoE-Ex.2); and Escherichia coli strain A2097 (ATCC Accession No. 39786: containing plasmid pSOD$\alpha$2).

33. A host plasmid system of claim 31, wherein the host is a prototroph.

34. A host plasmid system of claim 33, wherein the host is Escherichia coli strain A4200 (ATCC Accession No. 53218: containing pHG44 and the F' gal plasmid and designated A4346).

35. A host plasmid system of claim 33, wherein the host is Escherichia coli strain A4255 (ATCC Accession No. 53215: containing p9200 and designated A4320).

36. A host plasmid system of claim 33, wherein the host is biotin independent.

37. A host plasmid system of claim 31, wherein the host is a suitable lytic strain.

38. A host plasmid system of claim 37, wherein the host is strain A4048 (ATCC Accession No. 53217: containing pHG44 and designated A3111).

39. A host plasmid system comprising the plasmid of claim 19, 21, 23, 25, 27, or 29 in a suitable Escherichia coli host.

40. A method for producing a polypeptide which comprises growing the host plasmid system of claim 31

73

under suitable conditions permitting production of the polypeptide and recovering the resulting polypeptide.

**41.** A method for producing bovine growth hormone or an analog thereof which comprises growing a suitable Escherichia coli host containing the plasmid of claim 19 under suitable conditions permitting production of bovine growth hormone or an analog thereof and recovering the resulting bovine growth hormone or analog thereof.

**42.** A method for producing human growth hormone or an analog thereof which comprises growing a suitable Escherichia coli host containing the plasmid of claim 21 under suitable conditions permitting production of human growth hormone or an analog thereof and recovering the resulting human growth hormone or analog thereof.

**43.** A method for producing porcine growth hormone or an analog thereof which comprises growing a suitable Escherichia coli host containing the plasmid of claim 25 under suitable conditions permitting production of porcine growth hormone or an analog thereof and recovering the resulting porcine growth hormone or analog thereof.

**44.** A method for producing chicken growth hormone or an analog thereof which comprises growing a suitable Escherichia coli host containing the plasmid of claim 27 under suitable conditions permitting production of chicken growth hormone or an analog thereof and recovering the resulting chicken growth hormone or analog thereof.

**45.** A method for producing superoxide dismutase or an analog thereof which comprises growing a suitable Escherichia coli host containing the plasmid of claim 23 under suitable conditions permitting production of superoxide dismutase or an analog thereof and recovering the resulting superoxide dismutase or analog thereof.

**46.** A method for producing human apolipoprotein E which comprises growing a suitable Escherichia coli host containing the plasmid of claim 29 under suitable conditions permitting production of human apolipoprotein E and recovering the resulting human apolipoprotein E.

**47.** A method of claim 40, wherein the suitable conditions comprise growth of the host plasmid system for an appropriate period of time at about 42°C, said growth being carried out on a suitable medium.

**48.** A method of claim 47, wherein the appropriate period of time at 42°C is about 1-5 hours.

**49.** A method of claim 47, wherein the suitable medium is casein hydrolysate.

**Claims for the following Contracting State : AT**

**1.** A method for effecting expression of a desired eucaryotic gene by introducing a plasmid into a suitable bacterial host cell containing the thermolabile repressor $C_1$, whereby the plasmid renders the host cell capable, upon increasing the temperature of the host cell to a temperature at which the repressor is inactivated, of effecting expression of the desired eucaryotic gene inserted into the plasmid and production of a eucaryotic polypeptide encoded by the gene comprising:

a double-stranded DNA molecule which includes in 5' to 3' order the following:

a DNA sequence which contains the promoter and operator $P_LO_L$ from λ bacteriophage;

an N utilization site for binding antiterminator N protein;

a first restriction enzyme site permitting replacement of the DNA sequence containing the ribosomal binding site which follows thereafter;

a ribosomal binding site for rendering the mRNA of the desired gene capable of binding to ribosomes within the host cell selected from the group consisting of a mutant of $C_{II}$ from λ bacteriophage having

the sequence

TAAGGAAGTACTTACAT
ATTCCTTCATGAATGTA;

a natural β-lactamase ribosomal binding site derived from pBR322 (ATCC Accession No. 37017);

a synthetic oligonucleotide having the sequence

AATTCGAGCGCAAGGAAACAGGCTCA
GCTCGCGTTCCTTTGTCCGAGTAT;

and a synthetic oligonucleotide having the sequence

AATTCAATAATATTGAAAAAGGAAGAG
GTTATTATAACTTTTTCCTTCTCAT;

an ATG initiation codon; and

a second restriction enzyme site having inserted therein the desired gene encoding the eucaryotic polypeptide or analog thereof in phase with the ATG initiation codon;

and which additionally includes a DNA sequence which contains an origin of replication from a bacterial plasmid capable of autonomous replication in the host cell and a selection means selected from the group consisting of DNA sequences which contain a gene associated with a selectable or identifiable phenotypic trait which is manifested when the vector is present in the host cell and DNA sequences which contain the fragment designated cI$^{434}$, such fragment including the gene for the cI$^{434}$ repressor protein and its associated promoter and operator sequence, and wherein the distance between the 3' end of the P$_L$O$_L$ promoter and operator sequence and the 5' end of the N utilization site is less than about 80 base pairs and the distance between the 3' end of the N utilization site and the 5' end of the ribosomal binding site is less than about 300 base pairs.

2. A method of claim 1, wherein the plasmid additionally comprises a DNA sequence which contains a T$_1$T$_2$ rRNA transcription termination sequence located 3' of the second restriction enzyme site.

3. A method of claim 1, wherein the selection means is a DNA sequence which contains a gene associated with a selectable or identifiable phenotypic trait.

4. A method of claim 3, wherein the phenotypic trait is drug resistance.

5. A method of claim 4, wherein the drug resistance is resistance to ampicillin or tetracycline.

6. A method of claim 2, wherein the T$_1$T$_2$ rRNA transcription termination sequence is less than about 100 base pairs from the 3' end of the second restriction enzyme site.

7. A method of claim 6, wherein the T$_1$T$_2$ rRNA transcription termination sequence is less than about 20 base pairs from the 3' end of the second restriction enzyme site.

8. A method of claim 1, wherein the selection means is a DNA sequence which contains the fragment

designated cl$^{4\,34}$.

9. A method of claim 8, wherein the cl$^{4\,34}$ fragment is located after the 3' end of the $T_1 T_2$ rRNA transcription termination sequence.

10. A method of claim 1, wherein the origin of replication is derived from pBR322 (ATCC Accession No. 37017).

11. A method of claim 1, wherein the origin of replication is from a bacterial plasmid capable of autonomous reproduction in the host cell and production of at least 400 constitutive copies of the plasmid.

12. A method of claim 11, wherein the origin of replication is derived from ColE1.

13. A method of claim 12, wherein the origin of replication is pOP1Δ6.

14. A method of claim 1, wherein the plasmid further comprises a third restriction enzyme site located between the DNA sequence containing the ribosomal binding site and the ATG initiation codon.

15. A method of claim 1, wherein the first restriction enzyme site is a unique site within the plasmid.

16. A method of claim 15, wherein the first unique restriction enzyme site is EcoRI.

17. A method of claim 1, wherein the second restriction enzyme site is a unique site within the plasmid.

18. A method of claim 17, wherein the second unique restriction enzyme site is NdeI, BglII or SmaI.

19. A method for production of bovine growth hormone or an analog thereof which comprises the plasmid of the method of claim 1, wherein the DNA gene sequence encoding the eucaryotic polypeptide is DNA encoding bovine growth hormone or an analog thereof, inserted into the second restriction enzyme site.

20. A method of claim 19, wherein the plasmid is pRO12 having the restriction map shown in Fig. 2 and being 3.3 kb in length; pSAL 5200-6 having the restriction map shown in Fig. 3 and being 3.3 kb in length; pHG44 having the restriction map shown in Fig. 6 and deposited under ATCC Accession No. 39806; pSAL 5600-1 having the restriction map shown in Fig. 10 and being 4.4 kb in length; p7200-22 having the restriction map shown in Fig. 7 and being 4.9 kb in length; pHG50 having the restriction map shown in Fig. 6 and deposited under ATCC Accession No. 39805; pSAL-210/4 having the restriction map shown in Fig. 9 and being 12.6 kb in length; p8300-10A having the restriction map shown in Fig. 7 and deposited under ATCC Accession No. 39785; pSAL-130/5 having the restriction map shown in Fig. 8 and being 9.6 kb in length; pSAL-170/10 having the restriction map shown in Fig. 8 and being 11.0 kb in length; or p9200 having the restriction map shown in Fig. 26 and deposited under ATCC Accession No. 53215.

21. A method for production of human growth hormone or an analog thereof which comprises the plasmid of the method of claim 1, wherein the DNA gene sequence encoding the eucaryotic polypeptide is DNA encoding human growth hormone or an analog thereof inserted into the second restriction enzyme site.

22. A method of claim 21, wherein the plasmid is pTV300, has the restriction map shown in Fig. 27, and is 4.3 kb in length.

23. A method for production of superoxide dismutase or an analog thereof which comprises the plasmid of the method of claim 1, wherein the DNA gene sequence encoding the eucaryotic polypeptide is DNA encoding superoxide dismutase or an analog thereof inserted into the second restriction enzyme site.

24. A method of claim 23, wherein the plasmid is pSODα2 having the restriction map shown in Fig. 13 and deposited under ATCC Accession No. 39786; pSODβ1 having the restriction map shown in Fig. 14 and being 3.0 kb in length; pSODβ$_1$T$_{11}$ having the restriction map shown in Fig. 15 and being 3.7 kb in length; pSODβ$_1$-BA2 having the restriction map shown in Fig. 17; or pSODβ$_1$TT-1 having the restriction map shown in Fig. 16 and being 4.8 kb in length.

**25.** A method for production of porcine growth hormone or an analog thereof which comprises the plasmid of the method of claim 1, wherein the DNA gene sequence encoding the eucaryotic polypeptide is DNA encoding porcine growth hormone or an analog thereof inserted into the second restriction enzyme site.

**26.** A method of claim 25, wherein the plasmid is p3008, has the restriction map shown in Fig. 4, and is deposited under ATCC Accession No. 39804, or the plasmid is p3009, has the restriction map shown in Fig. 11, and is 4.3 kb in length.

**27.** A method for production of chicken growth hormone or an analog thereof which comprises the plasmid of the method of claim 1, wherein the DNA gene sequence encoding the eucaryotic polypeptide is DNA encoding chicken growth hormone or an analog thereof inserted into the second restriction enzyme site.

**28.** A method of claim 27, wherein the plasmid is p5002, has the restriction map shown in Fig. 5, and is 3.3 kb in length, or the plasmid is p5003, has the restriction map shown in Fig. 12, and is deposited under ATCC Accession No. 39792.

**29.** A method for production of human apolipoprotein E or an analog thereof which comprises the plasmid of the method of claim 1, wherein the DNA gene sequence encoding the eucaryotic polypeptide is DNA encoding human apolipoprotein E or an analog thereof inserted into the second restriction enzyme site.

**30.** A method of claim 29, wherein the plasmid is pTV-188 having the restriction map shown in Fig. 18 and being 4.2 kb in length; pSAL 160-5 having the restriction map shown in Fig. 23 and being 5.2 kb in length; pTV-170 having the restriction map shown in Fig. 20 and being 4.4 kb in length; pTV-190 having the restriction map shown in Fig. 21 and being 4.4 kb in length; pTV-194 having the restriction map shown in Fig. 22 and being 4.5 kb in length; pTV-214 having the restriction map shown in Fig. 24 and being 4.4 kb in length; pTV 264-45 having the restriction map shown in Fig. 25 and being 4.4 kb in length; or pTVR 279-8 having the restriction map shown in Fig. 25 and deposited under ATCC Accession No. 53216.

**31.** A method of claim 1, wherein the plasmid is introduced into a suitable Escherichia coli host.

**32.** A method of claim 31, wherein the host is Escherichia coli strain A1637 (ATCC Accession No. 39385: containing plasmid pRec 2/3); Escherichia coli strain A1645 (ATCC Accession No. 39787: containing plasmid pApoE-Ex.2); and Escherichia coli strain A2097 (ATCC Accession No. 39786: containing plasmid pSODα2).

**33.** A method of claim 31, wherein the host is a prototroph.

**34.** A method of claim 33, wherein the host is Escherichia coli strain A4200 (ATCC Accession No. 53218: containing pHG44 and the F' gal plasmid and designated A4346).

**35.** A method of claim 33, wherein the host is Escherichia coli strain A4255 (ATCC Accession No. 53215: containing p9200 and designated A4320).

**36.** A method of claim 33, wherein the host is biotin independent.

**37.** A method of claim 31, wherein the host is a suitable lytic strain.

**38.** A method of claim 37, wherein the host is strain A4048 (ATCC Accession No. 53217: containing pHG44 and designated A3111).

**39.** A method of claim 19, 21, 23, 25, 27, or 29, wherein the plasmid is introduced into a suitable Escherichia coli host.

**40.** A method for producing a polypeptide which comprises growing the host plasmid system of claim 31

EP 0 173 280 B1

under suitable conditions permitting production of the polypeptide and recovering the resulting polypeptide.

41. A method for producing bovine growth hormone or an analog thereof which comprises growing a suitable Escherichia coli host containing the plasmid of the method of claim 19 under suitable conditions permitting production of bovine growth hormone or an analog thereof and recovering the resulting bovine growth hormone or analog thereof.

42. A method for producing human growth hormone or an analog thereof which comprises growing a suitable Escherichia coli host containing the plasmid of the method of claim 21 under suitable conditions permitting production of human growth hormone or an analog thereof and recovering the resulting human growth hormone or analog thereof.

43. A method for producing porcine growth hormone or an analog thereof which comprises growing a suitable Escherichia coli host containing the plasmid of the method of claim 25 under suitable conditions permitting production of porcine growth hormone or an analog thereof and recovering the resulting porcine growth hormone or analog thereof.

44. A method for producing chicken growth hormone or an analog thereof which comprises growing a suitable Escherichia coli host containing the plasmid of the method of claim 27 under suitable conditions permitting production of chicken growth hormone or an analog thereof and recovering the resulting chicken growth hormone or analog thereof.

45. A method for producing superoxide dismutase or an analog thereof which comprises growing a suitable Escherichia coli host containing the plasmid of the method of claim 23 under suitable conditions permitting production of superoxide dismutase or an analog thereof and recovering the resulting superoxide dismutase or analog thereof.

46. A method for producing human apolipoprotein E which comprises growing a suitable Escherichia coli host containing the plasmid of the method of claim 29 under suitable conditions permitting production of human apolipoprotein E and recovering the resulting human apolipoprotein E.

47. A method of claim 40, wherein the suitable conditions comprise growth of the host plasmid system for an appropriate period of time at about 42°C, said growth being carried out on a suitable medium.

48. A method of claim 47, wherein the appropriate period of time at 42°C is about 1-5 hours.

49. A method of claim 47, wherein the suitable medium is casein hydrolysate.

**Revendications**

**Revendications pour les Etats contractants suivants : BE, CH, DE, DK, ES, FR, GB, GR, IT, LI, LU, NL, SE**

1. Plasmide qui, lors de l'introduction dans une cellule hôte bactérienne appropriée contenant le répresseur thermolabile $C_I$, rend la cellule hôte capable, lors d'un accroissement de la température de la cellule hôte à une valeur à laquelle le répresseur est désactivé, d'effectuer l'expression d'un gène eucaryotique désiré introduit dans le plasmide et la production d'un polypeptide eucaryotique encodé par le gène, qui comprend :
    une molécule ADN bicaténaire qui comprend dans l'ordre 5' à 3' les structures suivantes :
    une séquence ADN qui contient le promoteur et l'opérateur $P_LO_L$ provenant d'un bactériophage $\lambda$ ;
    un site d'utilisation de N pour fixer la protéine antiterminatrice N ;
    un premier site enzymatique de restriction permettant le remplacement de la séquence ADN contenant le site de fixation ribosomique qui suit ;
    un site de fixation ribosomique pour rendre le mARN du gène désiré capable de se lier aux ribosomes dans la cellule hôte choisie dans le groupe comprenant:
    un mutant de $C_{II}$ à partir du bactériophage $\lambda$ ayant la séquence :

78

EP 0 173 280 B1

TAAGGAAGTACTTACAT

ATTCCTTCATGAATGTA ;

un site de liaison naturel ribosomique bêtalactamase dérivé de pBR322 (ATCC Accession n°37017) :

un oligonucléotide synthétique ayant la séquence :

AATTCGAGCGCAAGGAAACAGGCTCA

GCTCGCGTTCCTTTGTCCGAGTAT ;

et un oligonucléotide synthétique de séquence:

AATTCAATAATATTGAAAAAGGAAGAG

GTTATTATAACTTTTTCCTTCTCAT ;

un codon d'initiation ATG ; et

un second site d'enzyme de restriction dans lequel est inséré le gène désiré d'encodage du polypeptide eucaryotique ou d'un analogue de celui-ci en phase avec le codon d'initiation ATG ;

et comportant en outre une séquence ADN qui contient une origine de réplication provenant d'un plasmide bactérien capable d'une réplication autonome dans la cellule hôte et des moyens de sélection choisis parmi les séquences ADN qui contiennent un gène associé avec un trait phénotypique sélectionnable ou identifiable qui se manifeste en présence d'un vecteur dans la cellule hôte et les séquences ADN qui contiennent le fragment appelé $cI^{434}$, ce fragment comportant le gène de la protéine du répresseur $cI^{434}$ et sa séquence associée de promoteur et d'opérateur, et dans lequel la distance entre l'extrémité 3' de la séquence promoteur et opérateur $P_LO_L$ et l'extrémité 5' du site d'utilisation N est inférieure à environ 80 paires de base et la distance entre l'extrémité 3' du site d'utilisation N et l'extrémité 5' du site de liaison ribosomique est inférieure à environ 300 paires de base.

2. Plasmide selon la revendication 1, qui comprend en outre une séquence ADN contenant une séquence de terminaison de transcription $T_1T_2$ rARN disposée en 3' par rapport au site de la seconde enzyme de restriction.

3. Plasmide selon la revendication 1, dans lequel les moyens de sélection sont une séquence ADN qui contient un gène associé avec un trait phénotypique sélectionnable ou identifiable.

4. Plasmide selon la revendication 3, dans lequel le trait phénotypique est la résistance aux médicaments.

5. Plasmide selon la revendication 4, dans lequel la résistance au médicament est la résistance à l'ampicilline ou à la tetracycline.

6. Plasmide selon la revendication 2, dans lequel la séquence de terminaison de transcription $T_1T_2$ rARN est inférieure à environ 100 paires de base à partir de l'extrémité 3' du second site d'enzyme de restriction.

7. Plasmide selon la revendication 6, dans lequel la séquence de terminaison de transcription $T_1T_2$ rARN est inférieure à environ 20 paires de base à partir de l'extrémité 3' du second site d'enzyme de restriction.

8. Plasmide selon la revendication 1, dans lequel les moyens de sélection sont une séquence ADN qui contient le fragment appelé $cI^{434}$.

79

**9.** Plasmide selon la revendication 8, dans lequel le fragment cI$^{434}$ est disposé après l'extrémité 3' de la séquence de terminaison de transcription $T_1 T_2$ rARN.

**10.** Plasmide selon la revendication 1, dans lequel l'origine de réplication est dérivée de pBR322 (ATCC Accession n° 37017).

**11.** Plasmide selon la revendication 1, dans lequel l'origine de réplication est à partir d'un plasmide bactérien capable de reproduction autonome dans la cellule hôte et de production d'au moins 400 copies constitutives du plasmide.

**12.** Plasmide selon la revendication 11, dans lequel l'origine de réplication est dérivée de ColE1.

**13.** Plasmide selon la revendication 12, dans lequel l'origine de réplication est pOP1Δ6.

**14.** Plasmide selon la revendication 1, qui comprend en outre un troisième site d'enzyme de restriction entre la séquence ADN contenant le site de liaison ribosomique et le codon d'initiation ATG.

**15.** Plasmide selon la revendication 1, dans lequel le premier site d'enzyme de restriction est un site unique dans le plasmide.

**16.** Plasmide selon la revendication 15, dans lequel le premier site unique d'enzyme de restriction est EcoRI.

**17.** Plasmide selon la revendication 1, dans lequel le second site d'enzyme de restriction est un site unique dans le plasmide.

**18.** Plasmide selon la revendication 17, dans lequel le second site unique d'enzyme de restriction est NdeI, BglII ou SmaI.

**19.** Plasmide pour la production d'une hormone de croissance bovine ou d'un analogue de celle-ci, qui comprend le plasmide selon la revendication 1, dans lequel la séquence des gènes ADN encodant le polypeptide eucaryotique est ADN encodant l'hormone de croissance bovine ou un analogue de celle-ci, inséré dans le second site d'enzyme de restriction.

**20.** Plasmide selon la revendication 19, dans lequel le plasmide est pRO12 ayant le schéma de restriction indiqué sur la figure 2 et ayant 3,3 kb de longueur ; pSAL 5200-6 ayant le schéma de restriction indiqué sur la figure 3 ayant une longueur de 3,3 kb ; pHG44 ayant le schéma de restriction indiqué sur la figure 6 et déposé sous ATCC Accession N° 39806 ; pSAL 5600-1 ayant le schéma de restriction indiqué sur la figure 10 et ayant 4,4 kb de longueur ; p7200-22 ayant le schéma de restriction indiqué sur la figure 7 et ayant 4,9 kb de longueur ; pHG50 ayant le schéma de restriction représenté sur la figure 6 et déposé sous ATTC Accession N° 39805 ; pSAL-210/4 ayant le schéma de restriction montré sur la figure 9 et ayant une longueur de 12,6 kb ; P8300-10A ayant le schéma de restriction indiqué sur la figure 7 et déposé sous ATCC Accession n° 39785 ; pSAL-130/5 ayant le schéma de restriction représenté sur la figure 8 et ayant une longueur de 9,6 kb ; pSAL-170/10 ayant le schéma de restriction représenté sur la figure 8 et ayant 11,0 kb de longueur ; ou p9200 ayant le schéma de restriction indiqué sur la figure 26 et déposé sous ATCC Accession n° 53215.

**21.** Plasmide pour la production de l'hormone de croissance humaine ou d'un analogue de celle-ci, qui comprend le plasmide de la revendication 1, dans lequel la séquence des gènes ADN encodant le polypeptide eucaryotique est ADN encodant l'hormone de croissance humaine ou son analogue inséré dans le second site d'enzyme de restriction.

**22.** Plasmide selon la revendication 21, dans lequel le plasmide est pTV300, son schéma de restriction est représenté sur la figure 27 et sa longueur est de 4,3 kb.

**23.** Plasmide de production de superoxydedismutase ou d'un analogue de celle-ci, qui comprend le plasmide de la revendication 1, dans lequel la séquence des gènes ADN encodant le polypeptide eucaryotique est ADN encodant la superoxyde-dismutase ou un analogue de celle-ci introduit dans le second site d'enzyme de restriction.

**24.** Plasmide selon la revendication 23, dans lequel le plasmide est pSODα2 ayant le schéma de restriction indiqué sur la figure 13 et déposé sous ATCC Accession n° 39786 ; pSODβ1 ayant le schéma de restriction indiqué sur la figure 14 et ayant une longueur de 3,0 kb ; pSODβ$_1$T$_{11}$ ayant le schéma de restriction apparaissant sur la figure 15 et ayant 3,7 kb de longueur ; pSODβ$_1$-BA2 ayant le schéma de restriction indiqué sur la figure 17 ou pSOD β$_1$ TT-1 ayant le schéma de restriction indiqué sur la figure 16 et ayant une longueur de 4,8 kb.

**25.** Plasmide de production d'une hormone de croissance porcine ou d'un analogue de celle-ci, qui comprend le plasmide de la revendication 1, dont la séquence des gènes ADN encodant le polypeptide eucaryotique est ADN encodant l'hormone de croissance porcine ou un analogue de celle-ci inséré dans le second site d'enzyme de restriction.

**26.** Plasmide selon la revendication 25, dans lequel le plasmide est p-3008, son schéma de restriction est indiqué sur la figure 4 et il est déposé sous ATCC Accession n° 39804, ou bien le plasmide est p3009, son schéma de restriction apparaît sur la figure 11 et sa longueur est de 4,3 kb.

**27.** Plasmide de production d'hormone de croissance de poulet ou d'un analogue de celle-ci, qui comprend le plasmide de la revendication 1, dont la séquence de gènes ADN encodant le polypeptide eucaryotique est ADN encodant l'hormone de croissance de poulet ou un analogue de celle-ci, inséré dans le second site d'enzyme de restriction.

**28.** Plasmide selon la revendication 27, dans lequel le plasmide est p 5002, son schéma de restriction est représenté sur la figure 5 et sa longueur est de 3,3 kb, ou le plasmide est p5003, son schéma de restriction est représenté sur la figure 12 et il est déposé sous ATCC Accession n° 39792.

**29.** Procédé de production d'apolipoprotéine humaine E ou d'un analogue de celle-ci, qui comprend le plasmide de la revendication 1, dans lequel la séquence des gènes ADN encodant le polypeptide eucaryotique est ADN encodant l'apolipoprotéine humaine E ou un analogue de celle-ci inséré dans le second site d'enzyme de restriction.

**30.** Plasmide selon la revendication 29, dans lequel le plasmide est pTV-188 ayant le schéma de restriction indiqué sur la figure 18 et ayant 4,2 kb de longueur ; pSAL 160-5 ayant le schéma de restriction indiqué sur la figure 23 et ayant 5,2 kb de longueur ; pTV-170 ayant le schéma de restriction représenté sur la figure 20 et ayant une longueur de 4,4 kb ; pTV 190 ayant le schéma de restriction représenté sur la figure 21 et une longueur de 4,4 kb ; pTV-194 ayant le schéma de restriction représenté sur la figure 22 et 4,5 kb de longueur ; pTV-214 ayant le schéma de restriction indiqué sur la figure 24 et 4,4 kb de longueur ; pTV 264-45 ayant le schéma de restriction représenté sur la figure 25 et 4,4 kb de longueur ; ou pTVR 279-8 ayant le schéma de restriction représenté sur la figure 25 et déposé sous ATCC Accession n° 53216.

**31.** Système plasmide hôte comprenant le plasmide de la revendication 1 dans un hôte approprié Escherichia coli.

**32.** Système plasmide hôte selon la revendication 31, dans lequel l'hôte est la souche Escherichia coli A1637 (ATCC Accession n° 39385:contenant le plasmide pRec 2/3) ; la souche Escherichia coli A1645 (ATCC Accession N° 39787 : contenant le plasmide pApoE-Ex.2) ; et la souche Escherichia coli A2097 (ATCC Accession n° 39786:contenant le plasmide pSODalpha2).

**33.** Système plasmide hôte selon la revendication 31, dans lequel l'hôte est un prototrophe.

**34.** Système plasmide hôte selon la revendication 33, dans lequel l'hôte est la souche Escherichia coli A 4200 (ATCC Accession n° 53218:contenant pHG44 et le plasmide F'gal et désigné par A4346).

**35.** Système plasmide hôte selon la revendication 33, dans lequel l'hôte est la souche Escherichia coli A 4255 (ATCC Accession n° 53215 : contenant p9200 et désigné par A4320).

**36.** Système plasmide hôte selon la revendication 33, dans lequel l'hôte est indépendant de la biotine.

**37.** Système plasmide hôte selon la revendication 31, dans lequel l'hôte est une souche lytique convenable.

**38.** Système plasmide hôte selon la revendication 37, dans lequel l'hôte est la souche A4048 (ATCC Accession n° 53217 : contenant pHG44 et désigné par A3111).

**39.** Système plasmide hôte comprenant le plasmide selon la revendication 19, 21, 23, 25, 27 ou 29 dans un hôte Escherichia coli approprié.

**40.** Procédé de production d'un polypeptide qui consiste à faire croître le système plasmide hôte selon la revendication 31 dans des conditions convenables qui permettent la production d'un polypeptide et à récupérer le polypeptide résultant.

**41.** Procédé de production d'une hormone de croissance bovine ou d'un analogue de celle-ci, qui consiste à faire croître un hôte Escherichia coli approprié contenant le plasmide de la revendication 19 dans des conditions appropriées permettant la production de l'hormone de croissance bovine ou d'un anologue de celle-ci et à récupérer l'hormone de croissance résultante ou un analogue de celle-ci.

**42.** Procédé de production d'une hormone de croissance humaine ou d'un analogue de celle-ci, qui consiste à faire croître un hôte Escherichia coli convenable contenant le plasmide de la revendication 21 dans des conditions appropriées permettant la production d'une hormone de croissance humaine ou d'un analogue de celle-ci et à récupérer l'hormone de croissance humaine résultante ou l'analogue de celle-ci.

**43.** Procédé de préparation de l'hormone de croissance porcine ou d'un analogue de celle-ci, qui consiste à faire croître un hôte approprié Escherichia coli contenant le plasmide de la revendication 25 dans des conditions appropriées pour permettre la production d'hormone de croissance porcine ou d'un analogue de celle-ci et à récupérer l'hormone de croissance porcine résultante ou son analogue.

**44.** Procédé de production d'une hormone de croissance de poulet ou d'un analogue de celle-ci, qui consiste à faire croître un hôte approprié Escherichia coli contenant le plasmide de la revendication 27 dans des conditions convenables permettant la production d'hormone de croissance de poulet ou de son analogue et à récupérer l'hormone de croissance résultante ou son analogue.

**45.** Procédé de production de superoxyde-dismutase, ou un analogue de celle-ci, qui consiste à faire croître un hôte approprié Escherichia coli contenant le plasmide de la revendication 23 dans des conditions appropriées permettant la production de superoxyde-dismutase ou d'un analogue de celle-ci et à récupérer la superoxyde-dismutase résultante ou son analogue.

**46.** Procédé de production d'apolipoprotéine humaine E, qui consiste à faire croître un hôte Escherichia coli convenable contenant le plasmide de la revendication 29 dans des conditions appropriées permettant la production d'apolipoprotéine humaine E et à récupérer l'apolipoprotéine E résultante.

**47.** Procédé selon la revendication 40, dans lequel les conditions appropriées comprennent la croissance du système plasmide hôte pendant une période appropriée à une température d'environ 42°C, ladite croissance étant effectuée sur un milieu approprié.

**48.** Procédé selon la revendication 47, dans lequel la période appropriée à 42°C, est d'environ 1 à 5 heures.

**49.** Procédé selon la revendication 47, dans lequel le milieu approprié est l'hydrolysat de caséine.

**Revendications pour l'Etat contractant suivant : AT**

**1.** Procédé pour effectuer l'expression d'un gène eucaryotique désiré en introduisant un plasmide dans une cellule hôte bactérienne convenable contenant le répresseur thermolabile $C_I$, de sorte que le plasmide rend la cellule hôte capable, lors de l'accroissement de la température de la cellule hôte à une valeur à laquelle le répresseur est désactivé, d'effectuer l'expression du gène eucaryotique désiré inséré dans le plasmide et la production du polypeptide eucaryotique encodé par le gène, qui comprend :

une molécule ADN bicaténaire qui comprend dans l'ordre 5' à 3' les structures suivantes :

une séquence ADN qui contient le promoteur et l'opérateur $P_LO_L$ provenant d'un bactériophage ;

un site d'utilisation de N pour fixer la protéine antiterminatrice N ;

un premier site enzymatique de restriction permettant le remplacement de la séquence ADN contenant le site de fixation ribosomique qui suit ;

un site de fixation ribosomique pour rendre le mARN du gène désiré capable de se lier aux ribosomes dans la cellule hôte choisie dans le groupe comprenant: un mutant de $C_{II}$ à partir du bactériophage λ ayant la séquence :

$$TAAGGAAGTACTTACAT$$

$$ATTCCTTCATGAATGTA \;;$$

un site de liaison naturel ribosomique bêtalactamase dérivé de pBR322 (ATCC Accession N°37017) ;

un oligonucléotide synthétique ayant la séquence :

$$AATTCGAGCGCAAGGAAACAGGCTCA$$

$$GCTCGCGTTCCTTTGTCCGAGTAT \;;$$

et un oligonucléotide synthétique ayant la séquence :

$$AATTCAATAATATTGAAAAAGGAAGAG$$

$$GTTATTATAACTTTTTCCTTCTCAT \;;$$

un codon d'initiation ATG ; et

un second site d'enzyme de restriction dans lequel est inséré le gène désiré encodant le polypeptide eucaryotique ou un analogue de celui-ci en phase avec le codon d'initiation ATG ;

et qui comprend en outre une séquence ADN contenant une origine de réplication à partir d'un plasmide bactérien capable d'une réplication autonome dans la cellule hôte et des moyens de sélection choisis parmi le groupe consistant en séquences ADN qui contiennent un gène associé avec un trait phénotypique sélectionnable ou identifiable qui se manifeste quand le vecteur est présent dans la cellule hôte et les séquences ADN qui contiennent le fragment cI[4 34], un tel fragment comprenant le gène pour la protéine de répresseur cI[4 34] et sa séquence promoteur et opérateur associée, et dans lequel la distance entre l'extrémité 3' de la séquence promoteur et opérateur $P_LO_L$ et l'extrémité 5' du site d'utilisation N est inférieure à environ 80 paires de base et la distance entre l'extrémité 3' du site d'utilisation N et l'extrémité 5' du site de liaison ribosomique est inférieure à environ 300 paires de base.

2. Procédé selon la revendication 1, dans lequel le plasmide comprend en outre une séquence ADN qui contient une séquence de terminaison de transcription $T_1 T_2$ rARN disposée en position 3' du second site d'enzyme de restriction.

3. Procédé selon la revendication 1, dans lequel les moyens de sélection sont une séquence ADN contenant un gène associé avec un trait phénotypique sélectionnable ou identifiable.

**4.** Procédé selon la revendication 3, dans lequel le trait phénotypique est la résistance aux médicaments.

**5.** Procédé selon la revendication 4, dans lequel la résistance aux médicaments est la résistance à l'ampicilline ou la tetracycline.

**6.** Procédé selon la revendication 2, dans lequel la séquence de terminaison de transcription $T_1 T_2$ rARN est inférieure à environ 100 paires de base à partir de l'extrémité 3' du second site d'enzyme de restriction.

**7.** Procédé selon la revendication 6, dans lequel la séquence de terminaison de transcription $T_1 T_2$ rARN est inférieure à environ 20 paires de base à partir de l'extrémité 3' du second site d'enzyme de restriction.

**8.** Procédé selon la revendication 1, dans lequel les moyens de sélection sont une séquence ADN qui contient le fragment désigné $cI^{434}$.

**9.** Procédé selon la revendication 8, dans lequel le fragment $cI^{434}$ est disposé après l'extrémité 3' de la séquence de terminaison de transcription $T_1 T_2$ rARN.

**10.** Procédé selon la revendication 1, dans lequel l'origine de réplication est dérivée de pBR322 (ATCC Accession N° 37017).

**11.** Procédé selon la revendication 1, dans lequel l'origine de réplication provient d'un plasmide bactérien capable de reproduction autonome dans la cellule hôte et de production d'au moins 400 copies constitutives du plasmide.

**12.** Procédé selon la revendication 11, dans lequel l'origine de réplication est dérivée de ColE1.

**13.** Procédé selon la revendication 12, dans lequel l'origine de réplication est pOP1Δ6.

**14.** Procédé selon la revendication 1, dans lequel le plasmide comprend en outre un troisième site d'enzyme de restriction entre la séquence ADN contenant la liaison ribosomique et le codon d'initiation ATG.

**15.** Procédé selon la revendication 1, dans lequel le premier site d'enzyme de restriction est un site unique dans le plasmide.

**16.** Procédé selon la revendication 15, dans lequel le premier site unique d'enzyme de restriction est ECORI.

**17.** Procédé selon la revendication 1, dans lequel le second site d'enzyme de restriction est un site unique dans le plasmide.

**18.** Procédé selon la revendication 17, dans lequel le second site unique d'enzyme de restriction est NdeI, BglII ou SmaI.

**19.** Procédé de production d'une hormone de croissance bovine ou d'un analogue de celle-ci, qui comprend le plasmide selon le procédé de la revendication 1, dans lequel la séquence de gènes ADN encodant le polypeptide eucaryotique est ADN encodant l'hormone de croissance bovine ou un analogue de celle-ci inséré dans le second site d'enzyme de restriction.

**20.** Procédé selon la revendication 19, dans lequel le plasmide est pRO12 présentant le schéma de restriction indiqué sur la figure 2 et ayant 3,3 kb de longueur ; pSAL 5200-6 ayant le schéma de restriction indiqué sur la figure 3, ayant une longueur de 3,3 kb ; pHG44 ayant le schéma de restriction indiqué sur la figure 6 et déposé sous ATCC Accession N° 39806 ; pSAL 5600-1 ayant le schéma de restriction indiqué sur la figure 10 et ayant 4,4 kb de longueur ; p7200-22 ayant le schéma de restriction représenté sur la figure 7 et ayant 4,9 kb de longueur ; PHG50 ayant le schéma de restriction représenté sur la figure 6 et déposé sous ATCC Accession n° 39805 ; pSAL-210/4 ayant le schéma de restriction représenté sur la figure 9 et ayant 12,6 kb de longueur ; P8300-10A ayant le schéma de restriction représenté sur la figure 7 et déposé sous ATCC Accession n° 39785 ; pSAL-130/5 ayant le schéma de restriction représenté sur la figure 8 et ayant 9,6 kg de longueur ; pSAL-170/10 ayant le schéma de restriction indiqué sur la figure 8 et ayant 11,0 kb de longueur ; ou p9200 ayant le schéma de restriction représenté sur la figure 26 et déposé sous ATCC Accession n° 53215.

21. Procédé de production d'une hormone de croissance humaine ou d'un analogue de celle-ci qui comprend le plasmide du procédé de la revendication 1, dans lequel la séquence de gènes ADN encodant le polypeptide eucaryotique est ADN encodant l'hormone de croissance humaine ou un analogue de celle-ci inséré dans le second site d'enzyme de restriction.

22. Procédé selon la revendication 21, dans lequel le plasmide est pTV300, son schéma de restriction est indiqué sur la figure 27 et sa longueur est de 4,3 kb.

23. Procédé de production d'une superoxyde-dismutase ou d'un analogue de celle-ci, qui comprend le plasmide du procédé de la revendication 1, dans lequel la séquence de gènes ADN encodant le polypeptide eucaryotique est ADN encodant la superoxyde-dismutase ou un analogue de celle-ci inséré dans le second site d'enzyme de restriction.

24. Procédé selon la revendication 23, dans lequel le plasmide est pSODalpha2 ayant le schéma de restriction indiqué sur la figure 13 et déposé sous ATCC Accession n° 39786 ; pSODbêta1 ayant le schéma de restriction indiqué sur la figure 14 et ayant une longueur de 3,0 kb ; pSODbêta$_1$ T$_{11}$ ayant le schéma de restriction indiqué sur la figure 15 et ayant 3,7 kb de longueur ; pSODbêta$_1$-BA2 ayant le schéma de restriction indiqué sur la figure 17 ; ou pSObêta$_1$ TT-1 ayant le schéma de restriction représenté sur la figure 16 et une longueur de 4,8 kb.

25. Procédé de production d'une hormone de croissance porcine ou d'un analogue de celle-ci, qui comprend le plasmide du procédé de la revendication 1, dans lequel la séquence des gènes ADN encodant le polypeptide eucaryotique est ADN encodant l'hormone de croissance porcine ou un analogue de celle-ci inséré dans le second site d'enzyme de restriction.

26. Procédé selon la revendication 25, dans lequel le plasmide est p 3008, son schéma de restriction étant représenté sur la figure 4 et il est déposé sous ATCC Acession n° 39804 ou bien le plasmide est p3009, son schéma de restriction est indiqué sur la figure 11 et sa longueur est de 4,3 kb.

27. Procédé de production d'une hormone de croissance de poulet ou d'un analogue de celle-ci qui comprend le plasmide du procédé de la revendication 1, dans lequel la séquence de gènes ADN encodant le polypeptide eucaryotique est ADN encodant l'hormone de croissance de poulet ou un analogue de celle-ci inséré dans le second site d'enzyme de restriction.

28. Procédé selon la revendication 27, dans lequel le plasmide est p5002, son schéma de restriction étant celui de la figure 5 et sa longueur de 3,3 kb, ou bien le plasmide est p5003, son schéma de restriction étant représenté sur la figure 12 et il est déposé sous ATCC Accession N° 39792.

29. Procédé de production d'apolipoprotéine humaine E ou d'un analogue de celle-ci, qui comprend le plasmide du procédé de la revendication 1, dans lequel la séquence des gènes ADN encodant le polypeptide eucaryotique est ADN encodant l'apolipoprotéine humaine E ou un analogue de celle-ci, inséré dans le second site d'enzyme de restriction.

30. Procédé selon la revendication 29, dans lequel le plasmide est pTV-188 ayant le schéma de restriction indiqué sur la figure 18 et ayant 4,2 kb de longueur ; pSAL 160-5 ayant le schéma de restriction indiqué sur la figure 23 et ayant 5,2 kb de longueur ; pTV-170 ayant le schéma de restriction représenté sur la figure 20 et ayant 4,4 kb de longueur ; pTV-190 ayant le schéma de restriction représenté sur la figure 21 et ayant 4,4 kb de longueur ; pTV-194 ayant le schéma de restriction indiqué sur la figure 22 et ayant 4,5 kb de longueur ; pTV-214 ayant le schéma de restriction indiqué sur la figure 24 et ayant 4,4 kb de longueur ; pTV 264-45 ayant le schéma de restriction de la figure 25 et ayant 4,4 kb de longueur ; ou pTVR 279-8 ayant le schéma de restriction indiqué sur la figure 25 et déposé sous ATCC Accession N° 53216.

**31.** Procédé selon la revendication 1, dans lequel on introduit le plasmide dans un hôte Escherichia coli approprié.

**32.** Procédé selon la revendication 31, dans lequel l'hôte est la souche Escherichia coli A1637 (ATCC Accession N°39385 : contenant le plasmide pRec 2/3) ; la souche Escherichia coli A1645 (ATCC Accession N°39787: contenant le plasmide pApoE-Ex.2) ; et la souche Escherichia coli A2097 (ATCC Accession n°39786 : contenant le plasmide pSODalpha2).

**33.** Procédé selon la revendication 31, dans lequel l'hôte est un prototrophe.

**34.** Procédé selon la revendication 33, dans lequel l'hôte est la souche Escherichia coli A4200 (ATCC Accession N°53218 : contenant pHG44 et le plasmide F'gal et désigné par A4346).

**35.** Procédé selon la revendication 33, dans lequel l'hôte est la souche Escherichia coli A4255 (ATCC Accession n°53215 : contenant p9200 et désigné A4320).

**36.** Procédé selon la revendication 33, dans lequel l'hôte est indépendant de la biotine.

**37.** Procédé selon la revendication 31, dans lequel l'hôte est une souche lytique convenable.

**38.** Procédé selon la revendication 37, dans lequel l'hôte est la souche A4048 (ATCC Accession n°53217: contenant pHG44 et désigné par A3111).

**39.** Procédé selon la revendication 19, 21, 23, 25, 27 ou 29, dans lequel le plasmide est introduit dans un hôte Escherichia coli convenable.

**40.** Procédé de production d'un polypeptide qui consiste à faire croître le système de plasmide hôte de la revendication 31 dans des conditions appropriées permettant la production du polypeptide et à récupérer le polypeptide résultant.

**41.** Procédé de production d'une hormone de croissance bovine ou d'un analogue de celle-ci, qui consiste à faire croître un hôte Escherichia coli convenable contenant le plasmide du procédé de la revendication 19 dans des conditions appropriées permettant la production de l'hormone de croissance bovine ou d'un analogue de celle-ci et à récupérer l'hormone résultante de croissance bovine ou son analogue.

**42.** Procédé de production d'hormone de croissance humaine ou d'un analogue de celle-ci, qui consiste à faire croître un hôte Escherichia coli convenable contenant le plasmide du procédé de la revendication 21, dans des conditions appropriées permettant la production de l'hormone de croissance humaine ou d'un analogue de celle-ci et à récupérer l'hormone de croissance humaine ou son analogue.

**43.** Procédé de production d'hormone de croissance porcine ou d'un analogue de celle-ci, qui consiste à faire croître un hôte Escherichia coli convenable contenant le plasmide du procédé de la revendication 25 dans des conditions appropriées permettant la production d'une hormone de croissance porcine ou d'un analogue de celle-ci et à récupérer l'hormone de croissance porcine ou son analogue.

**44.** Procédé de production d'une hormone de croissance de poulet ou d'un analogue de celle-ci, qui consiste à faire croître un hôte convenable Escherichia coli contenant le plasmide du procédé de la revendication 27 dans des conditions appropriées permettant la production de l'hormone de croissance de poulet ou de son analogue et à récupérer l'hormone de croissance de poulet résultante ou un analogue de celle-ci.

**45.** Procédé de production de superoxyde-dismutase ou d'un analogue de celle-ci, qui consiste à faire croître un hôte approprié Escherichia coli contenant le plasmide du procédé de la revendication 23, dans des conditions convenables permettant la production de superoxyde dismutase ou d'un analogue de celle-ci et à récupérer la superoxyde-dismutase résultante ou son analogue.

**46.** Procédé de production d'apolipoprotéine humaine E, qui consiste à faire croître un hôte approprié Escherichia coli contenant le plasmide du procédé de la revendication 29 dans des conditions convenables permettant la production d'apolipoprotéine humaine E et à récupérer l'apolipoprotéine humaine E résultante.

47. Procédé selon la revendication 40, dans lequel les conditions appropriées comprennent la croissance du système plasmide hôte pendant une période appropriée à environ 42°C, ladite croissance étant effectuée sur un milieu convenable.

48. Procédé selon la revendication 47, dans lequel la période appropriée à 42°C est d'environ 1 à 5 heures.

49. Procédé selon la revendication 47, dans lequel le milieu convenable est un hydrolysat de caséine.

**Patentansprüche**

**Patentansprüche für folgende Vertragsstaaten : BE, CH, DE, FR, GB, IT, LI, LU, NL, SE**

1. Plasmid, das nach Einschleusung in eine geeignete Bakterienwirtszelle mit dem thermolabilen Repressor $C_I$ die Wirtszelle bei Erhöhung der Wirtszellentemperatur auf eine Temperatur, bei der der Repressor inaktiviert wird, befähigt, ein in das Plasmid eingefügtes gewünschten eucaryotisches Gen zu exprimieren und ein durch das Gen codiertes eucaryotisches Polypeptin zu bilden, umfassend

ein doppelsträngiges DNA-Molekül mit in 5'- nach 3'-Richtung

eine DNA-Sequenz, die den Promoter und Operator $P_L O_L$ eines λ-Bakteriophagen enthält,

eine N-Erkennungsstelle zum Binden von Antiterminator N-Protein,

eine einen Austausch der DNA-Sequenz gestattenden ersten Restriktionsenzymstelle mit der folgenden ribosomalen Bindungsstelle,

eine ribosomalen Bindungsstelle, die die mRNA des gewünschten Gens zur Bindung von Ribosomen innerhalb der Wirtszelle befähigt, ausgewählt aus der Gruppe:

eine Mutante von $C_{II}$ aus einem λ-Bakteriophagen mit der Sequenz:

TAAGGAAGTACTTACAT

ATTCCTTCATGAATGTA;

eine von pBR322 (ATCC Hinterlegungs-Nr. 37017) herrührende natürliche $\beta$-Lactamase-Ribosombindungsstelle;

ein synthetisches Oligonucleotid der Sequenz:

AATTCGAGCGCAAGGAAACAGGCTCA

GCTCGCGTTCCTTTGTCCGAGTAT;

und ein synthetisches Oligonucleotid der Sequenz:

AATTCAATAATATTGAAAAAGGAAGAG

GTTATTATAACTTTTTCCTTCTCAT;

ein ATG-Initiationscodon und

eine zweiten Restriktionsenzymstelle mit dem darin eingefügten gewünschten Gen mit Codierung für eucaryotisches Polypeptid oder dessen Analoges in Phase mit dem ATG-Initiationscodon,

das zusätzlich eine DNA-Sequenz mit einem Replikationsursprung aus einem bakteriellen Plasmid mit der Fähigkeit zur autonomen Replikation in der Wirtszelle und einem Selektionsmittel, ausgewählt aus der Gruppe DNA-Sequenzen, die ein Gen mit assoziierter selektierbarer oder identifizierbarer phenotypischer und sich bei Anwesenheit des Vektors in der Wirtszelle manifestierender Eigenschaft enthalten, und DNA-Sequenzen, die das Fragment mit der Bezeichnung $cI^{434}$ mit dem Gen für das $cI^{434}$-Repressorprotein und seine assoziierte Promotor- und Operatorsequenz enthalten,

aufweist, wobei der Abstand zwischen dem 3'-Ende der $P_L O_L$-Promotor- und -Operatorsequenz und dem 5'-Ende der N-Erkennungsstelle weniger als etwa 80 Basenpaare und der Abstand zwischen dem 3'-Ende der N-Erkennungsstelle und dem 5'-Ende der ribosomalen Bindungsstelle weniger als etwa 300 Basenpaare betragen.

**2.** Plasmid nach Anspruch 1, welches zusätzlich eine DNA-Sequenz mit einer $T_1 T_2$ rRNA-Transkriptionsterminationssequenz 3' der zweiten Restriktionsenzymstelle enthält.

**3.** Plasmid nach Anspruch 1, wobei das Selektionsmittel aus einer DNA-Sequenz mit einem Gen in Verbindung mit einer selektierbaren oder identifizierbaren phenotypischen Eigenschaft besteht.

**4.** Plasmid nach Anspruch 3, dadurch gekennzeichnet, daß die phenotypische Eigenschaft Arzneimittelresistenz ist.

**5.** Plasmid nach Anspruch 4, dadurch gekennzeichnet, daß die Arzneimittelresistenz eine Resistenz gegen Ampicillin oder Tetracyclin ist.

**6.** Plasmid nach Anspruch 2, dadurch gekennzeichnet, daß die $T_1 T_2$ rRNA-Transkriptionsterminationssequenz weniger als etwa 100 Basenpaare vom 3'-Ende der zweiten Restriktionsenzymstelle (entfernt) ist.

**7.** Plasmid nach Anspruch 6, dadurch gekennzeichnet, daß die $T_1 T_2$ rRNA-Transkriptionsterminationsequenz weniger als etwa 20 Basenpaare vom 3'-Ende der zweiten Restriktionsenzymstelle (entfernt) ist.

**8.** Plasmid nach Anspruch 1, dadurch gekennzeichnet, daß das Selektionsmittel aus einer DNA-Sequenz mit dem mit $cI^{434}$ bezeichneten Fragment besteht.

**9.** Plasmid nach Anspruch 8, dadurch gekennzeichnet, daß sich das $cI^{434}$-Fragment nach dem 3'-Ende der $T_1 T_2$ rRNA-Transkriptionsterminationsequenz befindet.

**10.** Plasmid nach Anspruch 1, dadurch gekennzeichnet, daß der Replikationsursprung von pBR322 (ATCC Hinterlegungs-Nr. 37017) herrührt.

**11.** Plasmid nach Anspruch 1, dadurch gekennzeichnet, daß der Replikationsursprung von einem bakteriellen Plasmid mit der Fähigkeit zur autonomen Reproduktion in der Wirtszelle und zur Produktion von mindestens 400 grundlegenden Kopien des Plasmids herrührt.

**12.** Plasmid nach Anspruch 11, dadurch gekennzeichnet, daß der Replikationsursprung von ColEl herrührt.

**13.** Plasmid nach Anspruch 12, dadurch gekennzeichnet, daß der Replikationsursprung pOP1Δ6 ist.

**14.** Plasmid nach Anspruch 1, zusätzlich enthaltend eine dritte Restriktionsenzymstelle, die zwischen der DNA-Sequenz mit der ribosomalen Bindungsstelle und dem ATG-Initiationscodon liegt.

**15.** Plasmid nach Anspruch 1, dadurch gekennzeichnet, daß es sich bei der ersten Restriktionsenzymstelle um eine einzige Stelle innerhalb des Plasmids handelt.

**16.** Plasmid nach Anspruch 15, dadurch gekennzeichnet, daß die erste einzige Restriktionsenzymstelle EcoRI ist.

17. Plasmid nach Anspruch 1, dadurch gekennzeichnet, daß es sich bei der Zweiten Restriktionsenzymstelle um eine einzige Stelle innerhalb des Plasmids handelt.

18. Plasmid nach Anspruch 17, dadurch gekennzeichnet, daß die zweite einzige Restriktionsenzymstelle NdeI, BglIII oder SmaI ist.

19. Plasmid zur Herstellung von Rinderwachstumshormon oder eines Analogen desselben, enthaltend das Plasmid nach Anspruch 1, wobei es sich bei der DNA-Gensequenz mit Codierung für das eucaryotische Polypeptid um eine in die zweite Restriktionsenzymstelle eingefügte DNA mit Codierung für Rinderwachstumshormon oder ein Analoges desselben handelt.

20. Plasmid nach Anspruch 19, nämlich pRO12 mit der in Fig. 2 dargestellten Restriktionskarte und einer Länge von 3.3 kb, pSAL 5200-6 mit der in Fig. 3 dargestellten Restriktionskarte und einer Länge von 3.3 kb, pHG44 mit der in Fig. 6 dargestellten Restriktionskarte und der Hinterlegungs-Nr. ATCC 39806, pSAL 5600-1 mit der in Fig. 10 dargestellten Restriktionskarte und einer Länge von 4.4 kb, p7200-22 mit der in Fig. 7 dargestellten Restriktionskarte und einer Länge von 4.9 kb, pHG50 mit der in Fig. 6 dargestellten Restriktionskarte und der Hinterlegungs-Nr. ATCC 39805, pSAL-210/4 mit der in Fig. 9 dargestellten Restriktionskarte und einer Länge von 12.6 kb, p8300-10A mit der in Fig. 7 dargestellten Restriktionskarte und der Hinterlegungs-Nr. ATCC 39785, pSAL-130/5 mit der in Fig.8 dargestellten Restriktionskarte und einer Länge von 9.6 kb, pSAL-170/10 mit der in Fig. 8 dargestellten Restriktionskarte und einer Länge von 11.0 kb oder p9200 mit der in Fig. 26 dargestellten Restriktionskarte und der Hinterlegungs-Nr. ATCC 53215.

21. Plasmid zur Gewinnung von menschlichem Wachstumshormon oder eines Analogen desselben mit dem Plasmid nach Anspruch 1, wobei die DNA-Gensequenz mit der Codierung für das eucaryotische Polypeptid aus einer in die zweite Restriktionsenzymstelle eingefügten DNA mit Codierung für menschliches Wachstumshormon oder ein Analoges desselben besteht.

22. Plasmid nach Anspruch 21, nämlich pTV300 mit der in Fig. 27 dargestellten Restriktionskarte und einer Länge von 4.3 kb.

23. Verfahren zur Gewinnung einer Superoxiddismutase oder eines Analogen derselben mit dem Plasmid nach Anspruch 1, wobei die DNA-Gensequenz mit der Codierung für das eucaryotische Polypeptid aus einer in die zweite Restriktionsenzymstelle eingefügten DNA mit Codierung für Superoxiddismutase oder ein Analoges derselben besteht.

24. Plasmid nach Anspruch 23, nämlich pSODα2 mit der in Fig. 13 dargestellten Restriktionskarte und der Hinterlegungs-Nr. ATCC 39786, pSODß1 mit der in Fig. 14 dargestellten Restriktionskarte und einer Länge von 3.0 kb, pSOD$\beta_1$T$_{11}$ mit der in Fig. 15 dargestellten Restriktionskarte und einer Länge von 3.7 kb, pSOD$\beta_1$-BA2 mit der in Fig. 17 dargestellten Restriktionskarte oder pSOD$\beta_1$TT-1 mit der in Fig. 16 dargestellten Restriktionskarte und einer Länge von 4.8 kb.

25. Plasmid zur Gewinnung von Schweinewachstumshormon oder eines Analogen desselben mit dem Plasmid nach Anspruch 1, wobei die DNA-Gensequenz mit Codierung für das eucaryotische Polypeptid aus einer in die zweite Restriktionsenzymstelle eingefügten DNA mit Codierung für Schweinewachstumshormon oder ein Analoges desselben besteht.

26. Plasmid nach Anspruch 25, nämlich p3008 mit der in Fig. 4 dargestellten Restriktionskarte und der Hinterlegungs-Nr. ATCC 39804 oder p3009 mit der in Fig. 11 dargestellten Restriktionskarte und einer Länge von 4.3 kb.

27. Plasmid zur Gewinnung eines Hühnerwachstumshormons oder eines Analogen desselben mit dem Plasmid nach Anspruch 1, wobei die DNA-Gensequenz mit Codierung für das eurocaryotische Polypeptid aus einer in die zweite Restriktionsenzymstelle eingefügten DNA mit Codierung für Hühnerwachstumshormon oder ein Analoges desselben besteht.

**28.** Plasmid nach Anspruch 27, nämlich p5002 mit der in Fig. 5 dargestellten Restriktionskarte und einer Länge von 3.3 kb oder p5003 mit der in Fig. 12 dargestellten Restriktionskarte und der Hinterlegungs-Nr. ATCC 39792.

**29.** Plasmid zur Gewinnung von Humanapolipoprotein E oder eines Analogen desselben mit dem Plasmid nach Anspruch 1, wobei die DNA-Gensequenz mit Codierung für das eucaryotische Polypeptid aus einer in die zweite Restriktionsenzymstelle eingefügten DNA mit Codierung für Humanapolipoprotein E oder eines Analogen desselben besteht.

**30.** Plasmid nach Anspruch 29, nämlich pTV-188 mit der in Fig. 18 dargestellten Restriktionskarte und einer Länge von 4.2 kb, pSAL 160-5 mit der in Fig. 23 dargestellten Restriktionskarte und einer Länge von 5.2 kb, pTV-170 mit der in Fig. 20 dargestellten Restriktionskarte und einer Länge von 4.4 kb, pTV-190 mit der in Fig. 21 dargestellten Restriktionskarte und einer Länge von 4.4 kb, pTV-194 mit der in Fig. 22 dargestellten Restriktionskarte und einer Länge von 4.5 kb, pTV-214 mit der in Fig. 24 dargestellten Restriktionskarte und einer Länge von 4.4 kb, pTV-264-45 mit der in Fig. 25 dargestellten Restriktionskarte und einer Länge von 4.4 kb oder pTVR-279-8 mit der in Fig. 25 dargestellten Restriktionskarte und der Hinterlegungs-Nr. ATCC 53216.

**31.** Wirt/Plasmid-System mit dem Plasmid von Anspruch 1 in einem geeigneten Escherichia coli-Wirt.

**32.** Wirt/Plasmid-System nach Anspruch 31, dadurch gekennzeichnet, daß der Wirt aus dem Escherichia coli-Stamm A1637 (ATCC Hinterlegungs-Nr. 39385 mit dem Plasmid pRec 2/3), dem Escherichia coli-Stamm A1645 (ATCC Hinterlegungs-Nr. 39787 mit dem Plasmid pApoE-Ex.2) oder dem Escherichia coli-Stamm A2097 (ATCC Hinterlegungs-Nr. 39786 mit dem Plasmid pSODα2) besteht.

**33.** Wirt/Plasmid-System nach Anspruch 31, dadurch gekennzeichnet, das der Wirt aus einem Prototroph besteht.

**34.** Wirt/Plasmid-System nach Anspruch 33, dadurch gekennzeichnet, daß der Wirt aus dem Escherichia coli-Stamm A4200 (ATCC Hinterlegungs-Nr. 53218 mit pHG44 und dem F'-gal Plasmid und der Bezeichnung A 4346) besteht.

**35.** Wirt/Plasmid-System nach Anspruch 33, dadurch gekennzeichnet, daß der Wirt aus dem Escherichia coli-Stamm A4255 (ATCC Hinterlegungs-Nr. 53215 mit p9200 und mit der Bezeichnung A4320) besteht.

**36.** Wirt/Plasmid-System nach Anspruch 33, dadurch gekennzeichnet, daß der Wirt biotinunabhängig ist.

**37.** Wirt/Plasmid-System nach Anspruch 31, dadurch gekennzeichnet, daß der Wirt aus einem geeigneten lytischen Stamm besteht.

**38.** Wirt/Plasmid-System nach Anspruch 37, dadurch gekennzeichnet, daß der Wirt aus dem Stamm A4048 (ATCC Hinterlegungs-Nr. 53217 mit pHG44 und der Bezeichnung A3111) besteht.

**39.** Wirt/Plasmid-System mit dem Plasmid nach Ansprüchen 19, 21, 23, 25, 27 oder 29 in einem geeigneten Escherichia coli-Wirt.

**40.** Verfahren zur Herstellung eines Polypeptids durch Züchten des Wirt/Plasmid-Systems nach Anspruch 31 unter geeigneten Bedingungen, die die Produktion des Polypeptids und die Gewinnung des erhaltenen Polypeptids gestatten.

**41.** Verfahren zur Gewinnung von Rinderwachstumshormon oder eines Analogen desselben durch Züchten eines geeigneten Escherichia coli-Wirts mit dem Plasmid nach Anspruch 19 unter geeigneten Bedingungen, die die Produktion von Rinderwachstumshormon oder eines Analogen desselben und die Gewinnung des erhaltenen Rinderwachstumshormons oder Analogen desselben gestatten.

**42.** Verfahren zur Gewinnung von menschlichem Wachstumshormon oder eines Analogen desselben durch Züchten eines geeigneten Escherichia coli-Wirts mit dem Plasmid nach Anspruch 21 unter geeigneten Bedingungen, die die Produktion von menschlichem Wachstumshormon oder eines Analogen desselben und die Gewinnung des erhaltenen menschlichen Wachstumshormons oder Analogen desselben gestatten.

**43.** Verfahren zur Gewinnung von Schweinewachstumshormon oder eines Analogen desselben durch Züchten eines geeigneten Escherichia coli-Wirts mit dem Plasmid nach Anspruch 25 unter geeigneten Bedingungen, die die Produktion von Schweinewachstumhormon oder eines Analogen desselben und die Gewinnung des erhaltenen Schweinewachstumshormons oder Analogen desselben gestatten.

**44.** Verfahren zur Gewinnung von Hühnerwachstumshormon oder eines Analogen desselben durch Züchten eines geeigneten Escherichia coli-Wirts mit dem Plasmid nach Anspruch 27 unter geeigneten Bedingungen, die die Produktion von Hühnerwachstumshormon oder eines Analogen desselben und die Gewinnung des erhaltenen Hühnerwachstumshormons oder Analogen desselben gestatten.

**45.** Verfahren zur Gewinnung von Superoxiddismutase oder eines Analogen derselben durch Züchten eines geeigneten Escherichia coli-Wirts mit dem Plasmid nach Anspruch 23 unter geeigneten Bedingungen, die die Produktion von Superoxiddismutase oder eines Analogen derselben und die Gewinnung der erhaltenen Superoxiddismutase oder eines Analogen derselben gestatten.

**46.** Verfahren zur Gewinnung von Humanapolipoprotein E durch Züchten eines geeigneten Escherichia coli-Wirts mit dem Plasmid nach Anspruch 29 unter geeigneten Bedingungen, die die Produktion von Humanapolipoprotein E und die Gewinnung des gebildeten Humanapolipoprotein E gestatten.

**47.** Verfahren nach Anspruch 40, wobei unter "geeigneten Bedingungen" die Züchtung des Wirt/Plasmid-Systems während einer geeigneten Zeitdauer bei etwa 42°C auf einem geeigneten Medium zu verstehen ist.

**48.** Verfahren nach Anspruch 47, wobei unter der "geeigneten Zeitdauer" bei 42°C etwa 1-5 h zu verstehen ist (sind).

**49.** Verfahren nach Anspruch 47, dadurch gekennzeichnet, daß als geeignetes Medium Caseinhydrolysat verwendet wird.

**Patentansprüche für folgenden Vertragsstaat : AT**

**1.** Verfahren zur Herbeiführung einer Expression eines gewünschten eucaryotischen Gens durch Einschleusen eines Plasmids mit

einem doppelsträngigen DNA-Molekül mit in 5'- nach 3'-Richtung

einer DNA-Sequenz, die den Promoter und Operator $P_LO_L$ eines λ-Bakteriophagen enthält,

einer N-Erkennungsstelle zum Binden von Antiterminator N-Protein,

einer einen Austausch der DNA-Sequenz gestattenden ersten Restriktionsenzymstelle mit der folgenden ribosomalen Bindungsstelle,

einer ribosomalen Bindungsstelle, die die mRNA des gewünschten Gens zur Bindung von Ribosomen innerhalb der Wirtszelle befähigt, ausgewählt aus der Gruppe:

eine Mutante von $C_{II}$ aus einem λ-Bakteriophagen mit der Sequenz:

TAAGGAAGTACTTACAT

ATTCCTTCATGAATGTA;

eine von pBR322 (ATCC Hinterlegungs-Nr. 37017) herrührende natürliche β-Lactamase-Ribosombindungsstelle;

ein synthetisches Oligonucleotid der Sequenz:

AATTCGAGCGCAAGGAAACAGGCTCA

GCTCGCGTTCCTTTGTCCGAGTAT;

und ein synthetisches Oligonucleotid der Sequenz:

AATTCAATAATATTGAAAAAGGAAGAG

GTTATTATAACTTTTTCCTTCTCAT;

einem ATG-Initiationscodon und

einer zweiten Restriktionsenzymstelle mit dem darin eingefügten gewünschten Gen mit Codierung für eucaryotisches Polypeptid oder dessen Analoges in Phase mit dem ATG-Initiationscodon,

das zusätzlich eine DNA-Sequenz mit einem Replikationsursprung aus einem bakteriellen Plasmid mit der Fähigkeit zur autonomen Replikation in der Wirtszelle und einem Selektionsmittel, ausgewählt aus der Gruppe DNA-Sequenzen, die ein Gen mit einer selektierbaren oder identifizierbaren phenotypischen und sich bei Anwesenheit des Vektors in der Wirtzelle manifestierenden Eigenschaft enthalten, und DNA-Sequenzen, die das Fragment mit der Bezeichnung $cI^{434}$ mit dem Gen für das $cI^{434}$-Repressorprotein und seine assoziierte Promotor- und Operatorsequenz enthalten, aufweist, wobei der Abstand zwischen dem 3'-Ende der $P_LO_L$-Promotor- und -Operatorsequenz und dem 5'-Ende der N-Erkennungsstelle weniger als etwa 80 Basenpaare und der Abstand zwischen dem 3'-Ende der N-Erkennungsstelle und dem 5'-Ende der ribosomalen Bindungsstelle weniger als etwa 300 Basenpaare betragen,

in eine geeignete bakterielle Wirtszelle mit dem thermolabilen Repressor $C_I$, wobei das Plasmid die Wirtzelle bei Erhöhung der Wirtszellentemperatur auf eine Temperatur, bei der der Repressor inaktiviert wird, zum wirksamen Exprimieren des in das Plasmid eingeschleusten gewünschten eucaryotischen Gens und zur Produktion eines durch das Gen codierten eucaryotischen Polypeptids befähigt.

2. Verfahren nach Anspruch 1, dadurch gekennzeichnet, daß das Plasmid zusätzlich eine DNA-Sequenz mit einer $T_1T_2$ rRNA-Transkriptionsterminationssequenz 3' der zweiten Restriktionsenzymstelle enthält.

3. Verfahren nach Anspruch 1, dadurch gekennzeichnet, daß das Selektionsmittel aus einer DNA-Sequenz mit einem Gen in Verbindung mit einer selektierbaren oder identifizierbaren phenotypischen Eigenschaft besteht.

4. Verfahren nach Anspruch 3, dadurch gekennzeichnet, daß die phenotypische Eigenschaft aus Arzneimittelresistenz besteht.

5. Verfahren nach Anspruch 4, dadurch gekennzeichnet, daß die Arzneimittelresistenz aus einer Resistenz gegen Ampicillin oder Tetracyclin besteht.

6. Verfahren nach Anspruch 2, dadurch gekennzeichnet, daß die $T_1T_2$ rRNA-Transkriptionsterminationssequenz weniger als etwa 100 Basenpaare vom 3'-Ende der zweiten Restriktionsenzymstelle (entfernt) ist.

7. Verfahren nach Anspruch 6, dadurch gekennzeichnet, daß die $T_1T_2$ rRNA-Transkriptionsterminationsequenz weniger als etwa 20 Basenpaare vom 3'-Ende der zweiten Restriktionsenzymstelle (entfernt) ist.

8. Verfahren nach Anspruch 1, dadurch gekennzeichnet, daß das Selektionsmittel aus einer DNA-Sequenz mit dem mit $cI^{434}$ bezeichneten Fragment besteht.

**9.** Verfahren nach Anspruch 8, dadurch gekennzeichnet, daß das cl$^{4.34}$-Fragment nach dem 3'-Ende der $T_1 T_2$ rRNA-Transkriptionsterminationsequenz vorgesehen ist.

**10.** Verfahren nach Anspruch 1, dadurch gekennzeichnet, daß der Replikationsursprung von pBR322 (ATCC Hinterlegungs-Nr. 37017) herrührt.

**11.** Verfahren nach Anspruch 1, dadurch gekennzeichnet, daß der Replikationsursprung von einem bakteriellen Plasmid mit der Fähigkeit zur autonomen Reproduktion in der Wirtszelle und Produktion von mindestens 400 grundlegenden Kopien des Plasmids herrührt.

**12.** Verfahren nach Anspruch 11, dadurch gekennzeichnet, daß der Replikationsursprung von ColE1 herrührt.

**13.** Verfahren nach Anspruch 12, dadurch gekennzeichnet, daß der Replikationsursprung pOP1Δ6 ist.

**14.** Verfahren nach Anspruch 1, dadurch gekennzeichnet, daß das Plasmid zusätzlich eine zwischen der DNA-Sequenz mit der ribosomalen Bindungsstelle und dem ATG-Initiationscodon befindliche dritte Restriktionsenzymstelle aufweist.

**15.** Verfahren nach Anspruch 1, dadurch gekennzeichnet, daß die erste Restriktionsenzymstelle aus einer einzigen Stelle innerhalb des Plasmids besteht.

**16.** Verfahren nach Anspruch 15, dadurch gekennzeichnet, daß die erste einzige Restriktionsenzymstelle EcoRI ist.

**17.** Verfahren nach Anspruch 1, dadurch gekennzeichnet, daß die zweite Restriktionsenzymstelle aus einer einzigen Stelle innerhalb des Plasmids besteht.

**18.** Verfahren nach Anspruch 17, dadurch gekennzeichnet, daß die zweite einzige Restriktionsenzymstelle NdeI, BglII oder SmaI ist.

**19.** Verfahren zur Herstellung von Rinderwachstumshormon oder eines Analogen desselben, enthaltend das Plasmid nach Anspruch 1, wobei es sich bei der DNA-Gensequenz mit Codierung für das eucaryotische Polypeptid um eine in die zweite Restriktionsenzymstelle eingefügte DNA mit Codierung für Rinderwachstumshormon oder ein Analoges desselben handelt.

**20.** Verfahren nach Anspruch 19, dadurch gekennzeichnet, daß das Plasmid aus pRO122 mit der in Fig. 2 dargestellten Restriktionskarte und einer Länge von 3.3 kb, pSAL 5200-6 mit der in Fig. 3 dargestellten Restriktionskarte und einer Länge von 3.3 kb, pHG44 mit der in Fig. 6 dargestellten Restriktionskarte und der Hinterlegungs-Nr. ATCC 39806, pSAL 5600-1 mit der in Fig. 10 dargestellten Restriktionskarte und einer Länge von 4.4 kb, p7200-22 mit der in Fig. 7 dargestellten Restriktionskarte und einer Länge von 4.9 kb, pHG50 mit der in Fig. 6 dargestellten Restriktionskarte und der Hinterlegungs-Nr. ATCC 39805, pSAL-210/4 mit der in Fig. 9 dargestellten Restriktionskarte und einer Länge von 12.6 kb, p8300-10A mit der in Fig. 7 dargestellten Restriktionskarte und der Hinterlegungs-Nr. ATCC 39785, pSAL-130/5 mit der in Fig.8 dargestellten Restriktionskarte und einer Länge von 9.6 kb, pSAL-170/10 mit der in Fig. 8 dargestellten Restriktionskarte und einer Länge von 11.0 kb oder p9200 mit der in Fig. 26 dargestellten Restriktionskarte und der Hinterlegungs-Nr. ATCC 53215 besteht.

**21.** Verfahren zur Herstellung von menschlichem Wachstumshormon oder eines Analogen desselben mit dem Plasmid nach Anspruch 1, wobei die DNA-Gensequenz mit der Codierung für das eucaryotische Polypeptid aus einer in die zweite Restriktionsenzymstelle eingefügten DNA mit Codierung für menschliches Wachstumshormon oder ein Analoges desselben besteht.

**22.** Verfahren nach Anspruch 21, dadurch gekennzeichnet, daß das Plasmid aus pTV300 mit der in Fig. 27 dargestellten Restriktionskarte und einer Länge von 4.3 kb besteht.

**23.** Verfahren zur Herstellung einer Superoxiddismutase oder eines Analogen derselben mit dem Plasmid nach Anspruch 1, wobei die DNA-Gensequenz mit der Codierung für das eucaryotische Polypeptid aus einer in die zweite Restriktionsenzymstelle eingefügten DNA mit Codierung für Superoxiddismutase oder ein Analoges derselben besteht.

**24.** Verfahren nach Anspruch 23, dadurch gekennzeichnet, daß das Plasmid aus pSODα2 mit der in Fig. 13 dargestellten Restriktionskarte und der Hinterlegungs-Nr. ATCC 39786, pSODβ1 mit der in Fig. 14 dargestellten Restriktionskarte und einer Länge von 3.0 kb, pSODβ₁T₁₁ mit der in Fig. 15 dargestellten Restriktionskarte und einer Länge von 3.7 kb, pSODβ₁-BA2 mit der in Fig. 17 dargestellten Restriktionskarte oder pSODβ₁TT-1 mit der in Fig. 16 dargestellten Restriktionskarte und einer Länge von 4.8 kb besteht.

**25.** Verfahren zur Herstellung von Schweinewachstumshormon oder eines Analogen desselben mit dem Plasmid nach Anspruch 1, wobei die DNA-Gensequenz mit Codierung für das eucaryotische Polypeptid aus einer in die zweite Restriktionsenzymstelle eingefügten DNA mit Codierung für Schweinewachstumshormon oder ein Analoges desselben besteht.

**26.** Verfahren nach Anspruch 25, dadurch gekennzeichnet, daß das Plasmid aus p3008 mit der in Fig. 4 dargestellten Restriktionskarte und der Hinterlegungs-Nr. ATCC 39804 oder p3009 mit der in Fig. 11 dargestellten Restriktionskarte und einer Länge von 4.3 kb besteht.

**27.** Verfahren zur Herstellung eines Hühnerwachstumshormons oder eines Analogen desselben mit dem Plasmid nach Anspruch 1, wobei die DNA-Gensequenz mit Codierung für das eurocaryotische Polypeptid aus einer in die zweite Restriktionsenzymstelle eingefügten DNA mit Codierung für Hühnerwachstumshormon oder ein Analoges desselben besteht.

**28.** Verfahren nach Anspruch 27, dadurch gekennzeichnet, daß das Plasmid aus p5002 mit der in Fig. 5 dargestellten Restriktionskarte und einer Länge von 3.3 kb oder p5003 mit der in Fig. 12 dargestellten Restriktionskarte und der Hinterlegungs-Nr. ATCC 39792 besteht.

**29.** Verfahren zur Herstellung von Humanapolipoprotein E oder eines Analogen desselben mit dem Plasmid nach Anspruch 1, wobei die DNA-Gensequenz mit Codierung für das eucaryotische Polypeptid aus einer in die zweite Restriktionsenzymstelle eingefügten DNA mit Codierung für Humanapolipoprotein E oder eines Analogen desselben besteht.

**30.** Verfahren nach Anspruch 29, dadurch gekennzeichnet, daß das Plasmid aus pTV-188 mit der in Fig. 18 dargestellten Restriktionskarte und einer Länge von 4.2 kb, pSAL 160-5 mit der in Fig. 23 dargestellten Restriktionskarte und einer Länge von 5.2 kb, pTV-170 mit der in Fig. 20 dargestellten Restriktionskarte und einer Länge von 4.4 kb, pTV-190 mit der in Fig. 21 dargestellten Restriktionskarte und einer Länge von 4.4 kb, pTV-194 mit der in Fig. 22 dargestellten Restriktionskarte und einer Länge von 4.5 kb, pTV-214 mit der in Fig. 24 dargestellten Restriktionskarte und einer Länge von 4.4 kb, pTV-264-45 mit der in Fig. 25 dargestellten Restriktionskarte und einer Länge von 4.4 kb oder pTVR-279-8 mit der in Fig. 25 dargestellten Restriktionskarte und der Hinterlegungs-Nr. ATCC 53216 besteht.

**31.** Verfahren nach Anspruch 1, dadurch gekennzeichnet, daß das Plasmid in einen geeigneten Escherichia coli-Wirt eingeschleust ist.

**32.** Verfahren nach Anspruch 31, dadurch gekennzeichnet, daß der Wirt aus dem Escherichia coli-Stamm A1637 (ATCC Hinterlegungs-Nr. 39385 mit dem Plasmid pRec 2/3), dem Escherichia coli-Stamm A1645 (ATCC Hinterlegungs-Nr. 39787 mit dem Plasmid pApoE-Ex.2) oder dem Escherichia coli-Stamm A2097 (ATCC Hinterlegungs-Nr. 39786 mit dem Plasmid pSODα2) besteht.

**33.** Verfahren nach Anspruch 31, dadurch gekennzeichnet, daß der Wirt aus einem Prototroph besteht.

**34.** Verfahren nach Anspruch 33, dadurch gekennzeichnet, daß der Wirt aus dem Escherichia coli-Stamm A4200 (ATCC Hinterlegungs-Nr. 53218 mit pHG44 und dem F'-gal Plasmid und der Bezeichnung A 4346) besteht.

**35.** Verfahren nach Anspruch 33, dadurch gekennzeichnet, daß der Wirt aus dem Escherichia coli-Stamm A4255 (ATCC Hinterlegungs-Nr. 53215 mit p9200 mit der Bezeichnung A4320) besteht.

**36.** Verfahren nach Anspruch 33, dadurch gekennzeichnet, daß der Wirt biotinunabhängig ist.

**37.** Verfahren nach Anspruch 31, dadurch gekennzeichnet, daß der Wirt aus einem geeigneten lytischen Stamm besteht.

**38.** Verfahren nach Anspruch 37, dadurch gekennzeichnet, daß der Wirt aus dem Stamm A4048 (ATCC Hinterlegungs-Nr. 53217 mit pHG44 und der Bezeichnung A3111) besteht.

**39.** Verfahren nach Ansprüchen 19, 21, 23, 25, 27 oder 29, dadurch gekennzeichnet, daß das Plasmid in einem geeigneten Escherichiacoli-Wirt eingeschleust ist.

**40.** Verfahren zur Herstellung eines Polypeptids durch Züchten des Wirt/Plasmid-Systems nach Anspruch 31 unter geeigneten Bedingungen, die die Produktion des Polypeptids und die Gewinnung des erhaltenen Polypeptids gestatten.

**41.** Verfahren zur Gewinnung von Rinderwachstumshormon oder eines Analogen desselben durch Züchten eines geeigneten Escherichiacoli-Wirts mit dem Plasmid gemäß dem Verfahren nach Anspruch 19 unter geeigneten Bedingungen, die die Produktion von Rinderwachstumshormon oder eines Analogen desselben und die Gewinnung des erhaltenen Rinderwachstumshormons oder Analogen desselben gestatten.

**42.** Verfahren zur Gewinnung von menschlichem Wachstumshormon oder eines Analogen desselben durch Züchten eines geeigneten Escherichia coli-Wirts mit dem Plasmid gemäß dem Verfahren nach Anspruch 21 unter geeigneten Bedingungen, die die Produktion von menschlichem Wachstumshormon oder eines Analogen desselben und die Gewinnung des erhaltenen menschlichen Wachstumshormons oder Analogen desselben gestatten.

**43.** Verfahren zur Gewinnung von Schweinewachstumshormon oder eines Analogen desselben durch Züchten eines geeigneten Escherichia coli-Wirts mit dem Plasmid gemäß dem Verfahren nach Anspruch 25 unter geeigneten Bedingungen, die die Produktion von Schweinewachstumhormon oder eines Analogen desselben und die Gewinnung des erhaltenen Rinderwachstumshormons oder Analogen desselben gestatten.

**44.** Verfahren zur Gewinnung von Hühnerwachstumshormon oder eines Analogen desselben durch Züchten eines geeigneten Escherichia coli-Wirts mit dem Plasmid gemäß dem Verfahren nach Anspruch 27 unter geeigneten Bedingungen, die die Produktion von Hühnerwachstumshormon oder eines Analogen desselben und die Gewinnung des erhaltenen Hühnerwachstumshormons oder Analogen desselben gestatten.

**45.** Verfahren zur Gewinnung von Superoxiddismutase oder eines Analogen derselben durch Züchten eines geeigneten Escherichia coli-Wirts mit dem Plasmid gemäß dem Verfahren nach Anspruch 23 unter geeigneten Bedingungen, die die Produktion von Superoxiddismutase oder eines Analogen derselben und die Gewinnung des erhaltenen Superoxiddismutase oder des Analogen derselben gestatten.

**46.** Verfahren zur Gewinnung von Humanapolipoprotein E durch Züchten eines geeigneten Escherichia coli-Wirts mit dem Plasmid gemäß dem Verfahren nach Anspruch 29 unter geeigneten Bedingungen, die die Produktion von Humanapolipoprotein E und die Gewinnung des gebildeten Humanapolipoprotein E gestatten.

**47.** Verfahren nach Anspruch 40, wobei unter "geeigneten Bedingungen" die Züchtung des Wirt/Plasmid-Systems während einer geeigneten Zeitdauer bei etwa 42°C auf einem geeigneten Medium zu verstehen ist.

**48.** Verfahren nach Anspruch 47, wobei unter der "geeigneten Zeitdauer" bei 42°C etwa 1-5 h zu verstehen ist (sind).

**49.** Verfahren nach Anspruch 47, dadurch gekennzeichnet, daß als geeignetes Medium Caseinhydrolysat verwendet wird.

EP 0 173 280 B1

Figure 1

Figure 2

## Figure 3

Figure 4

Figure 5

Figure 6

Figure 7

Figure 8

1. Partial (NdeI + HindIII)
2. Isolate Large Fragment

1. HindIII + NdeI
2. Isolate bGH Fragment

Ligase

p8300-10A

pHG44

pSAL-130/5
9.6 Kb

pBR322

1. AvaI + EcoRI
2. Fill in
3. Isolate Tet$^R$ Fragment

1. BamHI
2. Fill in

Ligase

pSAL-170/10
11.0 Kb

Figure 9

Figure 10

Figure 11

Figure 12

Figure 13

Figure 14

Figure 15

Figure 16

Figure 17

Figure 18

Figure 19

Figure 20

Figure 21

Figure 22

EP 0 173 280 B1

Figure 23

118

Figure 24

Figure 25

Figure 26

Figure 27

FIG. 28

FIG. 29

FIG. 30

FIG. 31